# EUROPEAN PATENT APPLICATION

(11) **EP 3 708 168 A2**
(43) Date of publication of application: **16.09.2020**
(21) Application number: 20159783.8
(22) Date of filing: 14.09.2015
(51) Int. Cl.: A61K 31/485, A61K 31/381, A61K 31/4525, A61K 31/49, A61P 25/00, A61P 25/28, A61K 9/48

(54) **COMBINATIONS OF DEUTERATED DEXTROMETHORPHAN AND QUINIDINE FOR THE TREATMENT OF AGITATION IN DEMENTIA**

(30) Priority: 14.09.2014 US 201462050170 P; 08.10.2014 US 201462061451 P; 13.10.2014 US 201462063122 P; 14.10.2014 US 201462063861 P; 26.10.2014 US 201462068742 P; 02.02.2015 US 201562111053 P; 03.02.2015 US 201562111590 P; 04.03.2015 US 201562128446 P; 15.05.2015 US 201562162140 P; 22.05.2015 US 201562165535 P; 02.06.2015 US 201562169997 P; 15.06.2015 US 201562180026 P; 16.07.2015 US 201562193347 P; 14.08.2015 US 201562205061 P; 10.09.2015 US 201562216636 P; 11.09.2015 US 201562217470 P; 11.09.2015 US 201562217142 P
(62) Divisional of application: 15840718.9
(71) Applicant: Avanir Pharmaceuticals, Inc., Aliso Viejo CA 92656 (US)
(72) Inventor: SIFFERT, Joao, Aliso Viejo, California 92656 (US)
(74) Representative: Finnegan Europe LLP

(57) **Abstract**

The present disclosure is related to methods of treating agitation and/or aggression and/or associated symptoms in subjects with dementia, including, dementia associated with Alzheimer's disease. The method includes administering to a subject in need thereof a dextromethorphan compound, or a pharmaceutically acceptable salt thereof, in combination with quinidine, or a pharmaceutically acceptable salt thereof. The disclosure in certain aspects is also related to compositions that are used for treating agitation and/or aggression and/or associated symptoms in subjects suffering from Alzheimer's disease.

## Description

### PRIORITY

This application claims the benefit of U.S. Provisional Application Nos. 62/050,170, filed September 14, 2014; 62/061,451, filed October 8, 2014; 62/063,122, filed October 13, 2014; 62/063,861, filed October 14, 2014; 62/068,742, filed October 26, 2014; 62/111,053, filed February 2, 2015; 62/111,590, filed February 3, 2015; 62/128,446, filed March 4, 2015; 62/162,140, filed May 15, 2015; 62/165,535, filed May 22, 2015; 62/169,997, filed June 2, 2015; 62/180,026, filed June 15, 2015; 62/193,347, filed July 16, 2015; and 62/205,061, filed August 14, 2015; 62/216,636, filed September 10, 2015; 62/217,142, filed September 11, 2015; and 62/217,470, filed September 11, 2015, all of which are incorporated herein by reference.

### FIELD OF THE INVENTION

The present disclosure relates to the treatment of behavioral disorders, such as agitation and/or aggression and/or associated symptoms, associated with central nervous system (CNS) disorders, such as Alzheimer's disease. The methods related to the present disclosure include administering a dextromethorphan compound, alone or in combination with a CYP2D6 inhibitor, such as quinidine (Q), to a subject suffering from agitation and/or aggression and/or associated symptoms in central nervous system (CNS) disorders, such as Alzheimer's disease. The disclosure is also related to pharmaceutical combinations including a dextromethorphan compound, alone or in combination with a CYP2D6 inhibitor, such as quinidine (Q).

### BACKGROUND

Alzheimer's disease is a progressive neurodegenerative disease that eventually leads to death. An estimated 5.4 million Americans have Alzheimer's disease. That number has doubled since 1980 and is expected to be as high as 16 million by 2050 (Brookmeyer et al., Alzheimers Dement. 2011;7(1):61-73). Among US adults over age 65, prevalence estimates of dementia range from 5% to 15%, with Alzheimer's disease being the most common type of dementia (Kaplan and Sadock's Synopsis of Psychiatry: Behavioral Sciences, 1998; Evans et al., JAMA. 1989;262(18):2551-6; Losonczy et al., Public Health Reports., 1998;113:273-80).

Agitation is widely recognized as a common and important clinical feature of AD and other forms of dementia (Ballard CG et al., Nat Rev Neurol. 2009;5(5):245-255). Although readily recognized by clinicians and caregivers, a consensus definition of agitation in dementia was only recently developed by the International Psychogeriatric Association (IPA) Agitation Definition Working Group (ADWG) with the following criteria: "1) occurring in patients with a cognitive impairment or dementia syndrome; 2) exhibiting behavior consistent with emotional distress; 3) manifesting excessive motor activity, verbal or physical aggression; and 4) evidencing behaviors that cause excess disability impairing relationships and/or daily activities and are not solely attributable to another disorder (psychiatric, medical, or substance-related)" (Cummings J et al., Int. Psychogeriatr. 2014:1-11). Agitation and/or aggression are estimated to affect up to approximately 80% of patients with dementia (Ryu S-H et al., Am J Geriatr Psychiatry. 2005;13(11):976-983; Tractenberg RE et al., J Geriatr psychiatry Neurol. 2003;16(2):94-99) with an increase in prevalence as the disease progresses.

Agitation in patients with dementia is associated with increased functional disability (Rabins P V et al., Alzheimer's Dement. 2013;9(2):204-207), worse quality of life (Gonzdlez-Salvador T et al., Int J Geriatr Psychiatry. 2000;15(2):181-189), earlier institutionalization (Steele C et al., Am J Psychiatry. 1990;147(8):1049-1051), increased caregiver burden (Rabins P V et al., Alzheimer's Dement. 2013;9(2):204-207), increased healthcare costs (Murman DL et al., Neurology. 2002;59(11):1721-1729), shorter time to severe dementia (Peters ME et al., Am J Geriatr Psychiatry. 2014;22(3):S65-S66), and accelerated mortality (Peters ME et al., Am J Geriatr Psychiatry. 2014;22(3):S65-S66). For these reasons, agitation and aggression are the neuropsychiatric symptoms most likely to require pharmacological intervention in Alzheimer's patients (Ballard CG et al., Nat Rev Neurol. 2009;5(5):245-255).

However, there are currently no FDA-approved pharmacological treatments for agitation in AD, and clinicians ultimately resort to off-label use of antipsychotics, sedatives/hypnotics, anxiolytics, and antidepressants in an attempt to control symptoms (Maher AR et al. JAMA. 2011;306(12): 1359-1369). Unfortunately, these treatments have limited utility given a modest efficacy that is offset by relatively poor adherence, safety, and tolerability (Ballard CG et al., Nat Rev Neurol. 2009;5(5):245-255; Schneider LS et al., N Engl J Med. 2006;355(15):1525-1538; Huybrechts KF et al., BMJ 2012;344:e977). Thus a critical need exists to develop a safe and effective pharmacological intervention for the treatment of agitation in dementia. Such a treatment could profoundly impact patient care, reduce caregiver burden, and potentially improve overall disease prognosis.

Since there is no U.S. Food and Drug Administration (FDA)-approved treatment for Alzheimer's-related behavioral symptoms, prescription of off-label antipsychotic drugs has been commonly employed to treat symptoms such as aggression and agitation (Salzman et al., J. Clin. Psychiatry. 2008; 69(6):889-98; Levenson and Crecelius, AMDA: Publications - Caring for the Ages., 2003;4(6):31,34-35). Atypical antipsychotics have a modest effect in the short-term treatment of aggression (over 6-12 weeks) but only limited benefits in longer term therapy, as adverse effects tend to offset potential efficacy (Scheider et al., N. Engl. J. Med. 2006;355(15):1525-38). Benefits are less well established for other symptoms such as agitation (Sultzer et al., Am. J. Psychiatry. 2008; 165(7):844-54). In addition, there are significant concerns over the potential for serious adverse outcomes with the use of antipsychotic medications, including stroke and increased death rate (Hybrechts et al., BMJ. 2012; 344: e977). Non-antipsychotic psychotropic medications have also been studied both for symptom mitigation and secondary prevention, but to little effect (Tariot et al., Arch. Gen. Psychiatry. 2011; 68(8):853-61). A careful consideration of other pharmacological and nonpharmacological approaches to treating agitation and aggression in subjects with Alzheimer's disease is, therefore, imperative (Ballard et al., Curr. Opin. Psychiatry. 2009; 22(6):532-40; Lyketsos et al., The Journal of Alzheimer's Association. 2011; 7(5):532-9).

Dextromethorphan is the common name for (+)-3-methoxy-N-methylmorphinan. It is one of a class of molecules that are dextrorotatory analogs of morphine-like opioids. Dextromethorphan is known to have at least three distinct receptor activities that affect central nervous system neurons. Although the pharmacological profile of dextromethorphan points to clinical efficacy for several indications, when administered by itself the efficacy of dextromethorphan has been disappointing compared to placebo.

It has long been known that in most people (estimated to include about 90% of the general population in the United States), dextromethorphan undergoes extensive hepatic O-demethylation to dextrorphan that is catalyzed by CYP2D6 and is rapidly eliminated by the body (Ramachander et al., J. Pharm. Sci. 1977;66(7):1047-8; and Vetticaden et al., Pharm. Res. 1989;6(1):13-9). CYP2D6 is a member of a class of oxidative enzymes that exist in high concentrations in the liver, known as cytochrome P450 enzymes (Kronbach et al., Anal. Biochem. 1987; 1 62(1):24-32; and Dayer et al., Clin. Pharmacol. Ther. 1989;45(1):34-40).

In addition to metabolizing dextromethorphan, CYP2D6 is also responsible for polymorphic debrisoquine hydroxylation in humans (Schmid et al., Clin. Pharmacol. Ther. 1985;38:618-624). An alternate pathway is mediated primarily by CYP3A4 and N-demethylation to form 3-methoxymorphinan (Von Moltke et al., J. Pharm. Pharmacol., 1998;50:997-1004). Both dextrorphan and 3-methoxymorphinan can be further demethylated to 3-hydroxymorphinan that is then subject to glucuronidation. The metabolic pathway that converts dextromethorphan to dextrorphan is dominant in the majority of the population and is the principle behind using dextromethorphan as a probe to phenotype individuals as CYP2D6 extensive and poor metabolizers (Kupfer et al., Lancet. 1984;2:517-518; Guttendorf et al., Ther. Drug Monit. 1988;10:490-498). Approximately 7% of the Caucasian population shows the poor metabolizer phenotype, while the incidence of poor metabolizer phenotype in Chinese and Black African populations is even lower (Droll et al., Pharmacogenetics. 1998;8:325-333). A study examining the ability of dextromethorphan to increase pain threshold in extensive and poor metabolizers found antinociceptive effects of dextromethorphan were significant in poor metabolizers but not in extensive metabolizers (Desmeules et al., J. Pharmacol. Exp. Ther. 1999;288:607-612). The results are consistent with direct effects of parent dextromethorphan rather than the dextrorphan metabolite on neuromodulation.

### SUMMARY OF THE INVENTION

As described above, there remains an urgent need for additional or improved forms of treatment for agitation, aggression and/or associated symptoms in subjects with Alzheimer's disease. This disclosure provides methods of treating agitation and/or aggression and/or associated symptoms in subjects with Alzheimer's disease without an increased risk of serious adverse effects.

A first aspect of the present disclosure provides a method for treating agitation and/or aggression and/or associated symptoms in subjects with dementia by administering a dextromethorphan compound in combination with a CYP2D6 inhibitor, such as quinidine (Q). The disclosure also encompasses the use of pharmaceutically acceptable salts of either or both the dextromethorphan compound and quinidine in the described methods. An analog or a derivative of quinidine may also be used in the methods described in the present disclosure. In one embodiment the dementia is Alzheimer's type dementia.

A second aspect of the present disclosure is related to a combination of the dextromethorphan compound and a CYP2D6 inhibitor, such as quinidine. The disclosure also encompasses the use of pharmaceutically acceptable salts of either or both the dextromethorphan compound and quinidine in the described combination. The combination can be used for treating agitation and/or aggression and/or associated symptoms in a subject with dementia. Analogs or derivatives of quinidine may also be used in the combination. In one embodiment the dementia is Alzheimer's type dementia.

In some embodiments, according to the first and the second aspects of the disclosure, the dextromethorphan compound is administered or used in an amount ranging from about 10 mg per day to about 200 mg per day, and quinidine is administered or used in an amount ranging from about 0.05 mg per day to less than about 50 mg per day.

In some embodiments, quinidine is administered or used in an amount ranging from about 4.75 mg per day to about 20 mg per day. In some embodiments, the dextromethorphan compound is administered or used in an amount ranging from about 10 mg per day to about 90 mg per day.

In some embodiments, either or both of quinidine and the dextromethorphan compound is in the form of a pharmaceutically acceptable salt. The pharmaceutically acceptable salts according to the present disclosure include alkali metals, salts of lithium, salts of sodium, salts of potassium, salts of alkaline earth metals, salts of calcium, salts of magnesium, salts of lysine, salts of N,N'dibenzylethylenediamine, salts of chloroprocaine, salts of choline, salts of diethanolamine, salts of ethylenediamine, salts of meglumine, salts of procaine, salts of tris, salts of free acids, salts of free bases, inorganic salts, salts of sulfate, salts of hydrochloride, and salts of hydrobromide. In some embodiments, the dextromethorphan compound is in the form of dextromethorphan hydrobromide or deuterated dextromethorphan hydrobromide. In some embodiments, quinidine is in the form of quinidine sulfate.

In some embodiments, the dextromethorphan compound and quinidine are administered or used in a unit dosage form. In some embodiments, the unit dosage form includes about 4.75 mg, 4.9 mg, 9 mg, or 10 mg of quinidine (for example, quinidine sulfate) and about 45 mg, 34 mg, 30 mg, 28 mg, 24 mg, 23 mg, 20 mg, 18 mg, 15 mg, or 10 mg of dextromethorphan compound (for example, deuterated dextromethorphan hydrobromide or dextromethorphan hydrobromide). In one embodiment, the unit dosage form comprises about 10 mg of quinidine (for example, quinidine sulfate) and about 20 mg or 30 mg of dextromethorphan compound (for example, dextromethorphan hydrobromide or deuterated dextromethorphan hydrobromide). In another embodiment, the unit dosage form comprises about 9 mg of quinidine (for example, quinidine sulfate) and about 15 mg or 23 mg of dextromethorphan compound (for example, dextromethorphan hydrobromide or deuterated dextromethorphan hydrobromide). In another embodiment, the unit dosage form comprises about 4.9 mg of quinidine (for example, quinidine sulfate) and about 18 mg, 24 mg, 28 mg, 30 mg, or 34 mg of dextromethorphan compound (for example, dextromethorphan hydrobromide or deuterated dextromethorphan hydrobromide). In yet another embodiment, the unit dosage form comprises about 4.75 mg of quinidine (for example, quinidine sulfate) and about 18 mg, 24 mg, 28 mg, 30 mg, or 34mg of dextromethorphan compound (for example, dextromethorphan hydrobromide or deuterated dextromethorphan hydrobromide). In some embodiments, the unit dosage forms of the dextromethorphan compound and quinidine are in the form of a tablet or a capsule.

In some embodiments, the dextromethorphan compound and quinidine are administered or used in a combined dose or a separate dose in a weight ratio of the dextromethorphan compound to quinidine of about 1:1 or less. In some embodiments, the weight ratio is about 1:1, 1:0.95, 1:0.9, 1:0.85, 1:0.8, 1:0.75, 1:0.7, 1:0.65, 1:0.6, 1:0.55 or 1:0.5 or less. Likewise, in certain embodiments, dosages have a weight ratio of the dextromethorphan compound to quinidine less than about 1:0.5, for example, about 1:0.45, 1:0.4, 1:0.35, 1:0.3, 1:0.25, 1:0.2, 1:0.15, or 1:0.1, 1:0.09, 1:0.08, 1:0.07, 1:0.06, 1:0.05, 1:0.04, 1:0.03, 1:0.02, or 1:0.01, or less. The weight ratios can be for example, about 1:0.75, about 1:0.68, about 1:0.6, about 1:0.56, about 1:0.5, about 1:0.44, about 1:0.39, about 1:0.38, about 1:0.31, about 1:0.30, about 1:0.29, about 1:0.28, about 1:0.27, about 1:0.26, about 1:0.25, about 1:0.24, about 1:0.23, about 1:0.22, about 1:0.21, about 1:0.20, about 1:0.19, about 1:0.18, about 1:0.17, 1:0.16, about 1:0.15, about 1:0.14, about 1:0.13, about 1:0.12, about 1:0.11 and about 1:0.10. In some embodiments, the weight ratios for free base of dextromethorphan to free base of quinidine is about 1:0.68, about 1:0.56, about 1: 0.44, about 1:0.38. In certain other embodiments, the weight ratios for free base of d6-deuterated dextromethorphan to free base of quinidine is about 1: 0.30, about 1:0.22, about 1: 0.19, about 1: 0.18, about 1:0.16, and about 1:0.15.

The dextromethorphan compound and quinidine may be administered or used as one combined dose per day or as at least two combined doses per day. In one embodiment the dextromethorphan compound and quinidine are administered conjointly.

In some embodiments, the improvement by treatment with a dextromethorphan compound in combination with quinidine in agitation and/or aggression and/or associated symptoms in subjects with Alzheimer's disease may be measured by improvements of one or more of the following scores:
- Neuropsychiatric Inventory (NPI) agitation/aggression domain;
- NPI total;
- Composite of NPI agitation/aggression; irritability/lability; aberrant motor behavior and anxiety domains (NPI4A);
- Composite of NPI agitation/aggression; irritability/lability; aberrant motor behavior and disinhibition domains (NPI4D)
- NPI caregiver distress - agitation/aggression domain;
- Modified Alzheimer Disease Cooperative Study - Clinical Global Impression of Change (ADCS - CGIC) score of agitation; and/or
- Patient Global Impression of Change (PGI-C) score of agitation.

In one embodiment, the subject's NPI score for agitation/aggression is reduced by at least 1.5 compared to untreated subjects or subjects administered a placebo.

In one embodiment, the subject's NP14A score is reduced by at least 2.4 compared to untreated subjects or subjects administered a placebo.

In one embodiment, the subject's NP14D score is reduced by at least 3.0 compared to untreated subjects or subjects administered a placebo.

In one embodiment, the subject's ADCS - CGIC score of agitation is improved by at least 0.5 compared to untreated subjects or subjects administered a placebo.

In one embodiment, the subject's PGI-C score of agitation is improved by at least 0.6 compared to untreated subjects or subjects administered a placebo.

The pharmaceutical preparations disclosed herein may, optionally, include pharmaceutically acceptable carriers, adjuvants, fillers, or other pharmaceutical compositions, and may be administered in any of the numerous forms or routes known in the art.

The methods disclosed herein may also, optionally, include administration of the dextromethorphan compound and a CYP2D6 inhibitor, such as quinidine, in conjunction with other therapeutic agents, such as, for example, one or more therapeutic agents known or identified for treatment of Alzheimer's disease.

It is to be understood that the foregoing general description and the following detailed description are exemplary and explanatory only, and are intended to provide further, non-limiting explanation of the disclosure.

### BRIEF DESCRIPTION QF THE DRAWINGS

Figure 1 shows the chemical structure of an embodiment of the dextromethorphan compound of the present disclosure, namely, d6-deuterium-modified dextromethorphan.
Figure 2 provides the study design for the Agitation in Alzheimer's Disease Clinical Study. "Dextromethorphan/quinidine 20/10" refers to a dose of 20 mg dextromethorphan and 10 mg quinidine. QD and BID refer to dosages of once daily and twice per day, respectively. Asterisk (*) denotes participants who discontinued prior to the Week 1 visit and therefore did not have any post-baseline data for the primary efficacy endpoint.
Figure 3 provides a schematic of the Consolidated Standards of Reporting Trials (CONSORT) patient flow chart for the Agitation in Alzheimer's Disease Clinical Study described herein. The populations denoted with ‡ represent those included in the sequential parallel comparison design (SPCD).
Figure 4 illustrates the mean NPI agitation/aggression scores in stage 1 for subjects included in the Agitation in Alzheimer's Disease Clinical Study described herein, which utilized the sequential parallel comparison design (or SPCD). P-values, calculated from an Analysis of Covariance (ANCOVA) model with treatment as fixed effect and baseline as covariate, are given for each visit. ^{a} = Observed cases.
Figure 5 illustrates the mean NPI agitation/aggression scores in stage 2 for subjects included in the Agitation in Alzheimer's Disease Clinical Study (utilizing the SPCD). P-values, calculated from an ANCOVA model with treatment as fixed effect and baseline as covariate, are given for each visit. ^{a} = Observed cases.
Figure 6 illustrates the mean NPI agitation/aggression scores in the 10-week secondary analysis of the Agitation in Alzheimer's Disease Clinical Study described herein. The 10-week secondary analysis included only subjects who remained in the same treatment assignment during the study, i.e., were randomized to receive only dextromethorphan/quinidine or only placebo for the entirety of the study, thus simulating a parallel design. P-values, calculated from ANCOVA model with treatment as fixed effect and baseline as covariate, are given for each visit. ^{a} = Observed cases.
Figure 7 shows the mean plasma concentrations over time by treatment for paroxetine and AVP-786 - Group 1.
Figure 8 shows the mean plasma concentrations over time by treatment for paroxetine and AVP-786 - Group 2.
Figure 9 shows the mean plasma concentrations over time by treatment for duloxetine and AVP-786 - Group 3.
Figure 10 shows the mean plasma concentrations over time by treatment for duloxetine and AVP-786 - Group 4.

### DETAILED DESCRIPTION

The following detailed description and examples illustrate certain embodiments of the present disclosure. Those of skill in the art will recognize that there are numerous variations and modifications of this disclosure that are encompassed by its scope. Accordingly, the description of certain embodiments should not be deemed to limit the scope of the present disclosure.

All references cited herein, including, but not limited to, published and unpublished applications, patents, and literature references, are incorporated herein by reference in their entirety and are hereby made a part of this specification.

### Definitions

The terms "ameliorate" and "treat" are used interchangeably and include therapeutic treatment. Both terms mean improve, decrease, suppress, attenuate, diminish, arrest, or stabilize the development or progression of a disease (e.g., a disease or disorder delineated herein) or symptoms of a disease, alone or in constellations (e.g. syndrome). The term "treat" is used herein to mean to relieve or alleviate at least one symptom of a disease in a subject. For example, in relation to behavioral disorders, the term "treat" may mean to relieve or alleviate agitation and/or aggression and/or associated symptoms and any combination of its manifestations (e.g. punching, cursing, hitting, pacing, resisting, etc.) and associated behaviors (e.g. irritability, anxiety, etc.). Within the meaning of the present disclosure, the term "treat" also denotes to arrest, delay the onset (i.e., the period prior to clinical manifestation of a disease), and/or reduce the risk of developing or worsening a disease.

"Disease" means any condition or disorder that damages or interferes with the normal function of a cell, tissue, organ or an organism.

The term "dementia" refers to a general mental deterioration due to organic or psychological factors; characterized by disorientation, impaired memory, judgment, and intellect, and a shallow labile affect. Dementia herein includes vascular dementia, ischemic vascular dementia (IVD), frontotemporal dementia (FTD), Lewy body dementia, Alzheimer's dementia, etc. The most common form of dementia is associated with Alzheimer's disease (AD).

"Alzheimer's disease (AD)" refers to progressive mental deterioration manifested by memory loss, confusion, and disorientation, generally beginning later in life, and commonly resulting in death in 5-10 years. Alzheimer's disease can be diagnosed by a skilled neurologist or clinician. In one embodiment, the subject with AD will meet National Institute of Neurological and Communicative Disorders and Stroke/Alzheimer's Disease and Related Disorders Association (NINCDS/ADRDA) criteria for the presence of probable AD.

The term "analog" or "derivative" is used herein in the conventional pharmaceutical sense to refer to a molecule that structurally resembles a reference molecule (such as dextromethorphan, deuterated dextromethorphan, or quinidine), but has been modified in a targeted and controlled manner to replace one or more specific substituents of the reference molecule with an alternate substituent, thereby generating a molecule which is structurally similar to the reference molecule. Synthesis and screening of analogs (e.g., using structural and/or biochemical analysis) to identify slightly modified versions of a known compound that may have improved or biased traits (such as higher potency and/or selectivity at a specific targeted receptor type, greater ability to penetrate mammalian blood-brain barriers, fewer side effects, etc.) is a drug design approach that is well known in pharmaceutical chemistry. In addition, using methods known to those skilled in the art, analogs and derivatives of the compounds of the disclosure can be created which have improved therapeutic efficacy, i.e., higher potency and/or selectivity at a specific targeted receptor type, either greater or lower ability to penetrate mammalian blood-brain barriers (e.g., either higher or lower blood-brain barrier permeation rate), fewer side effects, longer existence in the subject, etc.

The term "agitation," as used in this disclosure, is an umbrella term that can refer to a range of behavioral disturbances or disorders, including aggression, combativeness, and hyperactivity. For the purposes of this disclosure the definition includes agitation as described by Cummings et al. International Psychogeriatrics; Volume 27; Issue 01; January 2015, pp 7-17. Broadly, Cummings et al. define agitation as: 1) occurring in patients with a cognitive impairment or dementia syndrome; 2) exhibiting behavior consistent with emotional distress; 3) manifesting excessive motor activity, verbal aggression, or physical aggression; and 4) evidencing behaviors that cause excess disability and are not solely attributable to another disorder (psychiatric, medical, or substance-related). The term agitation also includes:
1) Excessive motor activity associated with a feeling of inner tension. The activity is usually non-productive and repetitious and consists of such behavior as inability to sit still, pacing, wringing of hands, and pulling at clothes;
2) Inappropriate verbal, vocal, or motor activity that is not explained by needs or confusion per se. It includes behaviors such as aimless wandering, pacing, cursing, screaming, biting, and fighting;
3) Vocal or motor behavior that is either disruptive, unsafe, or interferes with the delivery of care in a particular environment. It includes four behavioral areas such as vocalization, motor disturbances, aggressiveness, and resisting care;
4) Behaviors that communicate to others that the subject is experiencing an unpleasant state of excitement and which remain after interventions to reduce internal or external stimuli by managing resistiveness, alleviating aversive physical signs, and decreasing sources of accumulated stress have been carried out; and
5) Behavior where the subject refuses to cooperate, won't let people help, or is hard to handle;

The term "associated symptoms" as used herein refers to symptoms associated with a patient that meets criteria for a cognitive impairment or dementia syndrome (e.g. AD, FTD, DLB, vascular dementia, other dementias, a pre-dementia cognitive impairment syndrome such as mild cognitive impairment or other cognitive disorder). Associated symptoms include, for example, behaviors that are associated with observed or inferred evidence of emotional distress (e.g. rapid changes in mood, irritability, outbursts). In some instances, the behavior is persistent or frequently recurrent for a minimum of two weeks' and represents a change from the patient's usual behavior. The term "associated symptoms" also includes excessive motor activity (examples include: pacing, rocking, gesturing, pointing fingers, restlessness, performing repetitious mannerisms), verbal aggression (e.g. yelling, speaking in an excessively loud voice, using profanity, screaming, shouting), physical aggression (e.g. grabbing, shoving, pushing, resisting, hitting others, kicking objects or people, scratching, biting, throwing objects, hitting self, slamming doors, tearing things, and destroying property).

The term "combination" applied to active Ingredients is used herein to define a single pharmaceutical composition (formulation) comprising both drugs of the disclosure (i.e., a dextromethorphan compound and quinidine) or two separate pharmaceutical compositions (formulations), each comprising a single drug of the disclosure (i.e., a dextromethorphan compound or quinidine), to be administered conjointly.

Within the meaning of the present disclosure, the term "conjoint administration" is used to refer to administration of a dextromethorphan compound and quinidine simultaneously in one composition, or simultaneously in different compositions, or sequentially. For sequential administration to be considered "conjoint," the dextromethorphan compound and quinidine are administered separated by a time interval that permits the resultant beneficial effect for treating, preventing, arresting, delaying the onset of, and/or reducing the risk of developing a behavioral disorder associated with a central nervous system (CNS) disorder in a subject. For example, in some embodiments, the dextromethorphan compound and quinidine are administered on the same day (e.g., each once or twice daily).

As used herein, the total NPI score is the composite of the scores for the standard 12 NPI domains. The NPI is a validated clinical instrument for evaluating psychopathology in a variety of disease settings, including dementia. The NPI is a retrospective caregiver-informant interview covering 12 neuropsychiatric symptom domains: delusions, hallucinations, agitation/aggression, dysphoria/depression, anxiety, euphoria/elation, apathy/indifference, disinhibition, irritability/lability, aberrant motor behaviors, nighttime behavioral disturbances, and appetite/eating disturbances. The scripted NPI interview includes a compound screening question for each symptom domain, followed by a list of interrogatives about domain-specific behaviors that is administered when a positive response to a screening question is elicited. Neuropsychiatric manifestations within a domain are collectively rated by the caregiver in terms of both frequency (0 to 4) and severity (1 to 3), yielding a composite (frequency x severity) symptom domain score of 1 to 12 for each positively endorsed domain. Frequency and severity rating scales have defined anchor points to enhance the reliability of caregiver responses. Caregiver distress is rated for each positive neuropsychiatric symptom domain on a scale anchored by scores of 0 (not distressing at all) to 5 (extremely distressing). As used herein, the NPI4A score is the composite score comprising the NPI agitation/aggression, aberrant motor behavior, irritability/lability, and anxiety domains. As used herein, the NPI4D score is the composite score comprising the NPI agitation/aggression, aberrant motor behavior, irritability/lability, and disinhibition domains.

The term "therapeutically effective" applied to dose or amount refers to that quantity of a compound or pharmaceutical composition that is sufficient to result in a desired activity upon administration to a subject in need thereof. As used herein with respect to pharmaceutical compositions comprising a dextromethorphan compound, the term "therapeutically effective amount/dose" is used interchangeably with the term "neurologically effective amount/dose" and refers to the amount/dose of a compound or pharmaceutical composition that is sufficient to produce an effective neurological response, i.e., improvement of a behavioral disorder associated with a CNS disorder, upon administration to a subject.

The phrase "pharmaceutically acceptable," as used in connection with compositions of the disclosure, refers to molecular entities and other ingredients of such compositions that are physiologically tolerable and do not typically produce untoward reactions when administered to a subject (e.g., human). In some embodiments, the term "pharmaceutically acceptable" means approved by a regulatory agency of a Federal or a state government or listed in the U.S. Pharmacopeia or other generally recognized pharmacopeia for use in mammals, and more particularly in humans.

The term "carrier" applied to pharmaceutical compositions of the disclosure refers to a diluent, excipient, or vehicle with which an active compound (e.g., the dextromethorphan compound or quinidine) is administered. Such pharmaceutical carriers can be sterile liquids, such as water, saline solutions, aqueous dextrose solutions, aqueous glycerol solutions, and oils, including those of petroleum, animal, vegetable, or synthetic origin, such as peanut oil, soybean oil, mineral oil, sesame oil, and the like. Suitable pharmaceutical carriers are described in "Remington's Pharmaceutical Sciences" by E. W. Martin, 18th Edition.

The term "subject" as used herein includes a mammal (e.g., a rodent such as mouse or rat). In some embodiments, the term refers to humans presenting with a behavioral disorder associated with a CNS disorder, such as, agitation and/or aggression. The term "subject" also includes a humans presenting with neuropsychiatric symptoms or behavioral symptoms of dementia.

It will be recognized that some variation of natural isotopic abundance occurs in a synthesized compound depending upon the origin of chemical materials used in the synthesis. Thus, a preparation of dextromethorphan will inherently contain small amounts of deuterated and/or ¹³C-containing isotopologues. The concentration of naturally abundant stable hydrogen and carbon isotopes, notwithstanding this variation, Is small and immaterial as compared to the degree of stable isotopic substitution of compounds of this disclosure. See, for instance, Wada E et al., Seikagaku 1994, 66:15; Ganes L Z et al., Comp Biochem Physiol A Mol Integr Physiol 1998, 119:725. In a compound of this disclosure, when a particular position is designated as having deuterium, it is understood that the abundance of deuterium at that position is substantially greater than the natural abundance of deuterium, which is generally about 0.015%. A position designated as having deuterium typically has a minimum isotopic enrichment factor of at least 3000 (45% deuterium incorporation) at each atom designated as deuterium in said compound.

The term "isotopic enrichment factor" as used herein means the ratio between the isotopic abundance and the natural abundance of a specified isotope.

In other embodiments, a compound of this disclosure has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

In the compounds of this disclosure any atom not specifically designated as a particular isotope is meant to represent any stable isotope of that atom. Unless otherwise stated, when a position is designated specifically as "H" or "hydrogen," the position is understood to have hydrogen at its natural abundance isotopic composition.

The term "isotopologue" refers to a species that has the same chemical structure and formula as a specific compound of this disclosure, with the exception of the isotopic composition at one or more positions, e.g., H vs. D. Thus an isotopologue differs from a specific compound of this disclosure in the isotopic composition thereof.

The term "compound," as used herein, is also intended to include any salts, solvates, or hydrates thereof. The term "dextromethorphan compound" will be used for ease of use in this application, and will include the following terms: dextromethorphan, or deuterated dextromethorphan, or an analog or a derivative of dextromethorphan, or an analog or derivative of deuterated dextromethorphan.

In general, the dextromethorphan compounds of the present disclosure have four molecular rings in a configuration known as a "morphinan" structure, which is depicted as follows:

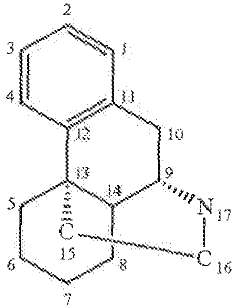

In this depiction, the carbon atoms are conventionally numbered as shown, and the wedge-shaped bonds coupled to carbon atoms 9 and 13 indicate that those bonds rise out of the plane of the three other rings in the morphinan structure.

A salt of a compound of this disclosure is formed between an acid and a basic group of the compound, such as an amino functional group, or a base and an acidic group of the compound, such as a carboxyl functional group. According to another embodiment, the compound is a pharmaceutically acceptable acid addition salt.

Acids commonly employed to form pharmaceutically acceptable salts include inorganic acids such as hydrogen bisulfide, hydrochloric acid, hydrobromic acid, hydroiodic acid, sulfuric acid, and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid, and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesutfonate, propanesulfonate, naphthalene-1-sulfonate, naphthalene-2-sulfonate, mandelate, and other salts. In one embodiment, pharmaceutically acceptable acid addition salts include those formed with mineral acids such as hydrochloric acid and hydrobromic acid, and those formed with organic acids such as maleic acid.

In some embodiments, the pharmaceutically acceptable salts include alkali metals, salts of lithium, salts of sodium, salts of potassium, salts of alkaline earth metals, salts of calcium, salts of magnesium, salts of lysine, salts of N,N'dibenzylethylenediamine, salts of chloroprocaine, salts of choline, salts of diethanolamine, salts of ethylenediamine, salts of meglumine, salts of procaine, salts of tris, salts of free acids, salts of free bases, inorganic salts, salts of sulfate, salts of hydrochloride, and salts of hydrobromide.

Unless otherwise specified, the doses described herein refer to the hydrobromide and sulfate salt forms of the dextromethorphan compound and quinidine, respectively. Based on such information, those skilled in the art can calculate corresponding dosages for the respective free-acid or free-base forms of the active ingredient. A person of skill in the art can calculate the molecular weight for the salt of dextromethorphan and the molecular weight for free base of dextromethorphan and use the ratio to calculate appropriate dosages for the free base as well as for the salt. For example, a dose of 15 mg dextromethorphan hydrobromide (of molecular formula C₁₈H₂₆NO.HBr.H₂O) and 9 mg quinidine sulfate (of molecular formula (C₂₀H₂₄N₂O₂)₂.H₂SO₄.2H₂O) may be administered or used (corresponding to approximately 11 mg dextromethorphan and 7.5 mg quinidine). Other dosages include, for example, 23 mg dextromethorphan hydrobromide and 9 quinidine sulfate (corresponding to approximately 17 mg dextromethorphan and approximately 7.5 mg quinidine); 20 mg dextromethorphan hydrobromide and 10 quinidine sulfate (corresponding to approximately 15 mg dextromethorphan and 8.3 mg quinidine); 30 mg dextromethorphan hydrobromide and 10 quinidine sulfate (corresponding to approximately 22 mg dextromethorphan and 8.3 mg quinidine).

A dose of 24 mg d6-deuterated dextromethorphan hydrobromide (of molecular formula C₁₈H₁₉D₆NO.HBr.H₂O) and 4.75 mg quinidine sulfate (of molecular formula (C₂₃H₂₄N₂O₂)₂.H₂SO₄.2H₂O) may be administered (corresponding to approximately 18 mg dextromethorphan and 3.96 mg quinidine). Other dosages for d6-deuterated dextromethorphan include, for example, 34 mg d6-dextromethorphan hydrobromide and 4.75 quinidine sulfate (corresponding to approximately 25.18 mg d6-dextromethorphan and approximately 3.96 mg quinidine); 18 mg d6-dextromethorphan hydrobromide and 4.9 quinidine sulfate (corresponding to approximately 13.33 mg d6-dextromethorphan and 4.08 mg quinidine); 24 mg d6-dextromethorphan hydrobromide and 4.9 quinidine sulfate (corresponding to approximately 17.78 mg d6-dextromethorphan and 4.08 mg quinidine); 28 mg d6-dextromethorphan hydrobromide and 4.9 quinidine sulfate (corresponding to approximately 20.74 mg d6-dextromethorphan and 4.08 mg quinidine); 30 mg d6-dextromethorphan hydrobromide and 4.9 quinidine sulfate (corresponding to approximately 22.22 mg d6-dextromethorphan and 4.08 mg quinidine); 34 mg d6-dextromethorphan hydrobromide and 4.9 quinidine sulfate (corresponding to approximately 25.18 mg d6-dextromethorphan and 4.08 mg quinidine).

As used herein, the term "hydrate" means a compound which further includes a stoichiometric or non-stoichiometric amount of water bound by non-covalent intermolecular forces.

As used herein, the term "solvate" means a compound which further includes a stoichiometric or non-stoichiometric amount of solvent such as water, acetone, ethanol, methanol, dichloromethane, 2-propanol, or the like, bound by non-covalent intermolecular forces.

The compounds of the present disclosure (e.g., compounds of Formula I described below), may contain an asymmetric carbon atom, for example, as the result of deuterium substitution or otherwise. As such, compounds of this disclosure can exist as either individual enantiomers, or mixtures of the two enantiomers. Accordingly, a compound of the present disclosure will include both racemic mixtures, and also individual respective stereoisomers that are substantially free from another possible stereoisomer. The term "substantially free of other stereoisomers" as used herein means less than 25% of other stereoisomers, in some embodiments less than 10% of other stereoisomers, in some embodiments less than 5% of other stereoisomers, and in some embodiments less than 2% of other stereoisomers, or less than "X"% of other stereoisomers (wherein X is a number between 0 and 100, inclusive) are present. Methods of obtaining or synthesizing an individual enantiomer for a given compound are well known in the art and may be applied as practicable to final compounds or to starting material or intermediates.

The term "stable compounds," as used herein, refers to compounds which possess stability sufficient to allow for their manufacture and which maintain the integrity of the compound for a sufficient period of time to be useful for the purposes detailed herein (e.g., formulation into therapeutic products, intermediates for use in production of therapeutic compounds, isolatable or storable intermediate compounds, treating a disease or condition responsive to therapeutic agents).

"D" refers to deuterium.

"Stereoisomer" refers to both enantiomers and diastereomers.

Throughout this specification, a variable may be referred to generally (e.g., "each R") or may be referred to specifically (e.g., R¹ or R²). Unless otherwise indicated, when a variable is referred to generally, it is meant to include all specific embodiments of that particular variable.

The term "comprising" as used herein is synonymous with "including," "containing," or "characterized by," and is inclusive or open-ended and does not exclude additional, unrecited elements or method steps.

All numbers expressing quantities of ingredients, reaction conditions, and so forth used in the specification are to be understood as being modified in all instances by the term "about." Accordingly, unless indicated to the contrary, the numerical parameters set forth herein are approximations that may vary depending upon the desired properties. At the very least, and not as an attempt to limit the application of the doctrine of equivalents to the scope of any claims in any application claiming priority to the present application, each numerical parameter should be construed in light of the number of significant digits and ordinary rounding approaches.

### Therapeutic Compounds

Dextromethorphan (DM), the non-opioid d-isomer of the codeine analog levorphanol, has been extensively used for about 50 years as an over-the-counter (OTC) antitussive agent. DM has a complex pharmacology, with binding affinity to a number of different receptors, with primary activity in the central nervous system (CNS). DM is well known for its activity as a weak uncompetitive N-methyl-D-aspartate (NMDA) receptor antagonist (Kᵢ = 1500 nM), (Tortella et al. Trends Pharmacol Sci. 1989;10(12):501-7; Chou YC et al., Brain Res. 1999;821(2):516-9; Netzer R et al., Eur J Pharmacol. 1993;238(2-3):209-16; Jaffe DB et al., Neurosci Lett. 1989;105(1-2):227-32) with the associated potential for anti-glutamate excitatory activity. DM is also a potent sigma-1 agonist (Zhou GZ et al., Eur J Pharmacol. 1991;206(4):261-9; Maurice T et al., Brain Res Brain Res Rev. 2001;37(1-3):116-32; Cobos EJ et al., Curr Neuropharmacol. 2008;6(4):344-66), (Kᵢ = 200 nM) and binds with high affinity to the serotonin transporter (SERT; Kᵢ = 40 nM). Although DM has only a moderate affinity for the norepinephrine transporter (Kᵢ = 13 µM), it effectively inhibits uptake of norepinephrine (Kᵢ = 240 nM) (Codd EE et al., J Pharmacol Exp Ther. 1995;274(3):1263-70). DM is an antagonist of α3β4 nicotinic acetylcholine receptors, with a reported IC50 (concentration resulting in 50% inhibition) value of 0.7 µM (Damaj et al., J Pharmacol Exp Ther. 2005;312(2):780-5).

As a result of one or more of these interactions, DM decreases potassium-stimulated glutamate release (Annels SJ et al., Brain res. 1991;564(2):341-3), and modulates monoamine (serotonin, norepinephrine, and dopamine) neurotransmission (Codd EE et al., J Pharmacol Exp Ther. 1995;274(3):1263-70; Maurice T et al., Pharmacol Ther. 2009;124(2):195-206; Maurice T et al., Prog Neuropsychopharmacol Biol Psychiatry. 1997;21(1):69-102). DM's antagonism of α3β4 nicotinic acetylcholine receptors (Damaj Ml et al., J Pharmacol Exp Ther. 2005;312(2):780-5) may have implications for certain CNS movement disorders and addiction (Silver AA et al., J Am Acad Child Adolesc Psychiatry. 2001;40(9):1103-10). When administered alone, DM is rapidly metabolized in the liver primarily to dextrorphan (DX) resulting in exceedingly low bioavailability and thus limiting CNS exposure. Although DX interacts with some of the same receptors as DM, but with differing affinities for key receptors, it undergoes rapid glucuronide conjugation, which largely prevents it from crossing the blood-brain barrier, thus reducing CNS effects at prescribed doses (Church J et al., Eur J Pharmacol. 1985;111 (2):185-90; Franklin PH et al., Mol Pharmacol. 1992;41(1): 134-46).

The present disclosure provides a compound of Formula I, including pharmaceutically acceptable salts, solvates, and hydrates thereof: 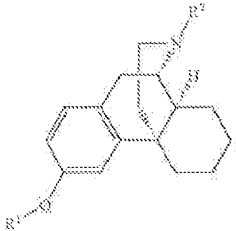 wherein, R¹ is selected from CH₃, CH₂D, CHD₂, and CD₃; and R² is selected from CH₃, CH₂D, CHD₂, and CD₃.

All compounds according to Formula I that have at least one deuterium are called deuterated dextromethorphan. In one embodiment both R¹ and R² are CD₃. This compound is also referred to as d6-dextromethorphan or d6-DM. In another embodiment, only one out of R¹ and R² is CD₃.

In yet another embodiment, the compound is selected from any one of the compounds set forth in Table 1.

**Table 1: Exemplary Compounds of Formula I**

| Compound No. | R¹ | R² |
|---|---|---|
| 100 | CD₃ | CH₃ |
| 101 | CD₃ | CD₃ |
| 102 | CD₂H | CD₃ |
| 103 | CD₃ | CD₂H |
| 104 | CH₃ | CD₃ |
| 105 | CH₂D | CH₂D |
| 106 | CH₂D | CD₃ |
| 107 | CD₃ | CH₂D |
| 108 | CH₃ | CH₃ |

In another set of embodiments, any atom not designated as deuterium in any of the embodiments set forth above or below is present at its natural isotopic abundance.

In another set of embodiments, the compound of Formula I is isolated or purified, e.g., the compound of Formula I is present at a purity of at least 50% by weight (e.g., at least 55%, 60%, 65%, 70%, 75%, 80%, 85%, 90%, 95%, 97%, 98%, 98.5%, 99%, 99.5% or 99.9%) of the total amount of isotopologues of Formula I present, respectively. Thus, in some embodiments, a composition comprising a compound of Formula I can include a distribution of isotopologues of the compound, provided at least 50% of the isotopologues by weight are the recited compound.

In some embodiments, any position in the compound of Formula I designated as having D has a minimum deuterium incorporation of at least 45% (e.g., at least 52.5%, at least 60%, at least 67.5%, at least 75%, at least 82.5%, at least 90%, at least 95%, at least 97%, at least 99%, or at least 99.5%) at the designated position(s) of the compound of Formula I. Thus, in some embodiments, a composition comprising a compound of Formula I can include a distribution of isotopologues of the compound, provided at least 45% of the isotopologues include a D at the designated position(s).

In some embodiments, a compound of Formula I is "substantially free of other isotopologues of the compound, e.g., less than 50%, less than 25%, less than 10%, less than 5%, less than 2%, less than 1%, or less than 0.5% of other isotopologues are present.

The synthesis of compounds of Formula I can be readily achieved by synthetic chemists of ordinary skill. Relevant procedures and intermediates are disclosed, for instance in Kim H C et al., Bioorg Med Chem Lett 2001, 11:1651 and Newman A H et al., J Med Chem 1992, 35:4135.

Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure.

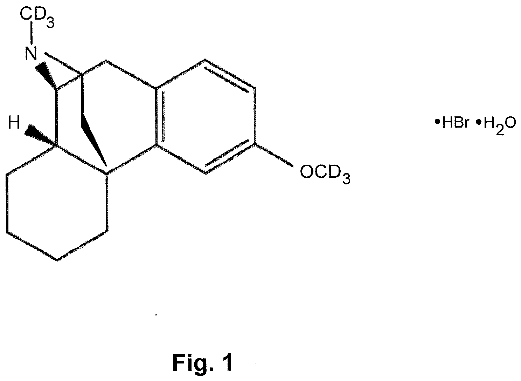

Deuterated (d6)-DM (d6-DM) (Figure 1) is a deuterated isotope of DM in which deuterium replaces 6 hydrogen atoms at specific locations. Deuterium is a stable, nonradioactive isotope of hydrogen that is ubiquitous in the environment, including in water. Stable isotopes are commonly used in medical research, with a long history of use in individuals of all ages, from neonates to adults. Deuterium is most commonly used in PK and metabolism studies at tracer levels. Levels of exposure anticipated during chronic administration of deuterated therapeutic drugs are also expected to be quite small compared to daily exposure from natural sources such as water. For example, the exposure that would result from a 30 mg dose of d6-DM is estimated to be approximately equivalent to 20 mL (0.7 oz) of water. This is based on the 0.0156% natural abundance of deuterium in water, and assumptions that all of the d6-DM (MW = 277:43) dose consumed would be metabolized to d3-dextrorphan (d3-DX), with the release of deuterated water (MW = 20.03). The latter assumption overestimates the amount of deuterated water released, but provides an idea of the comparative amount of deuterium being consumed.

Pharmacology studies conducted by the inventors with deuterated DM have demonstrated that deuteration does not alter the basic pharmacology of DM. PK and drug metabolism studies indicate d6-DM is metabolized by the same metabolic pathways as DM. Metabolism by CYP2D6 was most susceptible to inhibition by substitution with deuterium. In vitro metabolism studies also indicate that metabolism bf d6-DM and DM (by various species, including human) produces the same metabolites, i.e., no unique metabolites of d6-DM were identified compared to DM. A tissue distribution study showed similar distribution of radiolabel from ¹⁴C-labeled d6-DM (¹⁴C-d6-DM) as from ¹⁴C-labeled DM (¹⁴C-DM). Toxicology studies with d6-DM indicated that, at comparable exposures, the dose-limiting toxicities (DLTs) of d6-DM and DM are comparable and are based on adverse clinical signs associated with CNS effects. Toxicity measures assessed in a 13-week toxicity study with d6-DM/Q, which included a high dose of DM/Q for comparison, showed similar findings for d6-DM/Q as for non-deuterated DM/Q.

### Exemplary Synthesis

A convenient method for synthesizing compounds of Formula I substitutes the appropriate deuterated intermediates and reagents in synthesis methods utilized for the preparation of dextromethorphan. These methods are described, for example, in U.S. Patent No. 7,973,049, which is incorporated by reference in its entirety. Compounds of Formula I may be prepared from one of the known intermediates X, XI, and XII shown below, and from related intermediates that may be readily obtained from known procedures.

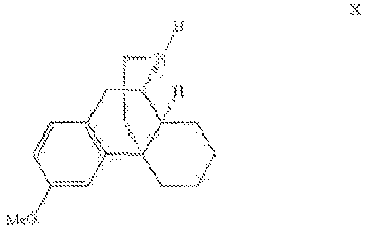

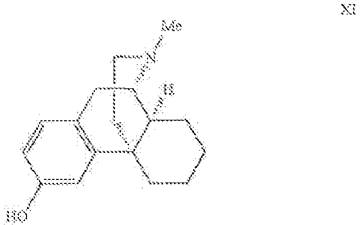

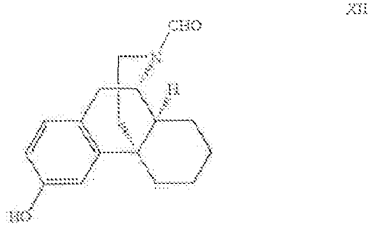

Scheme 1 shows a general route to the compounds of Formula I.

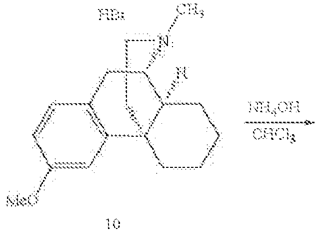

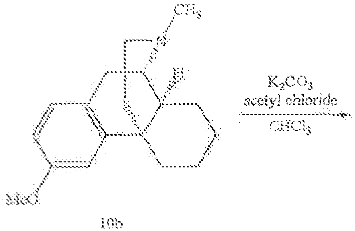

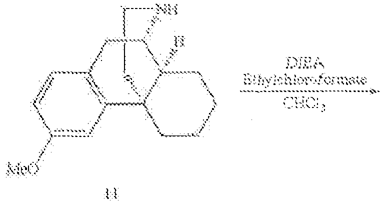

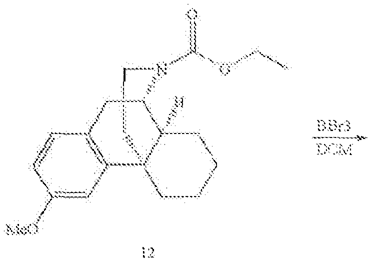

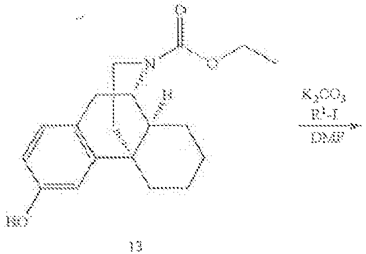

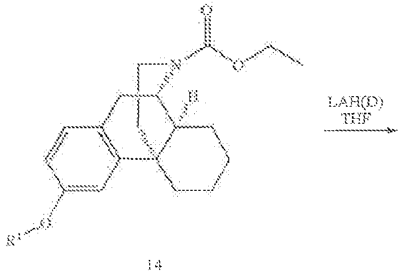

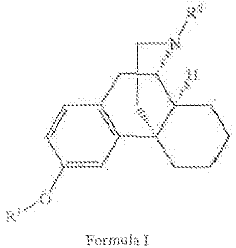

Scheme 1 shows a general route for preparing compounds of Formula I wherein R¹ is not CH₃. The HBr salt, 10, after treatment with NH₄OH, is N-demethylated to yield 11. Acylation of the amine 11 using the ethylchloroformate provides the carbamate 12, which is then O-demethylated using BBr₃ to yield the alcohol 13. Compound 13 is treated, in the presence of base, with an appropriately deuterated iodomethane to yield the ether 14, which is reduced using either lithium aluminum deuteride (LAD) to yield compounds of Formula I wherein R²=CD₃, or lithium aluminum hydride (LAH) to yield compounds wherein R²=CH₃. For those compounds wherein R¹ is CH₃, carbamate 12 is directly treated with LAD to produce a compound where R² is CD₃.

The specific approaches and compounds shown above are not intended to be limiting. The chemical structures in the schemes herein depict variables that are hereby defined commensurately with chemical group definitions (moieties, atoms, etc.) of the corresponding position in the compound formulae herein, whether identified by the same variable name (i.e., R¹ or R²) or not. The suitability of a chemical group in a compound structure for use in the synthesis of another compound is within the knowledge of one of ordinary skill in the art.

Additional methods of synthesizing compounds of Formula I and their synthetic precursors, including those within routes not explicitly shown in schemes herein, are within the means of chemists of ordinary skill in the art. Synthetic chemistry transformations and protecting group methodologies (protection and deprotection) useful in synthesizing the applicable compounds are known in the art and include, for example, those described in: Larock R, Comprehensive Organic Transformations, VCH Publishers (1989); Greene T W et al., Protective Groups in Organic Synthesis, 3rd Ed., John Wiley and Sons (1999); Fieser L et al., Fieser and Fieser's Reagents for Organic Synthesis, John Wiley and Sons (1994); and Paquette L, ed., Encyclopedia of Reagents for Organic Synthesis, John Wiley and Sons (1995); and subsequent editions thereof.

Combinations of substituents and variables envisioned by this disclosure are those that result in the formation of stable compounds.

### Analogs or Derivatives of Deuterated Dextromethorphan and Dextromethorphan.

It should be understood that the dextromethorphan compounds used in the methods and combination of this disclosure include analogs or derivatives of both dextromethorphan and deuterated dextromethorphan. For instance, in one embodiment, the combinations or methods according to this disclosure include deuterated dextromethorphan and in another embodiment, the combinations and methods include analogs or derivatives of deuterated dextromethorphan. Similarly, in another embodiment, the combinations or methods according to this disclosure include dextromethorphan and in another embodiment, the combinations or methods include analogs or derivatives of dextromethorphan.

The term "alkyl," as used herein, means any unbranched or branched, substituted or unsubstituted, saturated hydrocarbon. The alkyl moiety may be a branched or linear chain. The alkyl group may have 1 to 10 carbon atoms (whenever it appears herein, a numerical range such as "1 to 10" refers to each integer in the given range; e.g., "1 to 10 carbon atoms" means that the alkyl group may consist of 1 carbon atom, 2 carbon atoms, 3 carbon atoms, etc., up to and including 10 carbon atoms, although the present definition also covers the occurrence of the term "alkyl" where no numerical range is designated).

The term "substituted" has its ordinary meaning, as found in numerous contemporary patents from the related art. See, for example, U.S. Patent Nos. 6,509,331; 6,506,787; 6,500,825; 5,922,683; 5,886,210; 5,874,443; and 6,350,759; all of which are incorporated herein in their entirety by reference. Specifically, the definition of substituted is as broad as that provided in U.S. Patent No. 6,509,331, which defines the term "substituted alkyl" such that it refers to an alkyl group, in some embodiments of from 1 to 10 carbon atoms having from 1 to 5 substituents, and in some embodiments 1 to 3 substituents, selected from the group consisting of alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyacylamino, cyano, halogen, hydroxyl, carboxyl, carboxylalkyl, keto, thioketo, thiol, thioalkoxy, substituted thioalkoxy, aryl, aryloxy, heteroaryl, heteroaryloxy, heterocyclic, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl, and -SO-heteroaryl. The other above-listed patents also provide standard definitions for the term "substituted" that are well-understood by those of skill in the art.

The term "cycloalkyl" refers to any non-aromatic hydrocarbon ring, in some embodiments having five to twelve atoms comprising the ring. The term "heterocycle" or "heterocyclic" refer to any to non-aromatic hydrocarbon rings in which at least one heteroatom, e.g., oxygen, sulfur, or nitrogen atom, is in the ring along with at least one carbon atom.

The term "alkenyl," as used herein, means any unbranched or branched, substituted or unsubstituted, unsaturated hydrocarbon containing a double bond between two carbons. The term "alkynyl" as used herein, means any unbranched or branched substituted or unsubstituted, unsaturated hydrocarbon containing a triple bond between two carbons.

The terms "aryl" and "heteroaryl," as used herein, refer to aromatic hydrocarbon rings, in some embodiments having five, six, or seven atoms, and in other embodiments having six atoms comprising the ring. "Heteroaryl" refers to aromatic hydrocarbon rings in which at least one heteroatom, e.g., oxygen, sulfur, or nitrogen atom, is in the ring along with at least one carbon atom. The term "heterocycle" or "heterocyclic" refer to any cyclic compound containing one or more heteroatoms. The aryls, heterocycles, and heteroaryls can be substituted with any substituent, including those described above and those known in the art.

The substituent "R" appearing by itself and without a number designation refers to a substituent selected from the group consisting of alkyl, cycloalkyl, aryl, heteroaryl (bonded through a ring carbon), and heteroalicyclyl (bonded through a ring carbon).

The term "aminoalkyl" refers to a substituent selected from the group consisting of -RNR'R", -RNHR', and -RNH₂, with R, R', and R" independently being as R is defined herein.

The term "halogen atom," as used herein, means any one of the radio-stable atoms of column 7 of the Periodic Table of the Elements, e.g., fluorine, chlorine, bromine, or iodine.

The term "alkoxy" refers to any unbranched, or branched, substituted or unsubstituted, saturated or unsaturated ether, for example C₁-C₆ unbranched, saturated, unsubstituted ethers, methoxy, and dimethyl, diethyl, methyl-isobutyl, and methyl-tert-butyl ethers.

Several analogs of dextromethorphan have been discovered which may slow or prevent the metabolism of dextromethorphan. In some embodiments, the hydrogen at the 2-position of the aryl ring, R₁, can be replaced with a bulkier group.

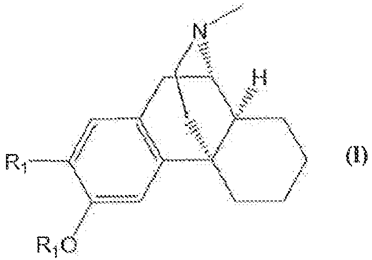

As used herein, the term "bulky" or "bulkier" refers to a substituent with a larger cone angle than hydrogen. Suitable substituents include but are not limited to C₁-C₁₀ alkyl, C₂-C₁₀ alkenyl, C₂-C₁₀ alkynyl, C₈-C₁₂ aryl, C₇-C₁₃ arylalkyl, C₃-C₁₀ cycloalkyl, C₂-C₁₀ heterocycle, C₃-C₁₀ heterocyclealkyl, C₃-C₁₂ heteroaryl, C₄-C₁₂ heteroarylalkyl, alkylamino, and dialkylamino, wherein each hydrogen is optionally substituted with a lower alkyl, an alkoxy, or a halogen.

While not wanting to be bound to any particular theory, it is believed that the introduction of a bulkier group may block CYP2D6 from initiating the catalytic process that converts dextromethorphan compounds to dextrorphan and other metabolites. As a result, the rapid metabolism of the dextromethorphan analogs, including deuterated analogs, may be prevented or drastically reduced. For example, a *t*-butyl alkyl group, with its relatively large cone angle, may protect the methyl group bonded to the oxygen and prevent the O-demethylation of dextromethorphan or deuterated dextromethorphan. Alternatively, if the bulky group contains basic nitrogen, the presence of such nitrogen 5 to 7 angstroms away from the methoxy group may prevent oxidation by inhibiting CYP2D6.

Suitable analogs with bulky groups at position 2 include compounds (1-1) and (1-2), shown below.

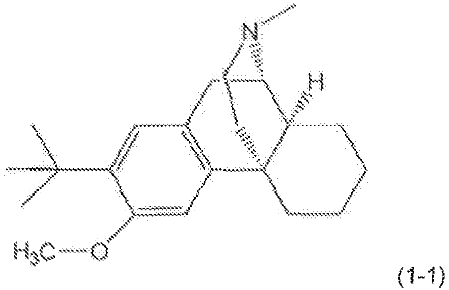

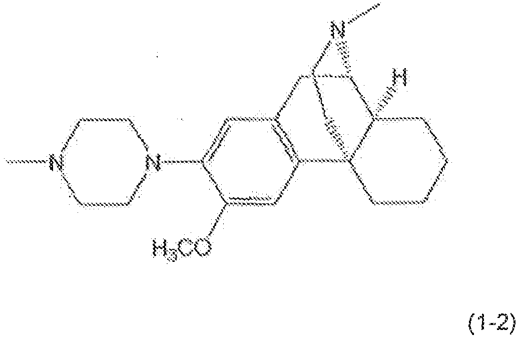

In other embodiments, the methoxy group at the 3-position may be substituted with a bulkier group, OR₂.

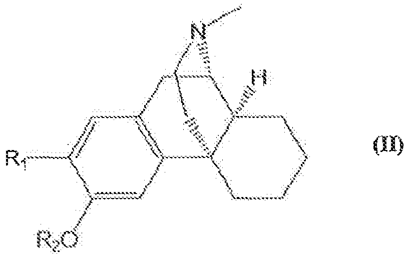

Suitable substituents include but are not limited to the following: R₁ is selected from the group consisting of hydrogen, C₁-C₁₀ alkyl, C₂-C₁₀ alkenyl, C₂-C₁₀ alkynyl, C₆-C₁₂ aryl, C₇-C₁₃ arylalkyl, C₃-C₁₀ cycloalkyl, C₂-C₁₀ heterocycle, C₃-C₁₀ heterocyclealkyl, C₃-C₁₂ heteroaryl, C₄-C₁₂ heteroarylalkyl, alkylamino, and dialkylamino, wherein each hydrogen is optionally substituted with a lower alkyl, an alkoxy or a halogen; and R₂ is selected from the group consisting C₂-C₁₀ alkyl, C₂-C₁₀ alkenyl, C₂-C₁₀ alkynyl, C₆-C₁₂ aryl, C₇-C₁₃ arylalkyl, C₃-C₁₀ cycloalkyl, C₂-C₁₀ heterocycle, C₄-C₁₀ heterocyclealkyl, C₃-C₁₂ heteroaryl, C₄-C₁₂ heteroarylalkyl, alkylamino, and dialkylamino, wherein each hydrogen is optionally substituted with a lower alkyl, an alkoxy, or a halogen. When R₁ is hydrogen, R₂ cannot be either hydrogen or methyl in order to exclude dextromethorphan and dextrorphan.

Suitable analogs with bulky -OR₂ group at the 3-position include compounds (II-1) - (II-4), shown below.

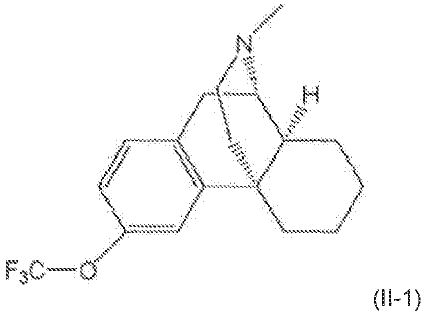

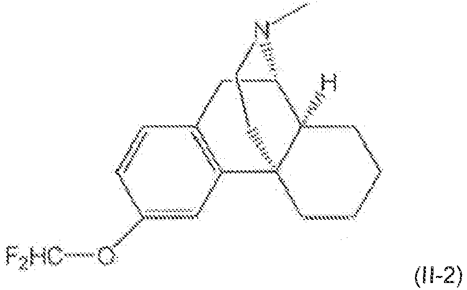

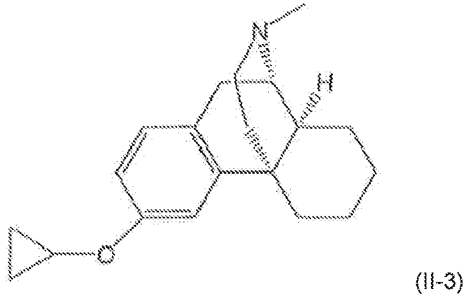

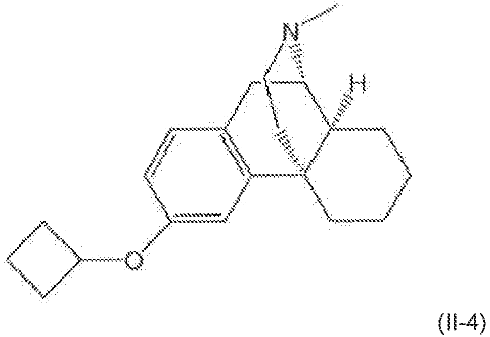

In still other embodiments, the methoxy group at the 3-position may be substituted with a bulkier group that includes a sulfur atom.

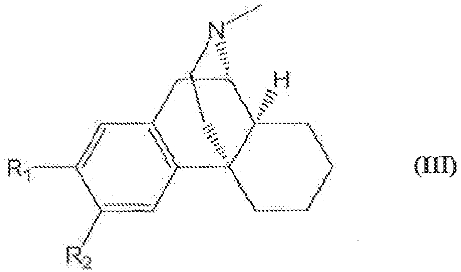

Suitable substituents include but are not limited to the following: each R₁ is independently selected from the group consisting of hydrogen, C₁-C₁₀ alkyl, C₂-C₁₀ alkenyl, C₂-C₁₀ alkynyl, C₆-C₁₂ aryl, C₇-C₁₃ arylalkyl, C₃-C₁₀ cycloalkyl, C₂-C₁₀ heterocycle, C₃-C₁₀ heterocyclealkyl, C₃-C₁₂ heteroaryl, C₄-C₁₂ heteroarylalkyl, alkylamino, and dialkylamino, wherein each hydrogen is optionally substituted with a lower alkyl, an alkoxy or a halogen; and R₂ is selected from the group consisting of -SR₁,

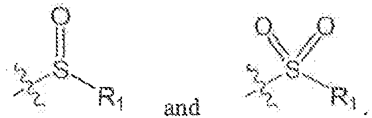

Suitable analogs with sulfur containing bulky group at the 3-position include compounds (III-1) - (III-3), shown below.

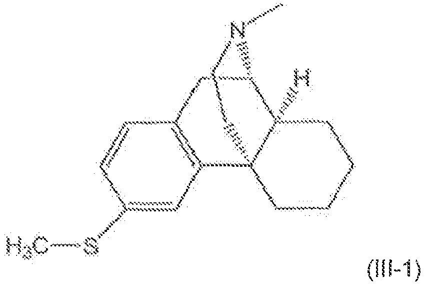

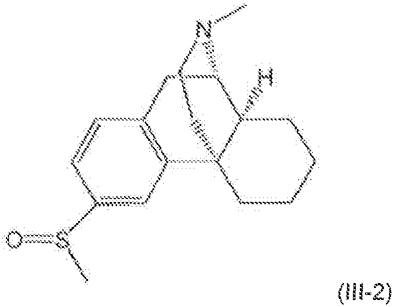

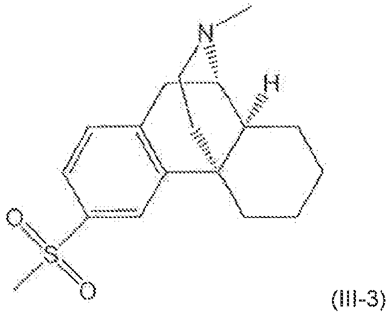

While not wanting to be bound to any particular theory, it is believed that the introduction a bulkier group that contains either an oxygen or sulfur atom at the 3- position may improve the half-life (t_{1/2}) of dextromethorphan or deuterated dextromethorphan. By increasing the half-life, the concentration of the dextromethorphan analogs in the blood may be increased over a longer period of time before it is metabolized to dextrorphan. Alternative substituents and substitutions are also within the spirit of the scope of this invention. Particularly, substituents and substitutions that include one or more deuterium atoms, for example d6DM, are within the scope of this disclosure. A person of ordinary skill in the art would know how to modify the dextromethorphan analogs of the present invention to provide deuterated dextromethorphan analogs.

Also included within the scope of this disclosure are compounds described by Newman et al. in J Med Chem. 1992 Oct 30;35(22):4135-42. These compounds were identified by Newman as exhibiting anticonvulsant activity in a variety of *in vitro* and *in vivo* models of convulsive action.

### CYP2D6 Inhibitors

The present disclosure envisions the use of a dextromethorphan compound along with a CYP2D6 inhibitor, such as quinidine. While quinidine is most commonly used for coadministration, other antioxidants, such as those described in Inaba et al., Drug Metabolism and Disposition. 1985;13:443-447, Forme-Pfister et al., Biochem. Pharmacol. 1988;37:3829-3835, and Broly et al., Biochem. Pharmacol. 1990;39:1045-1053, can also be co-administered with the dextromethorphan compound to reduce its metabolism. As reported in Inaba et al., CYP2D6 inhibitors with a Ki value (Michaelis-Menton inhibition value) of 50 micromolar or lower include nortriptyline, chlorpromazine, domperidone, haloperidol, pipamperone, labetalol, metaprolol, oxprenolol, propranolol, timolol, mexiletine, quinine, diphenhydramine, ajmaline, lobeline, papaverine, and yohimbine. Compounds having particularly potent inhibitory activities include yohimbine, haloperidol, ajmaline, lobeline, and pipamperone, which have Kᵢ values ranging from 4 to 0.33 µM. In addition to the antioxidants reported above, it has also been found that fluoxetine, sold by Eli Lilly and Co. under the trade name Prozac®, is effective in increasing dextromethorphan concentrations in the blood of some people. In addition, any of the following compounds may be used to inhibit CYP2D6: terbinafine, cinacalcet, buprenorphine, imipramine, bupropion, ritonavir, sertraline, duloxetine, thioridazine, metoclopramide, paroxetine, or fluvoxamine. Dosages of other antioxidants will vary with the antioxidant, and are determined on an individual basis.

It has been unexpectedly discovered that subjects suffering from agitation and/or aggression in Alzheimer's disease can be treated with a dextromethorphan compound in combination with an amount of quinidine substantially lower than the minimum amount heretofore believed to be necessary to provide a significant therapeutic effect.

Metabolism of dextromethorphan or its deuterated form may be further circumvented by co-administration of a CYP2D6 inhibitor along with the dextromethorphan. Quinidine is a potent CYP2D6 inhibitor, and has been particularly studied in this use (U.S. Pat. No. 5,206,248 to Smith). The chemical structure of quinidine is as follows:

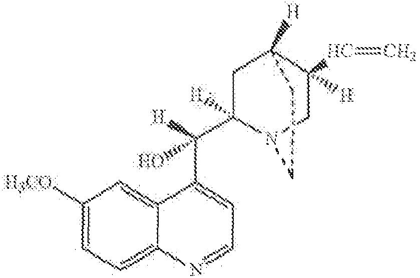

Quinidine co-administration has at least two distinct beneficial effects. First, it greatly increases the quantity of the dextromethorphan compounds circulating in the blood. In addition, it also yields more consistent and predictable dextromethorphan concentrations. Research involving dextromethorphan or co-administration of quinidine and dextromethorphan, and the effects of quinidine on blood plasma concentrations, are described in the patent literature (see, e.g., U.S. Pat. No. 5,166,207, U.S. Pat. No. 5,863,927, U.S. Pat. No. 5,366,980, U.S. Pat. No. 5,206,248, U.S. Pat. No. 5,350,756, and U.S. Pat. No. 7,973,049).

Quinidine administration can convert subjects with extensive metabolizer phenotype to poor metabolizer phenotype (Inaba et al., Br. J. Clin. Pharmacol. 1986; 22: 199-200). Blood levels of dextromethorphan increase linearly with dextromethorphan dose upon co-administration with quinidine, but are undetectable in most subjects given dextromethorphan alone, even at high doses (Zhang et al. Clin. Pharmac. & Therap. 1992;51:647-55). The observed plasma levels in rapid metabolizers following dextromethorphan co-administered with quinidine thus mimic the plasma levels observed in poor metabolizers. Accordingly, doctors should be cautious about administering quinidine to subjects who may be poor metabolizers.

### Pharmaceutical Compositions

One of the characteristics of the disclosed methods and combinations is that they function to reduce agitation and/or aggression in subjects with Alzheimer's disease. In some instances this reduction is achieved without tranquilizing or otherwise significantly interfering with consciousness or alertness, and/or without increasing the risk of serious adverse effects. As used herein, "significant interference" refers to adverse events that would be significant either on a clinical level (e.g., they would provoke a specific concern in a doctor or psychologist) or on a personal or social level (such as by causing drowsiness sufficiently severe that it would impair someone's ability to drive an automobile). In contrast, the types of very minor side effects that can be caused by an over-the-counter drug, such as a dextromethorphan-containing cough syrup, when used at recommended dosages are not regarded as significant interference.

The magnitude of a therapeutic dose of a dextromethorphan compound in combination with quinidine in the acute or chronic management of agitation and/or aggression in subjects with Alzheimer's disease can vary with factors, such as, the particular cause of the condition, the severity of the condition, and the route of administration. The dose and/or the dose frequency can also vary according to the age, body weight, and response of the individual subject.

In one embodiment, the dextromethorphan compound and quinidine are administered in a combined dose, or in separate doses. The separate doses may be administered substantially simultaneously. In one embodiment, the weight ratio of the dextromethorphan compound to quinidine is about 1:1 or less. In some embodiments, the weight ratio is about 1:1, 1:0.95, 1:0.9, 1:0.85, 1:0.8, 1:0.75, 1:0.7, 1:0.65, 1:0.6, 1:0.55 or 1:0.5 or less. Likewise, in certain embodiments, dosages have a weight ratio of the dextromethorphan compound to quinidine less than about 1:0.5, for example, about 1:0.45, 1:0.4, 1:0.35, 1:0.3, 1:0.25, 1:0.2, 1:0.15, or 1:0.1, 1:0.09, 1:0.08, 1:0.07, 1:0.06, 1:0.05, 1:0.04, 1:0.03, 1:0.02, or 1:0.01, or less. The weight ratios can be for example, about 1:0.75, about 1:0.68, about 1:0.6, about 1:0.56, about 1:0.5, about 1:0.44, about 1:0.39, about 1:0.38, about 1:0.31, about 1:0.30, about 1:0.29, about 1:0.28, about 1:0.27, about 1:0.26, about 1:0.25, about 1:0.24, about 1:0.23, about 1:0.22, about 1:0.21, about 1:0.20, about 1:0.19, about 1:0.18, about 1:0.17, 1:0.16, about 1:0.15, about 1:0.14, about 1:0.13, about 1:0.12, about 1:0.11 and about 1:0.10. In some embodiments, the weight ratios for free base of dextromethorphan to free base of quinidine is about 1:0.68, about 1:0.56, about 1: 0.44, about 1:0.38. In certain other embodiments, the weight ratios for free base of d6-deuterated dextromethorphan to free base of quinidine is about 1: 0.30, about 1:0.22, about 1: 0.19, about 1: 0.18, about 1:0.16, and about 1:0.15.

In certain embodiments, when the dextromethorphan compound and quinidine are administered at a weight ratio of 1:1 or less, less than 50 mg quinidine is administered at any one time. For example, in certain embodiments, quinidine is administered at about 30, 25, or 20 mg or less. In other embodiments, quinidine is administered at about 15, 10, 9.5, 9.0, 8.5, 8.0, 7.5, 7.0, 6.5, 6.0, 5.5, 5.0, or less. In other embodiments, quinidine is administered at about 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05, or less.

In some embodiments, the combined dose (or separate doses simultaneously administered) at a weight ratio of 1:1 or less is administered once daily, twice daily, three times daily, four times daily, or more frequently so as to provide the subject with a certain dosage level per day, for example: 60 mg quinidine and 60 mg dextromethorphan compound per day provided in two doses, each dose containing 30 mg quinidine and 30 mg dextromethorphan compound; 50 mg quinidine and 50 mg dextromethorphan compound per day provided in two doses, each dose containing 25 mg quinidine and 25 mg dextromethorphan compound; 40 mg quinidine and 40 mg dextromethorphan compound per day provided in two doses, each dose containing 20 mg quinidine and 20 mg dextromethorphan compound; 30 mg quinidine and 30 mg dextromethorphan compound per day provided in two doses, each dose containing 15 mg quinidine and 15 mg dextromethorphan compound; or 20 mg quinidine and 20 mg dextromethorphan compound per day provided in two doses, each dose containing 10 mg quinidine (i.e., about 9 mg of quinidine free base) and 10 mg dextromethorphan compound. In some embodiments, the total amount of dextromethorphan compound and quinidine in a combined dose may be adjusted, depending upon the number of doses to be administered per day, so as to provide a suitable daily total dosage to the subject, while maintaining a weight ratio of 1:1 or less.

In some embodiments, the total daily dose for the dextromethorphan compound in combination with quinidine, for the treatment of agitation and/or aggression in subjects with Alzheimer's disease, is about 10 mg or less up to about 200 mg or more dextromethorphan compound in combination with about 0.05 mg or less up to about 60 mg or more quinidine. In some embodiments, a daily dose for treating agitation and/or aggression in subjects with Alzheimer's disease is about 10 mg to about 90 mg dextromethorphan compound in combination with about 2.5 mg to about 60 mg quinidine, in single or divided doses. In some embodiments, the total daily dose of dextromethorphan compound is about 15, 16, 17, 18, 19 or 20 mg in combination with about 15, 10, 9.5, 9.0, 8.5, 8.0, 7.5, 7.0, 6.5, 6.0, 5.5, 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine.

In some embodiments, the daily dose for treating agitation and/or aggression in subjects with Alzheimer's disease is about 20, 21, 22, 23, 24, 25, 26, 27, 28, 29, or 30 mg dextromethorphan compound in combination with about 15, 10, 9.5, 9.0, 8.5, 8.0, 7.5, 7.0, 6.5, 6.0, 5.5, 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine; or about 30, 31, 32, 33, 34, 35, 36, 37, 38, 39, or 40 mg dextromethorphan compound in combination with about 15, 10, 9.5, 9.0, 8.5, 8.0, 7.5, 7.0, 6.5, 6.0, 5.5, 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine; or about 40, 41, 42, 43, 44, 45, 46, 47, 48, 49, or 50 mg dextromethorphan compound in combination with about 15, 10, 9.5, 9.0, 8.5, 8.0, 7.5, 7.0, 6.5, 6.0, 5.5, 5.00; 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine; or about 50, 51, 52, 53, 54, 55, 56, 57, 58, 59, or 60 mg dextromethorphan compound in combination with about 15, 10, 9.5, 9.0, 8.5, 8.0, 7.5, 7.0, 6.5, 6.0, 5.5, 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine; or about 60, 61, 62, 63, 64, 65, 66, 67, 68, 69, or 70 mg dextromethorphan compound in combination with about 15, 10, 9.5, 9.0, 8.5, 8.0, 7.5, 7.0, 6.5, 6.0, 5.5, 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine; or about 70, 71, 72, 73, 74, 75, 76, 77, 78, 79 or 80 mg dextromethorphan compound in combination with about 15, 10, 9.5, 9.0, 8.5, 8.0, 7.5, 7.0, 6.5, 6.0, 5.5, 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.60, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine; or about 80, 81, 82, 83, 84, 85, 86, 87, 88, 89, 90 mg dextromethorphan compound in combination with about 15, 10, 9.5, 9.0, 8.5, 8.0, 7.5, 7.0, 6.5, 6.0, 5.5, 5.00, 4.95, 4.90, 4.85, 4.80, 4.75, 4.70, 4.65, 4.60, 4.55, 4.50, 4.45, 4.40, 4.35, 4.30, 4.25, 4.20, 4.15, 4.10, 4.05, 4.00, 3.95, 3.90, 3.85, 3.80, 3.75, 3.70, 3.65, 3.60, 3.55, 3.50, 3.45, 3.40, 3.35, 3.30, 3.25, 3.20, 3.15, 3.10, 3.05, 3.00, 2.95, 2.90, 2.85, 2.80, 2.75, 2.70, 2.65, 2.60, 2.55, 2.50, 2.45, 2.40, 2.35, 2.30, 2.25, 2.20, 2.15, 2.10, 2.05, 2.00, 1.95, 1.90, 1.85, 1.80, 1.75, 1.70, 1.65, 1.80, 1.55, 1.50, 1.45, 1.40, 1.35, 1.30, 1.25, 1.20, 1.15, 1.10, 1.05, 1.00, 0.95, 0.90, 0.85, 0.80, 0.75, 0.70, 0.65, 0.60, 0.55, 0.50, 0.45, 0.40, 0.35, 0.30, 0.25, 0.20, 0.15, 0.10, or 0.05 mg or less of quinidine; in single or divided doses.

In some embodiments, the daily dose of dextromethorphan compound and quinidine is 30 mg dextromethorphan hydrobromide and 30 mg quinidine sulfate. Other dosages include, for example, 15 mg dextromethorphan hydrobromide and 9 mg quinidine sulfate (corresponding to approximately 11 mg dextromethorphan and approximately 7.5 mg quinidine); 23 mg dextromethorphan hydrobromide and 9 quinidine sulfate (corresponding to approximately 17 mg dextromethorphan and approximately 7.5 mg quinidine); 20 mg dextromethorphan hydrobromide and 10 quinidine sulfate (corresponding to approximately 15 mg dextromethorphan and 8.3 mg quinidine); 30 mg dextromethorphan hydrobromide and 10 quinidine sulfate (corresponding to approximately 22 mg dextromethorphan and 8.3 mg quinidine).

A dose of 30 mg d6-dextromethorphan hydrobromide (of molecular formula C₁₈H₁₉D₆NO.HBr.H₂O) and 30 mg quinidine sulfate (of molecular formula (C₂₂H₂₄N₂O₂)₂.H₂SO₄.2H₂O) may be administered or used (corresponding to approximately 22.22 mg dextromethorphan and 25 mg quinidine). Other dosages for d6-deuterated dextromethorphan include, for example, 24 mg d6-dextromethorphan hydrobromide and 4.75 mg quinidine sulfate (corresponding to approximately 18 mg d6-dextromethorphan and approximately 3.96 mg quinidine); 34 mg d6-dextromethorphan hydrobromide and 4.75 quinidine sulfate (corresponding to approximately 25.18 mg d6-dextromethorphan and approximately 3.96 mg quinidine); 18 mg d6-dextromethorphan hydrobromide and 4.9 quinidine sulfate (corresponding to approximately 13.33 mg d6-dextromethorphan and 4.08 mg quinidine); 24 mg d6-dextromethorphan hydrobromide and 4.9 quinidine sulfate (corresponding to approximately 17.78 mg d6-dextromethorphan and 4.08 mg quinidine); 28 mg d6-dextromethorphan hydrobromide and 4.9 quinidine sulfate (corresponding to approximately 20.74 mg d6-dextromethorphan and 4.08 mg quinidine); 30 mg d6-dextromethorphan hydrobromide and 4.9 quinidine sulfate (corresponding to approximately 22.22 mg d6-dextromethorphan and 4.08 mg quinidine); 34 mg d6-dextromethorphan hydrobromide and 4.9 quinidine sulfate (corresponding to approximately 25.18 mg d6-dextromethorphan and 4.08 mg quinidine).

In some embodiments, the therapy is initiated at a lower daily dose, for example about 18 or 30 mg dextromethorphan compound in combination with about 2.5 to 10 mg quinidine per day, and increased up to about 90 mg dextromethorphan compound in combination with about 10 to 20 mg quinidine, or higher, depending on the subject's global response. In some embodiments, infants, children, subjects over 65 years, and those with impaired renal or hepatic function, initially receive low doses, that may be titrated based on individual response(s) and blood level(s). Generally, a daily dosage of 18 to 90 mg dextromethorphan compound and 4.75 to 20 mg quinidine is well-tolerated by most subjects.

As will be apparent to those skilled in the art, dosages outside of these disclosed ranges may be administered in some cases. Further, it is noted that the ordinary skilled clinician or treating physician will know how and when to interrupt, adjust, or terminate therapy in consideration of individual response.

Any suitable route of administration can be employed for providing the subject with an effective dosage of a dextromethorphan compound in combination with quinidine for treating agitation and/or aggression in subjects with Alzheimer's disease. For example, oral, rectal, transdermal, parenteral (subcutaneous, intramuscular, intravenous), intrathecal, topical, inhalable, and like forms of administration can be employed. Suitable dosage forms include tablets, troches, dispersions, suspensions, solutions, capsules, patches, and the like. Administration of medicaments prepared from the compounds described herein can be by any suitable method capable of introducing the compounds into the bloodstream. In some embodiments, the formulations can contain a mixture of active compounds with pharmaceutically acceptable carriers or diluents known to those of skill in the art.

The pharmaceutical compositions disclosed herein comprise a dextromethorphan compound in combination with a CYP2D6 inhibitor, such as quinidine, or pharmacoutically acceptable salts of the dextromethorphan compound and/or quinidine, as active ingredients, and can also contain a pharmaceutically acceptable carrier, and optionally, other therapeutic ingredients.

The terms "pharmaceutically acceptable salts" or "a pharmaceutically acceptable salt thereof refer to salts prepared from pharmaceutically acceptable, non-toxic acids or bases. Suitable pharmaceutically acceptable salts, include metallic salts, e.g., salts of aluminum, zinc; alkali metal salts such as lithium, sodium, and potassium salts; alkaline earth metal salts such as calcium and magnesium salts; organic salts, e.g., salts of lysine, N,N'-dibenzylethylenediamine, chloroprocaine, choline, diethanolamine, ethylenediamine, meglumine (N-methylglucamine), procaine, and tris; salts of free acids and bases; inorganic salts, e.g., sulfate, hydrochloride, and hydrobromide; and other salts which are currently in widespread pharmaceutical use and are listed in sources well known to those of skill in the art, such as The Merck Index.

Any suitable constituent can be selected to make a salt of an active drug discussed herein, provided that it is non-toxic and does not substantially interfere with the desired activity. In addition to salts, pharmaceutically acceptable precursors and derivatives of the compounds can be employed. Pharmaceutically acceptable amides, lower alkyl esters, and protected derivatives of deuterated dextromethorphan and/or quinidine can also be suitable for use in the compositions and methods disclosed herein. In certain embodiments, the deuterated dextromethorphan is administered in the form of deuterated dextromethorphan hydrobromide, the dextromethorphan is administered as dextromethorphan hydrobromide, and the quinidine is administered in the form of quinidine sulfate.

The compositions can be prepared in any desired form, for example, tablets, powders, capsules, injectables, suspensions, sachets, cachets, patches, solutions, elixirs, and aerosols. Carriers such as starches, sugars, microcrystalline cellulose, diluents, granulating agents, lubricants, binders, disintegrating agents, and the like can be used in oral solid preparations. In certain embodiments, the compositions are prepared as oral solid preparations (such as powders, capsules, and tablets). In certain embodiments, the compositions are prepared as oral liquid preparations. In some embodiments, the oral solid preparations are tablets. If desired, tablets can be coated by standard aqueous or nonaqueous techniques.

In addition to the dosage forms set out above, the compounds disclosed herein can also be administered by sustained release, delayed release, or controlled release compositions and/or delivery devices, for example, such as those described in U.S. Pat. Nos. 3,845,770; 3,916,899; 3,536,809; 3,598,123; and 4,008,719.

Pharmaceutical compositions suitable for oral administration can be provided as discrete units such as capsules, cachets, sachets, patchs, injectables, tablets, and aerosol sprays, each containing predetermined amounts of the active ingredients, as powder or granules, or as a solution or a suspension in an aqueous liquid, a non-aqueous liquid, an oil-in-water emulsion, or a water-in-oil liquid emulsion. Such compositions can be prepared by any of the conventional methods of pharmacy, but the majority of the methods typically include the step of bringing into association the active ingredients with a carrier that constitutes one or more ingredients. In general, the compositions are prepared by uniformly and intimately admixing the active ingredients with liquid carriers, finely divided solid carriers, or both, and then, optionally, shaping the product into the desired presentation.

For example, a tablet can be prepared by compression or molding, optionally, with one or more additional ingredients. Compressed tablets can be prepared by compressing in a suitable machine the active ingredient in a free-flowing form such as powder or granules, optionally mixed with a binder, lubricant, inert diluent, surface active or dispersing agent. Molded tablets can be made by molding, in a suitable machine, a mixture of the powdered compound moistened with an inert liquid diluent.

In some embodiments, each tablet contains from about 30 mg dextromethorphan hydrobromide and 30 mg quinidine sulfate. The tablet may include, for example, 15 mg dextromethorphan hydrobromide and 9 mg quinidine sulfate (corresponding to approximately 11 mg dextromethorphan and approximately 7.5 mg quinidine); 23 mg dextromethorphan hydrobromide and 9 quinidine sulfate (corresponding to approximately 17 mg dextromethorphan and approximately 7.5 mg quinidine); 20 mg dextromethorphan hydrobromide and 10 quinidine sulfate (corresponding to approximately 15 mg dextromethorphan and 8.3 mg quinidine); 30 mg dextromethorphan hydrobromide and 10 quinidine sulfate (corresponding to approximately 22 mg dextromethorphan and 8.3 mg quinidine).

The tablet may include 30 mg d6-dextromethorphan hydrobromide (of molecular formula C₁₈H₁₉D₆NO.HBr.H₂O) and 30 mg quinidine sulfate (of molecular formula (C₂₀H₂₄N₂O₂)₂.H₂SO₄.2H₂O) (corresponding to approximately 22.22 mg dextromethorphan and 25 mg quinidine). The tablet may contain other dosages, for example, 24 mg d6-dextromethorphan hydrobromide and 4.75 mg quinidine sulfate (corresponding to approximately 18 mg d6-dextromethorphan and approximately 3.96 mg quinidine); 34 mg d6-dextromethorphan hydrobromide and 4.75 quinidine sulfate (corresponding to approximately 25.18 mg d6-dextromethorphan and approximately 3.96 mg quinidine); 18 mg d6-dextromethorphan hydrobromide and 4.9 quinidine sulfate (corresponding to approximately 13.33 mg d6-dextromethorphan and 4.08 mg quinidine); 24 mg d6-dextromethorphan hydrobromide and 4.9 quinidine sulfate (corresponding to 17.78 mg d6-dextromethorphan and 4.08 mg quinidine); 28 mg d6-dextromethorphan hydrobromide and 4.9 quinidine sulfate (corresponding to approximately 20.74 mg d6-dextromethorphan and 4.08 mg quinidine); 30 mg d6-dextromethorphan hydrobromide and 4.9 quinidine sulfate (corresponding to approximately 22.22 mg d6-dextromethorphan and 4.08 mg quinidine); 34 mg d6-dextromethorphan hydrobromide and 4.9 quinidine sulfate (corresponding to approximately 25.18 mg d6-dextromethorphan and 4.08 mg quinidine).

As used herein, a "minimum effective therapeutic amount" is that amount which provides a satisfactory degree of inhibition of the rapid elimination of the dextromethorphan compound from the body, while producing no adverse effect or only adverse events of an acceptable degree and nature. More specifically, in some embodiments, an effective therapeutic amount is within the range of from about 9, 10, 18, 20, 25 or 30 mg to about 90 mg of dextromethorphan compound and less than about 50 mg of quinidine per day. In some embodiments, the effective therapeutic amount is within a range from about 20 or 30 mg to about 90 mg of dextromethorphan compound and about 0.5 mg to about 30 mg of quinidine per day. In some embodiments, the amount is administered in a divided dose based on the plasma half-life of the dextromethorphan compound. For example, in one embodiment deuterated dextromethorphan and quinidine are administered in specified mg increments to achieve a target concentration of deuterated dextromethorphan of a specified level in µg/mL plasma, with a maximum specified dosage of deuterated dextromethorphan and quinidine based on body weight. In some embodiments, the target dose is administered every 12 hours. In some embodiments, the target dose is administered once daily. In some embodiments, the level of quinidine is minimized and the side effects observed at high dosages for quinidine are minimized or eliminated, providing a significant benefit over compositions containing the dextromethorphan compound in combination with higher levels of quinidine.

In some embodiments, other therapeutic agents are administered in combination with the dextromethorphan compound and quinidine. For example, the dextromethorphan compound and quinidine may be administered in combination with a compound to treat depression or anxiety.

In some embodiments, the dextromethorphan compound and quinidine are administered as an adjuvant to known therapeutic agents for treating symptoms of Alzheimer's disease. Agents for treating symptoms of Alzheimer's disease include, but are not limited to, cholinesterase inhibitors such as donepezil, rivastigmine, galantamine and tacrine, memantine and Vitamin E.

### EXAMPLES

### Example 1: Agitation and Aggression in Alzheimer's Disease Clinical Study

A clinical study was conducted to determine if the combination of dextromethorphan and quinidine was effective in reducing agitation and/or aggression in subjects with Alzheimer's disease.

This investigation was a 10-week, randomized, double-dummy, placebo-controlled, multi-center study of the efficacy of oral dextromethorphan/quinidine in subjects with probable Alzheimer's disease and clinically significant agitation. The study was conducted at 42 U.S. sites, including outpatient Alzheimer's disease clinics and assisted living and nursing facilities.

Eligible participants were aged 50 to 90 years with probable Alzheimer's disease (2011 National Institute on Aging-Alzheimer Association criteria) and clinically significant agitation defined as a state of poorly organized and purposeless psychomotor activity characterized by at least one of the following: aggressive verbal (e.g., screaming, cussing); aggressive physical (e.g., destroying objects, grabbing, fighting); and nonaggressive physical (e.g., pacing, restlessness) behaviors. Eligible participants had agitation (intermittently or constantly) within 7 days prior to screening and the agitation symptoms had to be severe enough such that they interfered with daily routine and warranted pharmacological treatment. Eligible participants also scored ≥4 (moderately ill) on the Clinical Global Impression of Severity of Illness scale (CGIS) for agitation, and had a Mini Mental State Examination (MMSE) score of 8 to 28. Stable doses of Alzheimer's disease medications (≥2 months; memantine and/or acetylcholinesterase inhibitors), and antidepressants, antipsychotics, or hypnotics (≥1 month; including short-acting benzodiazepines and nonbenzodiazepines) were allowed; dosages were to remain stable throughout the study. Oral lorazepam (maximum 1.5 mg/day and maximum 3 days in a 7-day period) was allowed during the study as "rescue" medication for agitation if deemed necessary by the study investigator.

Exclusion criteria were non-Alzheimer's disease dementia, agitation not secondary to Alzheimer disease, hospitalization in a mental health facility, significant depression (Cornell Scale for Depression in Dementia [CSDD] ≥10), ' schizophrenia, schizoaffective or bipolar disorder, myasthenia gravis (because quinidine use is contraindicated), or clinically significant/unstable systemic disease; history of complete heart block, corrected change in QT interval (QTc) prolongation or torsades de pointes; family history of congenital QT prolongation; history of postural or unexplained syncope within the last year; or substance/alcohol abuse within 3 years. First generation antipsychotics, tricyclic and monoamine oxidase inhibitor antidepressants were not allowed.

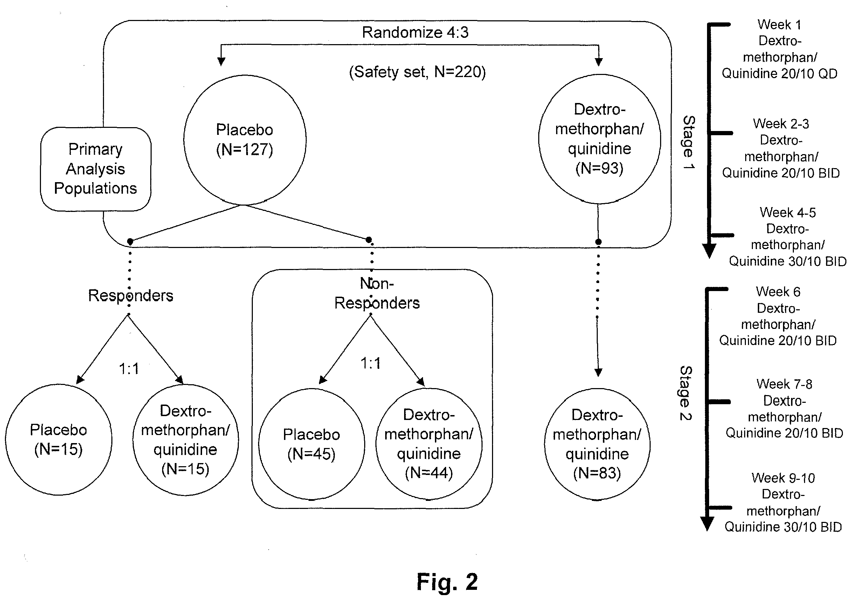

The 10-week trial had 2 consecutive double-blind 5-week stages (Stage 1 and Stage 2) (Figure 2). Participants were randomized into Stage 1 in a 3:4 (active:placebo) ratio. Randomization in Stage 1 was stratified by baseline cognitive function (MMSE >15 vs ≥15) and agitation severity (CGIS 4-5 vs 6-7); blocked randomization ensured treatment balance in each stratum. For the initial 7 days of Stage 1 (Days 1-7), the active treatment group received AVP-923-20 (20 mg dextromethorphan and 10 mg quinidine) in the morning and placebo in the evening and the placebo group received placebo twice a day. For the following 2 weeks (Days 8-21) of Stage 1, the AVP-923 group received AVP-923-20 twice a day and the placebo group received placebo twice a day. On day 22 the dose of medication was increased for the AVP group to AVP-923-30 (30 mg dextromethorphan and 10 mg quinidine) twice a day. The AVP group continued to receive AVP-923-30 twice a day for the remaining 2 weeks of Stage 1 (Days 22-35) and participants receiving placebo continued to receive placebo twice a day.

In Stage 2, participants who received AVP-923 in Stage 1 continued to receive AVP-923 twice daily for the entire 5 week duration. Participants who received placebo in Stage 1 were stratified into two sub-groups, depending on their clinical response assessed by their Clinical Global Impression of Severity of Illness (CGIS) scores and their Neuropsychiatric Inventory (NPI) Agitation/Aggression domain scores of agitation at the end of Stage 1 (Visit 4). Participants were considered "responders" if their CGIS score for agitation was less than 3 (mildly ill) and their NPI Agitation/Aggression domain score decreased by 25% or greater from baseline. Participants who did not meet these criteria were considered "non-responders." Each placebo sub-group (responders and non-responders) was then re-randomized in a 1:1 ratio to receive either AVP-923 or matching placebo. Participants who received placebo during Stage 1 and were re-randomized to AVP-923 in Stage 2 received AVP-923-20 in the morning and matching placebo in the evening for the initial 7 days (Stage 2, Days 36-42) of the study. Starting on Day 43, participants received AVP-923-20 twice-a-day for 2 consecutive weeks (Stage 2, Days 43-56) and starting on Day 57 participants received AVP-923-30 twice a day for the remaining 2 weeks (Stage 2, Days 57-70) until study completion.

Participants attended clinic visits at Screening, Baseline (Day 1), and on Days 8, 22, 36, 43, 57, and 70 (Visits 2-7). Including the screening phase, the length of each participant's participation in this study was approximately 14 weeks. Blood samples for measurement of drug levels in plasma were collected on Day 36 (Visit 4) and on Day 70 (Visit 7). A blood sample for cytochrome P450-2D6 (CYP2D6) genotyping was collected on Day 1 (Baseline visit).

The investigator or sponsor could discontinue a participant from the study in the event of an intercurrent illness, adverse event, other reasons concerning the health or well-being of the participant, or in the case of lack of cooperation, non-compliance, protocol violation, or other administrative reasons. In addition, participants who presented a QTc interval (Bazett-corrected QT (QTcB) or Fridericia-corrected QT (QTcF)) >500 msec (unless due to ventricular pacing) or a QTc interval change from the screening electrocardiographic (ECG) result of >60 msec at any time after randomization, was withdrawn from the study. The QTc values were assessed for clinical significance and recorded. Participants who withdrew prior to study completion were asked to return to the clinic to complete the Visit 7 (End of Study) assessments. If a participant withdrew or was discontinued from the study before completion, every effort was made to document participant outcome. If the participant withdrew from the study, and consent was withdrawn by the caregiver and/or participant's representative for disclosure of future information, no further evaluations were performed, and no additional data was collected.

Participants and caregivers were instructed that the participant should take the study medication approximately every 12 hours ± 4 hours orally with water (morning and evening). AVP-923 and placebo were provided in identically-appearing capsules and packaged in 85cc white plastic bottles with child-resistant caps, one bottle with white label for the morning dosing and one bottle with blue label for the evening dosing. The compositions of the AVP-923 and placebo capsules are given in Table 2.

**Table 2:**

| **Ingredient (amounts in mg)** | **AVP-923-30** | **AVP-923-20** | **Placebo** |
|---|---|---|---|
| Dextromethorphan hydrobromide USP, EP | 30.00 | 20.00 | 0 |
| Quinidine sulfate dihydrate USP, EP | 10.00 | 10.00 | 0 |
| Croscarmellose sodium NF | 7.80 | 7.80 | 7.80 |
| Microcrystalline cellulose NF | 94.00 | 94.00 | 94.00 |
| Colloidal silicone dioxide NF | 0.65 | 0.65 | 0.65 |
| Lactose monohydrate NF | 116.90 | 126.90 | 156.90 0.65 |
| Magnesium stearate NF | 0.65 | 0.65 | |

| | | | |
|---|---|---|---|
| EP = European Pharmacopoeia; USP = United States Pharmacopoeia; NF=National Formulary | | | |

Participants and caregivers were instructed to bring any unused study medication and empty containers to the clinic on Days 8, 22, 36, 43, 57, and 70 (Visits 2-7). For this study, compliance was defined as when a participant takes at least 80% of their scheduled doses. Caregivers were provided with diary cards and were instructed to record daily the number of capsules taken and the time of administration. Diary cards were collected on Days 8, 22, 36, 43, 57, and 70 (Visits 2-7), or at the time of early study discontinuation.

### Efficacy

The primary efficacy endpoint was an improvement in the Agitation/Aggression NPI domain. Secondary efficacy endpoints included changes from baseline in NPI total score (range: 1-144), individual NPI domain scores, and NPI composite scores comprising Agitation/Aggression, Aberrant Motor Behavior, and Irritability/Lability domains plus either Anxiety (NPI4A) or Disinhibition (NPI4D). A NPI-caregiver distress score (NPI-CDS; 0-5, not at all to very severely) was captured for each positively endorsed NPI domain. Alzheimer's Disease Cooperative Study-Clinical Global Impression of Change (ADCS-CGIC; 1-7, marked improvement to marked worsening) and Patient Global Impression of Change (PGI-C), rated by a caregiver (1-7, very much improved to very much worse) scores were assessed at weeks 5 and 10 and provided measures of clinical meaningfulness. Additional secondary endpoints included ADCS-Activities of Daily Living Inventory (ADCS-ADL; 0-54, higher scores signifying better function); CSDD (0-38, higher scores signifying more severe depression); Caregiver Strain Index (CSI; 0-13, higher scores signifying higher stress levels); Quality of Life-Alzheimer Disease (QOL-AD; 13-52, with higher scores signifying better QOL); and psychotropic medication changes/rescue use of lorazepam. Cognition was assessed using the MMSE (0-30, with lower scores signifying greater cognitive impairment) and the Alzheimer Disease Assessment Scale-Cognitive Subscale (ADAS-cog; 0-70, with higher scores signifying greater cognitive impairment). Safety outcomes included adverse events (AEs), vital signs, clinical laboratory test results, and ECG results. Results for QT interval were corrected for variation in heart rate and the QTcF (QT/³√[RR]) calculations were used.

The parameters of efficacy described above were assessed at the following time points during the study: CSI and all of the NPI domains were assessed at baseline and weeks 1, 3, 5, 6, 8, and 10; ADCS-CGIC Agitation, QOL-AD (Caregiver), and ADAS-cog were assessed at baseline and weeks 5 and 10; CSDD and MMSE were assessed at screening and weeks 5 and 10; and PGI-C was assessed at weeks 5 and 10.

Primary and secondary efficacy endpoints were analyzed based on published sequential parallel comparison design (SPCD) methods (Fava et al., Psychother. Psychosom. 2003;72(3):115-127; Chen et al., Contemp. Clin. Trials., 2011;32(4):592-604) analyzing data from both 5-week stages with 1:1 weighting using ordinary least squares (OLS), and including all participants in stage 1 and only the rerandomized placebo nonresponders (Figure 2) in stage 2. The primary study endpoint analysis was prespecified; no correction was performed to address multiplicity in the secondary endpoints. Dextromethorphan/quinidine and placebo groups were compared using 2-sided tests at the alpha = 0.05 level of significance. Additionally, Analysis of Covariance (ANCOVA) with treatment as the fixed effect and baseline as the covariate was used to compare treatment group means at each stage and visit, separately. Finally, to simulate a 10-week parallel-arm design (as shown in Figure 2), a pre-specified comparison of NPI Agitation/Aggression scores was conducted between participants who were randomized to receive only dextromethorphan/quinidine (n = 93) or only placebo (n = 66) for the entire 10 weeks of the trial (regardless of responder status). All statistical analyses were performed using SAS® version 9.1 or higher (SAS Institute, Cary, NC, USA).

Given the use of SPCD methodology, and in order to provide assurance on findings from the primary analysis, additional exploratory sensitivity analyses of the primary endpoint were carried out. One used the repeated measures model (MMRM, prespecified) described by Doros et al (Doros et al., Stat. Med. 2013;32(16):2767-2789) to test the potential impact of missing data and the exclusion of rerandomized placebo "responders" in stage 2. This model used all available data for the NPI Agitation/Aggression domain. Three separate models were used to estimate treatment effect and included data collected at baseline, end of stage 1, and end of stage 2, with a general model that allowed inclusion of data from intermediate visits. Based on FDA recommendation, the second sensitivity analysis of the primary endpoint using the Seemingly Unrelated Regression (SUR) method (Doros et al., Stat. Med. 2013;32(16):2767-2789; Zellner et al., J. Am. Stat. Assoc. 1962;57(298):348-368; Tamura and Huang, Clin. Trials. 2007;4(4):309-317) in the SPCD, instead of the OLS method, was conducted after unblinding of the study, to address whether missing data could be missing not at random. In addition to the above, a prespecified exploratory analysis of the primary endpoint was carried out that used the same SPCD methodology described above for the primary analysis, but including both placebo responders and nonresponders who were rerandomized in stage 2.

In published treatment studies for dementia-related agitation, standard deviation (SD) estimates for change in NPI Agitation/Aggression scores range from 3.1 to 5.2 points (Herrmann et al., CNS Drugs. 2011 ;25(5):425-433; Mintzer et al., Am. J. Geriatr. Psychiatry. 2007;15(11):918-931; Herrmann et al., Dement. Geriatr. Cogn. Disord. 2007;23(2):116-119). Assuming a SD of 5.0 points, and based on a 2-sided, 2-sample comparison of means from independent samples at the 5% significance level, a sample size of 196 participants was calculated to provide 90% power to detect a mean difference of 2.5 points. The sample size calculation was based on a parallel design as there was no precedent for an SPCD trial in treatment of agitation in subjects with Alzheimer disease.

The safety analysis set included all participants who took at least 1 dose of study medication. The modified intention-to-treat (mITT) analysis set for efficacy included all participants with a post baseline NPI Agitation/Aggression assessment in stage 1. Missing data were imputed using the last observation carried forward.

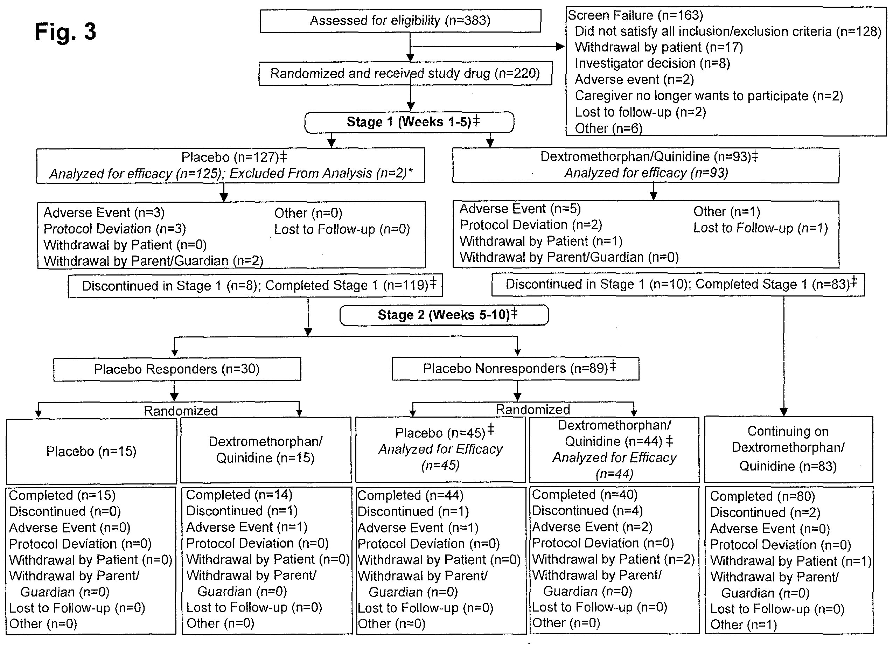

All 220 randomized participants (126 females, 94 males) were included in the safety analysis set; 218 participants composed the mITT analysis set for efficacy, and 194 (88.2%) completed the study (Figure 3). With the SPCD and rerandomization of the placebo group upon entry into Stage 2, a total of 152 participants received dextromethorphan/quinidine (93 starting from Stage 1 and an additional 49 rerandomized from placebo in Stage 2), and 127 participants received placebo, resulting in an approximately 26.7% greater exposure for dextromethorphan/quinidine (1153 patient-weeks) than for placebo (911 patient-weeks). Seventeen (11.2%) participants discontinued while receiving dextromethorphan/quinidine and 9 (7.1%) while receiving placebo, including 8 (5.3%) and 4 (3.1%) for AEs, respectively. Participant characteristics were well-balanced across treatment groups and are provided in Table 3 and Table 4 (mITT efficacy set). The rerandomized groups in Stage 2 were also well-balanced. The mITT SPCD rerandomized placebo group characteristics are provided in Table 5.

**TABLE 3:**

| **Characteristic** | **Placebo (n** = **127)^{a}** | **Dextromethorphan/quinidine (n** = **93)^{a}** |
|---|---|---|
| **Age (years), mean (SD)** | 77.8 (7.2) | 77.8 (8.0) |
| **Age ≥75 years, n (%)** | 86 (67.7) | 68 (73.1) |
| **Women, n (%)** | 74 (58.3) | 52 (55.9) |

| **Race, n (%)** | | |
|---|---|---|
| **White** | 118 (92.9) | 84 (90.3) |
| **Black or African American** | 6 (4.7) | 5 (5.4) |
| **Asian** | 1 (0.8) | 3 (3.2) |
| **Native Hawailan or Other Pacific Islander** | 0 | 1 (1.1) |
| **Other** | 2 (1.6) | 0 |

| **Ethnicity, n (%)** | | |
|---|---|---|
| **Hispanic or Latino** | 13 (10.2) | 7 (7.5) |

| **Residence, n (%)** | | |
|---|---|---|
| **Outpatient** | 111 (87.4) | 82 (88.2) |
| **Assisted living** | 10 (7.9) | 5 (5.4) |
| **Nursing home** | 6 (4.7) | 6 (6.5) |

| **Concomitant medications, n (%)** | | |
|---|---|---|
| **Acetylcholinesterase Inhibitors** | 95 (74.8) | 67 (72.0) |
| **Memantine** | 66 (52.0) | 43 (46.2) |
| **Antidepressants** | 65 (51.2) | 57 (61.3) |
| **Antipsychotics** | 29 (22.8) | 16 (17.2) |
| **Benzodiazepines** | 12 (9.5) | 6 (6.5) |
| **Benzodiazopine-like derivatives** | 12 (9,5) | 6 (6.5) |
| **History of falls, n (%)** | 16 (12.6) | 16 (17.2) |

| **Rating scale scores, mean (SD)** | | |
|---|---|---|
| **CGI-S Agitation** | 4.5 (0.7) | 4.4 (0.6) |
| **NPI Agitation/Aggression** | 7.0 (2.4 | 7.1 (2.6) |
| **NPI Total** | 38.0 (18.7) | 40.1 (19.6) |
| **NPI-Aberrant Motor Behavior** | 3.5 (4.2) | 4.3 (4.4) |
| **NPI-Irritability/Lability** | 5.4 (3.2) | 5.8 (3.7) |
| **NPI 4A** | 20.1 (8.3) | 20.9(9.4) |
| **NPI 4D** | 18.5 (9.2) | 19.8 (9.1) |
| **NPI Caregiver Distress-Agitation** | 3.0 (1.0) | 3.3 (0.9 |
| **NPI Caregiver Distress-Total** | 17.0 (8.3) | 17.9(8.0) |
| **CSI** | 6.8 (3.6) | 6.9 (3.2) |
| **CSDD** | 5.8 (2.4) | 5.9 (2.4) |
| **QOL-AD (Patient)** | 37.2 (6,4) | 36.5 (7.4) |
| **QOL-AD (Caregiver)** | 30.1 (6.0) | 30.9 (6.0) |
| **MMSE** | 17.2 (5.8) | 17.4 6.0) |
| **ADAS-cog** | 32.0 (15.2) | 30,6 (14.1) |
| **ADCS-ADL** | 34.1 (12.8) | 35.8 |

| **CGlS Agitation baseline scores,^{b} n (%)** | | |
|---|---|---|
| **4 (moderately ill)** | 77 (60.6) | 61 (65.6) |
| **5 (markedly ill)** | 40 (31.5) | 28 (30.1) |
| **6 or 7 (severely ill or among the most extremely ill patient)** | 10 (7.9) | 4 (4.3) |

| | | |
|---|---|---|
| Participant characteristics across treatment groups. ^{a}Safety analysis set at randomization; ^{b}Modified intention-to-treat analysis set for efficacy analysis (placebo, n = 125; dextromethorphan/quinidine, n = 93), | | |

**TABLE 4:**

| **Characteristic** | **Placebo** | **Dextromethorphan/quinidine** |
|---|---|---|
| **Gender** | | |
| **n** | 125 | 93 |
| **Female** | 74 (59.2%) | 52 (55.9%); |
| **Male** | 51 (40.8%) | 41 (44,1%) |

| **Race** | | |
|---|---|---|
| **n** | 125 | 93 |
| **White** | 116 (92.8%) | 84 (90.3%) |
| **Black or African American** | 6 (4.8%) | 5 (5,4%) |
| **Asian** | 1 (0.8%) | 3 (3,2%) |
| **American Indian or Alaska Native** | 0 | 0 |
| **Native Hawaiian Or Other Pacific Islander** | 0 | 1 (1.1%) |
| **Other** | 2 (1.6%) | 0 |

| **Ethnicity** | | |
|---|---|---|
| **n** | 125 | 93 |
| **Hispanic Or Latino** | 13 (10.4%) | 7 (7,5%) |
| **Not Hispanic Or Latino** | 112 (89.6%.) | 86 (92.5%) |

| **Age (years)** | | |
|---|---|---|
| **n** | 125 | 93 |
| **Mean** | 77.6 | 77.8 |
| **SD** | 7.19 | 8.01 |
| **Min** | 56 | 53 |
| **Median** | 78.0 | 78.0 |
| **Max** | 90 | 90 |

| **Age Group 2 (years)** | | |
|---|---|---|
| **n** | 125 | 93 |
| **<75** | 41 (32.8%) | 25 (26.9%) |
| **>=75** | 84 (67.2%) | 68 (73.1%) |
| **Patient Living Arrangements** | | |
| **n** | 125 | 93 |
| **Outpatient** | 109 (87.2%) | 82 (88,2%) |
| **Assisted Living** | 10 (8.0%) | 5 (5.4%) |
| **Nursing Home** | 6 (4.8%) | 6 (6.5%) |

| **CGI-8 Agitation Score** | | |
|---|---|---|
| **n** | 125 | 93 |
| **Mean** | 4.5 | 4,4 |
| **SD** | D.67 | 0.57 |
| **Min** | 4 | 4 |
| **Median** | 4.0 | 4.0 |
| **Max** | 7 | 6 |

| **CYP2D6 Metabolizer Subgroup** | | |
|---|---|---|
| **n** | 121 | 85 |
| **Poor metabolizers** | 7 (5.8%) | 9 (1.0.6%) |
| **Intermediate metabolizers** | 48 (39.7%) | 38 (44.7%) |
| **Extensive metabolizers** | 65 (53.7%) | 35 (41.2%) |
| **Ultra-rapid metabolizers** | 1 (0.8%) | 3 (3.5%) |

| | | |
|---|---|---|
| Modified Intent-to-treat (mITT) efficacy population based on Stage 1 randomization. "Extensive" metabolizers include "Normal" and "Normal or Intermediate" metabolizers | | |

**TABLE 5:**

| | | |
|---|---|---|
| **Characteristic** | **Placebo** | **Dextromethorphan/quinidine** |

| **Gender** | | |
|---|---|---|
| **n** | 45 | 44 |
| **Female** | 29 (64.4%) | 23 (52.3%) |
| **Male** | 16 (35.6%) | 21 (47.7%) |

| **Race** | | |
|---|---|---|
| **n** | 45 | 44 |
| **White** | 41 (91.1%) | 42 (95.5%) |
| **Black or African American** | 2 (4.4%) | 2 (4.5%) |
| **American Indian or Alaska Native** | 0 | 0 |
| **Other** | 2 (4.4%) | 0 |

| **Ethnicity** | | |
|---|---|---|
| **n** | 45 | 44 |
| **Hispanic Or Latino** | 6 (13.3%) | 2 (4.5%) |
| **Not Hispanic Or Latino** | 39 (86.7%) | 42 (95.5%) |

| **Age (years)** | | |
|---|---|---|
| **n** | 45 | 44 |
| **Mean** | 77.3 | 78.3 |
| **SD** | 7.02 | 7.40 |
| **Min** | 59 | 60 |
| **Median** | 78.0 | 80.0 |
| **Max** | 89 | 90 |

| **Age Group 2 (years)** | | |
|---|---|---|
| **n** | 45 | 44 |
| **<75** | 17 (37.8%) | 13 (29.5%) |
| **>=76** | 28 (62.2%) | 31 (70.5%) |

| **Patient Living Arrangements** | | |
|---|---|---|
| **n** | 45 | 44 |
| **Outpatient** | 39 (86.7%) | 41 (93.2%) |
| **Assisted Living** | 4 (8.9%) | 2 (4.5%) |
| **Nursing Home** | 2 (4.4%) | 1 (2.3%) |

| **CGI-S Agitation Score** | | |
|---|---|---|
| **n** | 45 | 44 |
| **Mean** | 4.6 | 4.6 |
| **SD** | 0.75 | 0.66 |
| **Min** | 4 | 4 |
| **Median** | 4.0 | 4.5 |
| **Max** | 7 | 6 |

| **CYP2D6 Metabolizer Subgroup** | | |
|---|---|---|
| **n** | 45 | 41 |
| **Poor metabolizers** | 2 (4.4%) | 3 (7.3%) |
| **Intermediate metabolizers** | 13 (28.9%) | 19 (46.3%) |
| **Extensive metabolizers** | 30 (66.7%) | 18 (43.9%) |
| **Ultra-rapid metabolizers** | | 1 (2.4%) |

| | | |
|---|---|---|
| Modified Intent-to-treat (miTT) Sequential Parallel Comparison Design (SPCD) Stage 2 rerandomized placebo non-responders. "Extensive" metabolizers include "Normal" and "Normal or Intermediate" metabolizers. | | |

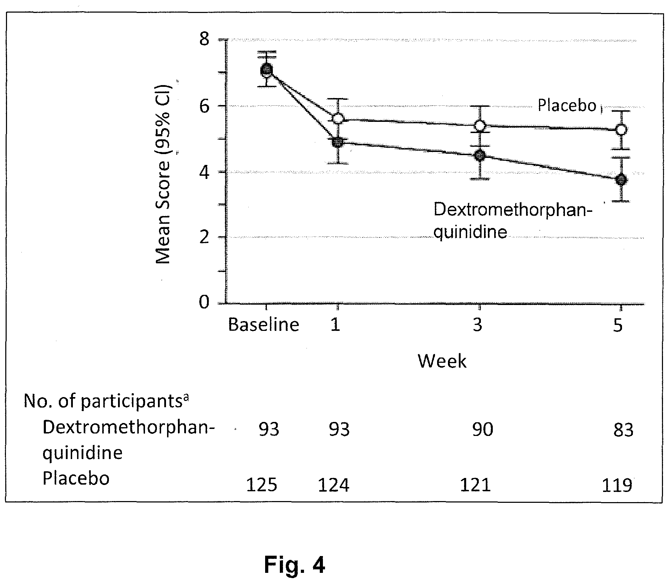

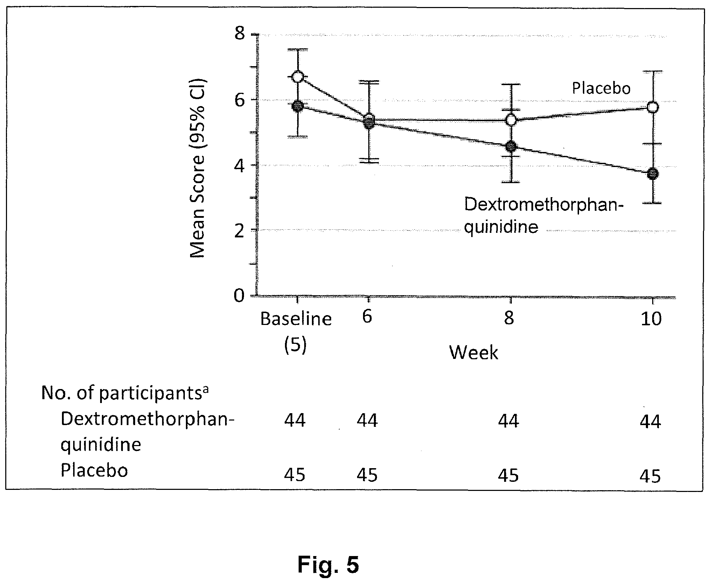

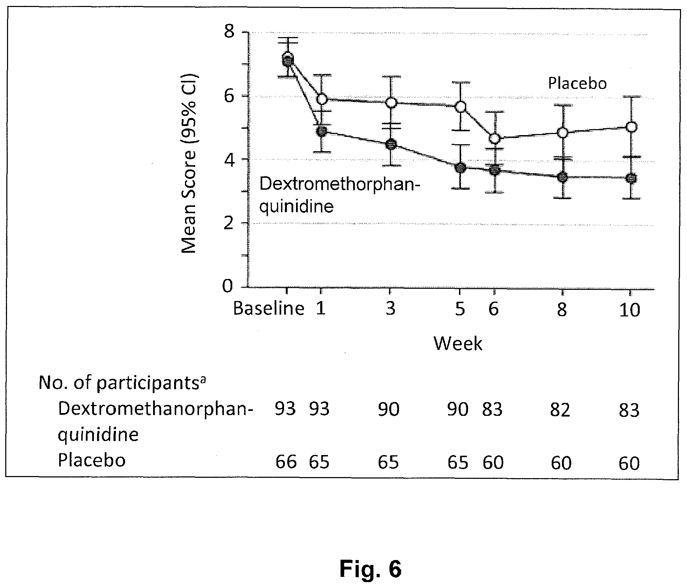

Dextromethorphan/quinidine significantly improved the NPI Agitation/Aggression score compared with placebo in the primary SPCD analysis (OLS Z-statistic: -3.95; *P* < .001) in the mITT population. Results for each stage also favored dextromethorphan/quinidine over placebo (Table 6). In stage 1, mean (95% Cl) NPI Agitation/Aggression scores were reduced from 7.1 (6.6, 7.6) to 3.8 (3.1, 4.5) with dextromethorphan/quinidine and from 7.0 (6.6, 7.4) to 5.3 (4.7, 5.9) with placebo (*P* < .001), with a least squares (LS) mean (95% Cl) treatment difference of -1.5 (-2.3, -0.7). Differential response was noted by week 1 (-0.8 [-1.5,-0.03]; *P* = .04; Figure 4. In stage 2 (placebo nonresponders rerandomized to either dextromethorphan/quinidine or placebo), mean (95% Cl) NPI Agitation/Aggression scores were reduced from 5.8 (4.9, 6.7) to 3.8 (2.9, 4.7) with dextromethorphan/quinidine and from 6.7 (5.9, 7.5) to 5.8 (4.7, 6.9) with placebo (P = .02), with an LS mean (95% Cl) treatment difference of -1.6 [-2.9, -0.3]; Figure 5). Improvement in the NPI Agitation/Aggression domain was statistically significant at week 1 and at every time point until study end, with exception of week 6 (during Stage 2). The prespecified comparison of NPI Agitation/Aggression scores between participants who were randomized to receive only dextromethorphan/quinidine (n = 93) or only placebo (n = 66) for the entire 10 weeks of the trial (regardless of responder status, simulating a parallel-arm design as shown in Figure 2), also favored dextromethorphan/quinidine over placebo (LS mean treatment difference [95% CI] of -1.8 [-2.8, -0.7]; Table 6, Figure 6). Response to dextromethorphan/quinidine compared with placebo did not appear to differ by disease stage. The stratified randomization by baseline MMSE score (>15 vs ≤15) and baseline CGIS (4 or 5 vs. 6 or 7) resulted in balanced treatment arms for both agitation and cognitive function. Supplemental analyses conducted to assess the potential influence of these factors did not suggest a difference in response, although the sizes of some strata in these analyses were small and this observation would require confirmation in larger trials.

**TABLE 6:**

| **Parameter** | **Stage** | **N/N Dextromethorphan/ quinidine/Placebo** | **Dextromethorphan/ quinidine, Mean (95% CI) Change from Baseline** | **Placebo, Mean (95% CI)** Change from **Baseline** | ***P* Value by stage^{a,b}** | **LS Mean Treatment Difference^{*} (95% CI)** | ***P* Value SPCD^{h}** |
|---|---|---|---|---|---|---|---|
| NPI-Agitation/ Aggression^{d} | 1^{a} | 93/125 | -3.3 (-3.9, -2.6) | -1.7 (-2.3, -1.2) | <.001 | -1.5 (-2.3, -0.7) | <.001 |
| | 2^{b} | 44/45 | -2.0 (-3.0, -1.0) | -0.8 (-1.9, 0.2) | 02 | -1.6 (-2.9, -0.3) | |
| | 10 wk^{c} | 93/66 | -3.6 (-4.3, -2.9) | -1.9 (-2.8, -1.0) | 001 | -1.8 (-2.8, -0.7) | N/A |
| NPI Total^{d} | 1^{a} | 93/125 | -13.5 (-17.1, -9.9) | -8.5 (-11.0, -5.9) | 03 | -4.2 (-8.0, -0.4) | 01 |
| | 2^{b} | 44/45 | -6.0 (-9.7, -2.2) | -2.5 (-6.0, 1.1) | 15 | -3.8 (-9.0, 1.4) | |
| | 10 wk^{c} | 93/66 | -16.0 (-19.5, -12.5) | -10.1 (-14.7, -5.5) | .02 | -5.7 (-10.7, -0.7) | N/A |
| NPI-Aberrant Motor Behavior^{d} | 1^{a} | 93/125 | -1.2 (-2.0,-0.4) | -0.4 (-1.1, 0.3) | .39 | -0.4 (-1.3, 0.5) | .03 |
| | 2^{b} | 44/45 | -0.8 (-1.6, -0.1) | 0.4 (-0.6, 1.3) | .04 | -1.2 (-2.4, -0.1) | |
| | 10 wk^{c} | 93/66 | -1.3 (-2.1, -0.5) | 0.1 (-0.7, 0.8) | .03 | -1.0 (-1.9, -0.1) | N/A |
| NPI-lrritability/ Lability^{d} | 1^{a} | 93/125 | -2.2 (-3.0, -1.4) | -1.2 (-1.8, -0.6) | .09 | -0.7 (-1.5, 0.1) | 0.03 |
| | 2^{b} | 44/45 | -1.0 (-2.0, 0.04) | -0.7 (-1.8, 0.5) | .14 | -0.9 (-2.2, 0.3) | |
| | 10 wk^{c} | 93/66 | -2.4 (-3.3, -1.6) | -1.8 (-2.8, -0.7) | .38 | -0.4 (-1.4, 0.6) | N/A |
| NPI4A^{d} | 1^{a} | 93/125 | -7.3 (-9.1, -5.4) | -4.5 (-6.0, -3.0) | .03 | -2.4 (-4.6, -0.2) | 001 |
| | 2^{b} | 44/45 | -4.8 (-6.9, -2.7) | -1.4 (-3.8, 1.0) | .01 | -3.9 (-7.0, -0.9) | |
| | 10 wk^{c} | 93/66 | -8.5 (-10.4, -6.7) | -5.0 (-7.4, -2.5) | .01 | -3.4 (-6.1, -0.7) | N/A |
| NPI 4D^{d} | 1^{a} | 93/125 | -7.6 (-9.4, -5.7) | -4.0 (-5.5. -2.6) | .006 | -3.0 (-5.1, -0.9) | <.001 |
| | 2^{b} | 44/45 | -4.6 (-6.8, -2.4) | -1.9 (-4.2, 0.4) | .02 | -3.5 (-6.5, -0.5) | |
| | 10 wk^{c} | 93/66 | -8.3 (-10.1, -6.5) | -5.0 (-7.4, -2.6) | .02 | -3.0 (-5.5, -0.4) | N/A |
| NPI Caregiver Distress-Agitation^{d} | 1^{a} | 93/125 | -1.4 (-1.6, -1.0) | -0.6 (-0.8, -0.4) | <.001 | -0.7 (-1.0, -0.3) | .01 |
| | 2^{b} | 44/45 | -0.5 (-0.9, -0.004) | -0.7 (-1.2, -0.2) | .49 | -0.2 (-0.8, 0.4) | |
| | 10 wk^{c} | 93/66 | N/A | N/A | N/A | N/A | N/A |
| NPI Caregiver Distress-Total^{d} | 1^{a} | 93/125 | -6.6 (-8.2, -5.0) | -3.6 (-4.8, -2.5) | N/A | N/A | .01 |
| | 2^{b} | 44/45 | -2.6 (-4.3, -1.0) | -2.0 (-3.8, -0.3) | N/A | N/A | |
| | 10 wk^{c} | 93/66 | N/A | N/A | N/A | N/A | N/A |
| CSI^{d} | 1^{a} | 93/125 | -1.2 (-1.7, -0.7) | -0.6 (-0.9, -0.2) | .03 | -0.6 (-1.2, -0.1) | .05 |
| | 2^{b} | 44/45 | -0.2 (-0.7, 0.3) | 0.1 (-0.5, 0.6) | .42 | -0.3 (-1.0, 0.4) | |
| | 10 wk^{c} | 93/66 | -1.2 (-1.7, 0.6) | -0.4 (-0.9,1.3) | .04 | -0.8 (-1.6, -0.02) | N/A |
| CSDD^{f} | 1^{a} | 88/123 | -1.0 (-1.8, -0.3) | 0.6 (-0.1, 1.3) | .002 | -1.6 (-2.5, -0.6) | .02 |
| | 2^{b} | 43/44 | -0.9 (-1.8, -0.004) | -0.7 (-1.5, 0.1) | .75 | -0.2 (-1.3, 0.9) | |
| | 10 wk^{c} | 88/64 | -1.2 (-2.0, -0.4) | 0.4 (-0.6, 1.5) | .03 | -1.3 (-2.6, -0.1) | N/A |
| ADCS-CGIC : Agitation^{e} | 1^{a} | 88/123 | 3.0 (2.8, 3.3) | 3.6 (3.4, 3.8) | | -0.6 (-0.9, -0.3) | <.001 |
| | 2^{b} | 42/42 | 3.3 (2.9, 3.6) | 3.7 (3.3, 4.2) | .07 | -0.5 (-1.0, 0.1) | |
| | 10 wk^{c} | 82/59 | 2.7 (2.3, 3.1) | 3.3 (3.0, 3.7) | .02 | -0.5 (-0.9, -0.1) | N/A |
| PGI-C^{g} | 1^{a} | 88/123 | 3.1 (2.8, 3.3) | 3.6 (3.4, 3.8) | .001 | -0.6 (-0.9, -0.2) | .001 |
| | 2^{b} | 43/44 | 3.2 (2.8. 3.6) | 3.8 (3.3, 4.2) | .04 | -0.6 (-1.1, -0.1) | |
| | 10 wk^{c} | 81/59 | 2.9 (2.7, 3.2) | 3.5 (3.2, 3.8) | .007 | -0.6 (-1.0, -0.2) | N/A |
| QOL-AD (Patient)^{e} | 1^{a} | 87/116 | 1.3 (-0.03, 2.6) | 0.0 (-1.0, 0.9) | .14 | 1.1 (-0.4, 2.6) | .16 |
| | 2^{b} | 40/40 | 1.5 (-0.1, 3.1) | 0.7 (-0.7, 2.0) | .50 | 0.7 (-1.4, 2.7) | |
| | 10 wk^{c} | 87/61 | 0.7 (-0.7, 2.1) | 0.5 (-1.1, 2.0) | .96 | -0.1 (-2.0, 1.9) | N/A |
| QOL-AD (Caregiver)^{e,i} | 1^{a} | 88/123 | 0.4 (-0.5,1.3) | 0.3 (-0.5, 1.1) | .63 | 0.3 (-0.9, 1.5) | .47 |
| | 2^{b} | 43/43 | -0.3 (-1.5, 0.9) | 0.9 (-0.4. 2.2) | .24 | 1.1 (-2.8, 0.7) | |
| | 10 wk^{c} | 88/64 | 1.3 (0.2, 2.4) | 0.9 (-0.5, 2.4) | .28 | 0.9 (-0.7, 2.6) | N/A |
| ADCS-ADL^{e} | 1^{a} | 88/123 | -0.9 (-1.8, -0.04) | -0.8 (-1.5, -0.1) | .90 | -0.1 (-1.2, 1.1) | .16 |
| | 2^{b} | 43/44 | -2.0 (-3.4, -0.5) | -0.6 (-1.7, 0.4) | .12 | -1.4 (-3.1, 0.4) | |
| | 10 wk^{c} | 88/64 | -0.8 (-1.8, 0.2) | -1.8 (-2.9, 0.7) | .17 | 1.0 (-0.5, 2.5) | N/A |
| MMSE Total Score^{f} | 1^{a} | 88/122 | 0.2 (-0.4, 0.9) | -0.3 (-0.8, 0.2) | .20 | 0.5 (-0.3, 1.3) | .05 |
| | 2 | 42/44 | 0.3 (-0.5, 1.2) | -0.5 (-1.3, 0.2) | .15 | 0.8 (-0.3, 2.0) | |
| | 10 wk^{a} | 88/63 | 0.1 (-0.5, 0.8) | -0.6 (-1.5, 0.3) | .21 | 0.7 (-0.4, 1.8) | N/A |
| ADAS-cog^{e} | 1^{a} | 87/121 | -0.9 (-2.5, 0.6) | 0.3 (-5.7, 1.3) | .11 | -1.4 (-3.0, 0.3) | .20 |
| | 2^{b} | 42/43 | 0.3 (-1.4, 1.9) | 0.8 (-0.7, 2.3) | .64 | -0.5 (-2.8, 1.7) | |
| | 10 wk^{c} | 81/58 | -0.7 (-1.9, 0.7) | 1.2 (-0.2, 2.4) | .07 | -1.7 (-3.5, 0.2) | N/A |

| | | | | | | | |
|---|---|---|---|---|---|---|---|
| *Treatment difference: dextromethorphan/quinidine - placebo; ^{a}Stage 1: Includes all participants and measures change from stage 1 baseline to week 5 for each outcome; ^{b}Stage 2: Includes only rerandomized placebo nonresponders from stage 1 and measures change from stage 2 baseline (week 5) to week 10 for all outcornes except PGI-C (original stage 1 baseline to week 10); ^{c}The 10-week analysis includes only participants who remained on their original treatment for their entire study participation (i.e., took only dextromethorphan/quinidine or only placebo. thereby simulating a parallel comparison design), and measures stage 1 baseline to week 10, ^{d}Assessed at baseline, weeks 1, 3, 5, 6, 8, and 10; ^{e}Assessed at baseline, weeks 5 and 10; ^{f}Assessed at screening, weeks 5 and 10; ^{g}Assessed at weeks 5 and 10. ^{h}SPCD (sequential parallel comparison design) analysis was protocol-specified for the primary efficacy analysis and combines results from all patients in Stage 1 and from "placebo nonresponders" re-randomized in Stage 2, based on a 50/50 weighting of the NPI agitation/aggression domain for each stage of the study; ⁱFor the QOL-AD (caregiver), the caregiver rates the patient's quality of life; *P* value by Stage based on Analysis of Covariance (ANCOVA) analysis; *P* value for SPCD analysis based on Ordinary Least Squares (OLS). | | | | | | | |

SPCD analysis of prespecified secondary outcomes (Table 6) showed significant improvement favoring dextromethorphan/quinidine on global rating scores (PGI-C and CGIC), NPI total, NPI Aberrant Motor Behavior and Irritability/Lability domains, NPI 4A and 4D composites, NPI caregiver distress (both Agitation/Aggression domain and total), CSI, and CSDD. Results for changes in QOL-AD, ADCS-ADL, MMSE, and ADAS-cog (an exploratory outcome) were not significant vs placebo. Post hoc analyses showed similar improvement in NPI Agitation/Aggression scores with dextromethorphan/quinidine in participants taking concomitant acetylcholinesterase inhibitors, memantine, antidepressants, or antipsychotics compared with those not receiving these agents. Lorazepam rescue was used by 10 of 152 (6.6%) and 13 of 125 (10.4%) participants while receiving dextromethorphan/quinidine and placebo, respectively. At the end of the 10-week treatment, 45.1% of dextromethorphan/quinidine-only treated participants (n=82) were judged to be "much improved" or "very much improved" on ADCS-CGIC vs 27.1% of participants who took only placebo (n=59).

### Safety and tolerability

Dextromethorphan/quinidine was generally well tolerated in this population receiving multiple concomitant medications and was not associated with cognitive impairment. Treatment-emergent adverse events (TEAEs) were attributed based on treatment assignment at the time of occurrence. TEAEs were reported by 93 of 152 (61.2%) and 55 of 127 (43.3%) participants (safety set) during treatment with dextromethorphan/quinidine or placebo, respectively. The most commonly occurring TEAEs (>3%) were fall (8.6% vs 3.9%), diarrhea (5.9% vs 3.1%), urinary tract infection (5.3% vs 3.9%), dizziness (4.6% vs 2.4%) and agitation (3.3% vs 4.7%) for dextromethorphan/quinidine vs placebo, respectively. Serious adverse events (SAEs) occurred in 12 (7.9%) of participants receiving dextromethorphan/quinidine and in 6 (4.7%) receiving placebo. SAEs in participants receiving dextromethorphan/quinidine included chest pain (n = 2), anemia, acute myocardial infarction (occurring 2 days after dosing ended), bradycardia, kidney infection, femur fracture, dehydration, colon cancer, cerebrovascular accident, aggression, and hematuria (n = 1 each). SAEs in participants receiving placebo included idiopathic thrombocytopenic purpura, vertigo, pneumonia, gastroenteritis, contusion, transient ischemic attack, and agitation (n = 1 each). Eight (5.3%) participants receiving dextromethorphan/quinidine and 4 (3.1%) receiving placebo discontinued treatment owing to AEs, including 4 (2.6%) and 2 (1.6%), respectively, for SAEs. No deaths occurred during the study.

Of the 13 participants who fell while receiving dextromethorphan/quinidine, 9 had a prior history of falls. Three fell 2 to 4 days after study completion, and 1 participant fell twice within 24 hours of receiving lorazepam rescue in both instances; no participants who fell while receiving placebo had a history of falls. Two falls were associated with serious AEs (SAEs): femur fracture on dextromethorphan/quinidine and contusion on placebo.

No clinically meaningful between-group differences in ECG parameters were observed. The mean (SD) QTcF was 5.3 (14.06) and -0.3 (12.96) msec for participants receiving dextromethorphan/quinidine (n = 138) and placebo (n = 60), respectively, at final visit. Fifteen (10.3%) receiving AVP 923 and 8 (6.7%) receiving placebo had a QTcF change ≥30 msec at any visit; one participant on placebo had a QTcF change >60 msec. No participant had a QTcF >500 msec.

It is clear from the data presented in Table 6 and Figure 4, Figure 5, and Figure 6, that the combination of dextromethorphan and quinidine is significantly effective in treating agitation and aggression in patients with probable Alzheimer's disease compared to placebo. Additionally, this combination was generally well tolerated in this elderly population and was not associated with cognitive impairment, sedation, or clinically significant QTc prolongation.

### Example 2: Rationale for Studying AVP-786 for Treatment of Agitation in Alzheimer's disease.

AVP-786 is a combination product of deuterated (d6)-dextromethorphan hydrobromide (d6-DM), the central nervous system (CNS)-active component, and quinidine sulfate (Q), used as an inhibitor of d6-DM metabolism via the cytochrome P450 (CYP) liver isoenzyme 2D6 (CYP2D6). AVP-786 can provide clinical benefit for treatment of agitation in subjects with Alzheimer's Disease (AD).

d6-DM binds to receptors responsible for modulation of glutamate, monoamine, sigma-1, and nicotinic cholinergic pathways that may be key to CNS therapeutics. Pharmacology studies, conducted by the inventors, with d6-DM have demonstrated that deuteration does not alter the basic pharmacology of DM. Also, pharmacokinetic (PK) and drug metabolism studies indicate that d6-DM is metabolized by the same metabolic pathways as DM, but that deuteration results in a decreased rate of metabolism by CYP2D6.

A recent Phase 2 Study, conducted by the inventors, met the primary efficacy endpoint and showed clear improvement of agitation in patients receiving AVP-923 compared with placebo. This Study was a 10-week, randomized, double-blind, placebo-controlled, 2-stage, sequential parallel comparison design (SPCD) study to assess the efficacy, safety, and tolerability of AVP-923 for the treatment of agitation in 220 patients with AD. Patients who received AVP-923 had significantly reduced agitation compared to patients who received placebo (p<0.001) as measured by the Agitation/Aggression domain of the Neuropsychiatric Inventory (NPI). The majority of the secondary endpoints studied (e.g., NPI-total, NPI-Agitation/Aggression domain Caregiver Distress, Clinical Global Impression of Change [CGIC]-Agitation, Clinical Global Impression of Severity [CGIS]-Agitation, and Patient Global Impression of Change [PGIC]) were also statistically significant. Both clinician and patient/caregiver impressions of change corroborated the clinical meaningfulness of improvements observed in agitation. Numerically favorable response in cognition as measured by the Mini Mental State Examination (MMSE) and the Alzheimer's Disease Assessment Scale-cognitive subscale (ADAS-cog) were observed but neither achieved statistical significance.

Further studies, conducted by inventors, have shown that d6-deuterium modification of dextromethorphan (DM) reduced the rate of CYP2D6 metabolism such that combination with a significantly lower dose of quinidine (< 50% of the amount of Q contained in AVP-923) was sufficient for bioequivalence of d6-DM to DM in AVP-786 and AVP-923, respectively. The lower amount of Q in AVP-786 may reduce interactions with other CYP2D6 substrates, limit Q levels even in the presence of CYP3A4 inhibitors, and reduce the effects on cardiac repolarization and QTc **interval.**

### Example 3: A Phase 3, multicenter, randomized, double-blind, placebo-controlled study to assess the efficacy, safety, and tolerability of AVP-786 (deuterated [d6]-dextromethorphan hydrobromide [d6-DM]/quinidine sulfate [Q]) for the treatment of agitation in patients with dementia of the Alzheimer's type.

This study is designed to evaluate the efficacy, safety, and tolerability of AVP-786 for the treatment of agitation in subjects with dementia of the Alzheimer's type.

### Rationale for AVP-786 Treatment Duration.

The treatment duration of 12 weeks is considered an optimal treatment duration to assess efficacy and is based on the review of data from the study 12-AVR-131 with AVP-923 (Papakostas GI et al., Am J Psychiatry. 2012;169(12):1267-1274; Marshall R, Leigh-Pemberton R, Memisoglu A, et al. Opioid modulation: a novel mechanism for the treatment of depression : results of the ALKS 5461 phase 2 study. 2004;2:5461). Patients assigned to the same treatment for the entire 12 weeks of the study, also allows assessment of treatment response over a longer period of time.

### Study Population

*Number of Patients:* Approximately 380 patients will be enrolled at approximately 60 centers in the US.

*Condition*/*Disease*: Patients with agitation secondary to dementia of the Alzheimer's type. The diagnosis of probable Alzheimer's disease will be based on the "2011 Diagnostic Guidelines for Alzheimer's Disease" issued by the National Institute on Aging (NIA)-Alzheimer's Association (AA) workgroups (McKhann GM et al., Alzheimers Dement. 2011;7(3):263- 269). Diagnosis of agitation will be based on the provisional consensus definition of agitation in patients with cognitive disorders developed by the International Psychogeriatric Association (IPA) Agitation Definition Work Group (Cummings J et al., Int Psychogeriatr. 2014:1-11).

*Key Inclusion Criteria:* Patients with clinically significant, moderate/severe agitation at the time.of screening and for at least 2 weeks prior to randomization, that interferes with daily routine and for which a prescription medication is indicated in the opinion of the investigator. A Clinical Global Impression of Severity of Illness scale (CGIS) Agitation score of ≥ 4 (moderately ill) at screening and baseline is required for study participation. Eligible patients must have a reliable caregiver who is able and willing to comply with study procedures, including not administering any prohibited medications during the course of the study.

*Key Exclusion Criteria:* Patients with dementia predominantly of the non-Alzheimer's type (e.g., vascular dementia, fronto-temporal dementia, Parkinson's disease, substance-induced dementia) and patients with agitation that are not secondary to Alzheimer's disease (e.g., secondary to pain, other psychiatric disorder, or delirium) are not eligible.

### Study Design

*Structure*: This is a phase 3, multicenter, randomized, double-blind, placebo-controlled study.

*Duration*: Patients will be enrolled in the study for approximately 16 weeks; with a 4-week screening period and 12-week treatment period.

*Study Treatment*: The investigational product is AVP-786 (deuterated [d6]-dextromethorphan hydrobromide [d6-DM]/quinidine sulfate [Q]). Two doses of AVP-786 will be evaluated: d6-DM 28 mg/Q 4.9 mg and d6-DM 18 mg/Q 4.9 mg, hereafter referred to as AVP-786-28/4.9 and AVP-786-18/4.9, respectively.

*Composition of AVP-786*: The qualitative and quantitative compositions of the 2 doses of the IP and the placebo are listed in Table 7 below.

**Table 7:**

| **Ingredient (amounts in mg)** | **AVP-786-28/4.9** | **AVP-786-18/4.9** | **AVP-786 Placebo** |
|---|---|---|---|
| D6-Dextromethorphan hydrobromide | 28.00 | 18.00 | 0 |
| Quinidine sulfate USP, EP | 4.90 | 4.90 | 0 |
| Croscarmellose sodium NF | 6.60 | 6.60 | 6.60 |
| Microcrystalline cellulose NF | 177.20 | 187.20 | 210.10 |
| Colloidal silicone dioxide NF | 2.20 | 2.20 | 2.20 |
| Magnesium stearate NF | 1.10 | 1.10 | 1.10 |
| **Total** | **220.00** | **220.00** | **220.00** |
| **Size 3 Blue Opaque capsules (average weight)** | 48.00 | 48.00 | 48.00 |
| **Total** | **268.00** | **268.00** | **268.00** |

| | | | |
|---|---|---|---|
| EP - European Pharmacopocia; USP - United States Pharmacopocia; NF - National Formulary | | | |

*Control*: Placebo capsules of identical appearance to study medication will be used as control.

*Randomization*/*Stratification*: Eligible patients will be randomized into the study to receive either AVP-786-28/4.9 capsules, AVP-786-18/4.9 capsules, or matching placebo capsules. The randomization will be stratified by the Neuropsychiatric Inventory (NPI) Agitation/Aggression domain score (≤ 6 vs. > 6), risk assessment for falls (normal/mild vs. moderate/severe), and concomitant use of antipsychotic medications (yes vs. no).

*Dose Regimen*: Eligible patients will be randomly assigned at the Baseline visit to receive AVP-786 or matching placebo capsules. Patients randomized to receive AVP-786-28/4.9 will start with AVP-786-18/4.9 once a day in the morning and placebo in the evening for the first 7 days of the study. From Day 8, patients will receive AVP-786-18/4.9 BID for 14 days. From Day 22, patients will receive AVP-786-28/4.9 BID for the remaining 9 weeks of the study. Patients randomized to receive AVP-786-18/4.9 will start with AVP-786-18/4.9 once a day in the morning and placebo in the evening for the first 7 days of the study. From Day 8, patients will receive AVP-786-18/4.9 BID for the remaining 11 weeks of the study. Patients will have at least a 50% chance of receiving AVP-786 at some point during the study. Study medication will be administered orally twice daily (BID, 1 capsule in the morning and 1 capsule in the evening approximately 12 hours apart) throughout the study.

### Assessments and Visits

Patients will attend clinic visits at Screening, Baseline (Day 1), and on Days 8 (Week 1), 22 (Week 3), 43 (Week 6), 64 (Week 9), and 85 (Week 12). Safety follow-up phone calls will be made on Days 29 (Week 4) and 71 (Week 10). Study procedures will be performed at each visit as outlined in the Schedule of Evaluations and Visits (Table 8). Assessments should be performed, whenever possible, by the same rater throughout the study.

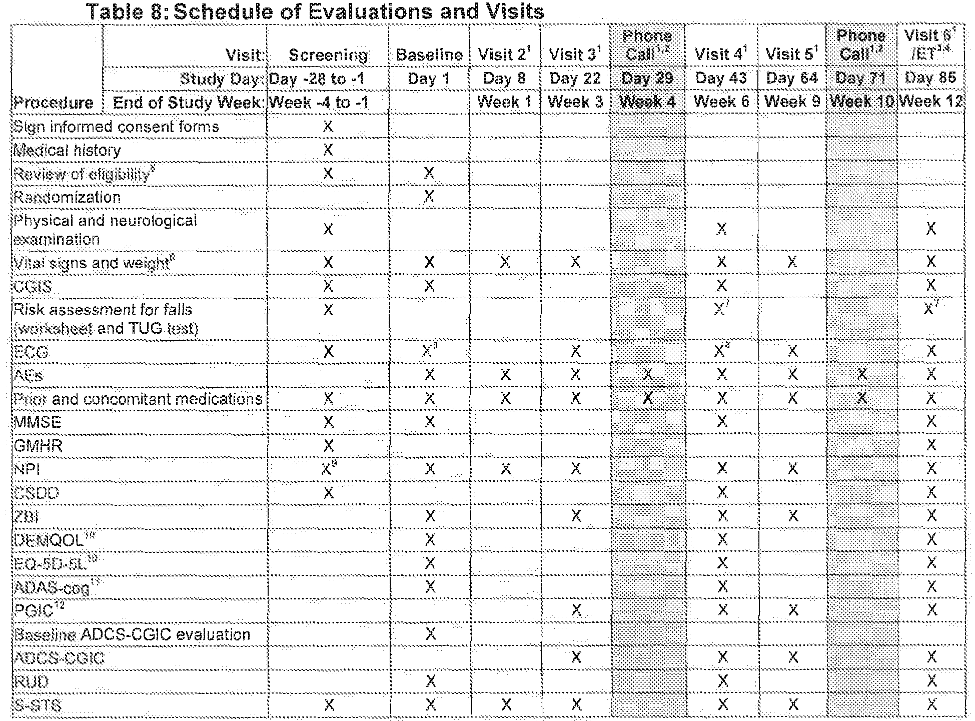

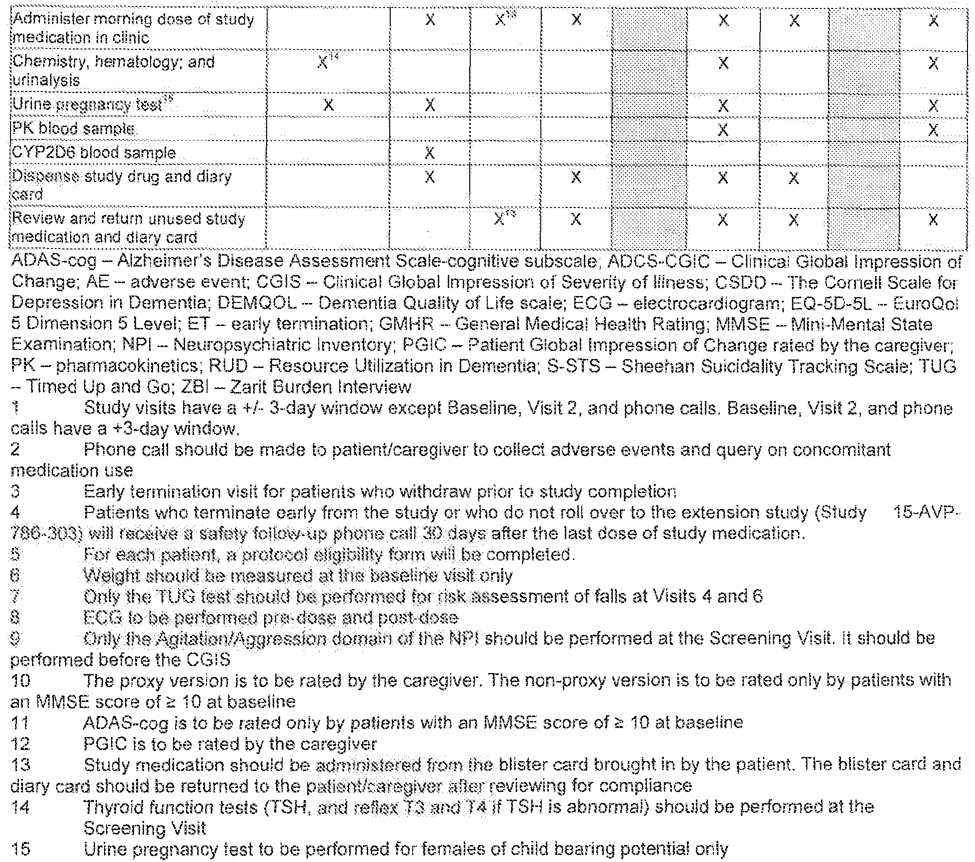

### Response Measures

*Efficacy Primary measure*: Primary efficacy will be assessed using the Agitation/Aggression domain of the Neuropsychiatric Inventory (NPI).

*Efficacy Secondary measures*: Secondary efficacy measures include Clinical Global Impression of Change (CGIC) Agitation, NPI-Agitation/Aggression domain Caregiver Distress score, NPI-Aberrant Motor Behavior domain, Zarit Burden Interview (ZBI), NPI-Irritability/Lability domain, Patient Global Impression of Change (PGIC, rated by caregiver), Dementia Quality of Life (DEMQOL), Cornell Scale for Depression in Dementia (CSDD), Resource Utilization in Dementia (RUD), total NPI, CGIS Agitation, Alzheimer's Disease Assessment Scale-Cognitive Subscale (ADAS-cog), General Medical Health Rating (GMHR), and EuroQol 5-Dimension 5-Level (EQ-5D-5L).

*Pharmacokinetics:* Plasma concentrations of d6-DM, its metabolites, and Q will be measured.

*Safety and Tolerability*: Safety and tolerability of AVP-786 will be assessed by reported adverse events (AEs), physical and neurological examinations, vital signs, clinical laboratory assessments, resting 12-lead electrocardiograms (ECGs), Sheehan Suicidality Tracking Scale (S-STS), Mini Mental State Examination (MMSE), and the Timed Up and Go (TUG) test. Pregnancy tests will be conducted for females of childbearing potential.

### General Statistical Methods and Types of Analyses

*Efficacy Analyses*: The primary efficacy endpoint of the study is the change from Baseline to Week 12 (Day 85) in the NPI Agitation/Aggression domain score. The treatment effect will be estimated by using a likelihood-based linear mixed effects model repeated measures (MMRM) on observed data. A gate-keeping procedure will be used to control the overall type I error at 2-sided α = 0.05 for the 2 primary comparisons: AVP-786-28/4.9 vs. placebo and AVP-786-18/4.9 vs. placebo. Secondary efficacy endpoints include change from Baseline to Week 12 (Day 85) for the following efficacy measures: CGIC Agitation, NPI-Agitation/Aggression domain Caregiver Distress score, NPI-Aberrant Motor Behavior domain, ZBI, NPI-Irritability/Lability domain, PGIC, DEMQOL, CSDD, RUD, total NPI, CGIS Agitation, ADAS-cog, GMHR, and EQ-5D-5L.

*Safety Analyses*: Safety measures will be summarized by treatment groups.

*Sample Size Calculation*: Power calculations were performed assuming a bivariate normal distribution for the primary efficacy endpoint with AVP-786-28/4.9 (high dose) versus placebo. Based on results of a completed Phase 2 study, treatment difference is assumed to be -2.1 and the standard deviations to be 4.7 (effect size of -0.45). The total cumulative dropout rate is 20%. The planned enrollment of 380 patients yields approximately 93% power to reject the null hypothesis in the comparison of AVP-786-28/4.9 versus placebo with type one error rate at two-sided α = 0.05 level. To control the overall type I error rate at 0.05 level, the null hypothesis about the low dose AVP-786-18/4.9 will be tested only if the null hypothesis about the high dose is rejected.

### Example 4: A Phase 3, multicenter, randomized, double-blind, placebo-controlled study to assess the efficacy, safety, and tolerability of AVP-786 (deuterated [d6]-dextromethorphan hydrobromide [d6-DM]/quinidine sulfate [Q]) for the treatment of agitation in patients with dementia of the Alzheimer's type.

The objectives of the study are to evaluate the efficacy, safety, and tolerability of AVP-786 compared to placebo, for the treatment of agitation in patients with dementia of the Alzheimer's type.

A drug being developed for treatment of a chronic condition needs to show maintenance of effect. A 12-week treatment duration is generally considered by experts and regulators as a reasonable time frame to assess acute and chronic effects and safety of a compound for chronic use. To ensure tolerability, patients allocated to the study treatment (AVP-786) will gradually be exposed to a higher dose; beginning with once-daily dosing of the low dose (AVP-786-18/4.9) for one week, followed by twice-daily dosing of the low dose for 2 weeks, and then twice-daily dosing of the high dose (AVP-786-28/4.9) for the remaining 9-week study duration. In addition, a one-time down titration to the low dose will be allowed for patients who exhibit tolerability problems with the high dose.

### Study Population

*Number of Patients*: Approximately 325 patients will be enrolled at approximately 50 centers in the US.

*Condition*/*Disease*: Patients with agitation secondary to dementia of the Alzheimer's type. The diagnosis of probable Alzheimer's disease will be based on the "2011 Diagnostic Guidelines for Alzheimer's Disease" issued by the National Institute on Aging (NIA)-Alzheimer's Association (AA) workgroups (McKhann GM et al., Alzheimers Dement. 2011;7(3):263-269). Diagnosis of agitation will be based on the provisional consensus definition of agitation in patients with cognitive disorders developed by the International Psychogeriatric Association (IPA) Agitation Definition Work Group (Cummings J et al., Mintzer J, Brodaty H, et al. Agitation in cognitive disorders: International Psychogeriatric Association provisional consensus clinical and research definition. Int Psychogeriatr. 2014:1-11).

*Key Inclusion Criteria*: Patients with clinically significant, moderate/severe agitation at the time of screening and for at least 2 weeks prior to randomization, that interferes with daily routine and for which a prescription medication is indicated in the opinion of the investigator. A Clinical Global Impression of Severity of Illness scale (CGIS) Agitation score of ≥ 4 (moderately ill) at screening and baseline is required for study participation. Eligible patients must have a reliable caregiver who is able and willing to comply with study procedures, including not administering any prohibited medications during the course of the study.

*Key Exclusion Criteria*: Patients with dementia predominantly of the non-Alzheimer's type (e.g., vascular dementia, frontotemporal dementia, Parkinson's disease, substance-induced dementia) and patients with agitation that are not secondary to Alzheimer's disease (e.g., secondary to pain, other psychiatric disorder, or delirium) are not eligible.

### Study Design

*Structure*: This is a phase 3, multicenter, randomized, double-blind, placebo-controlled study.

*Duration*: Patients will be enrolled in the study for approximately 16 weeks; with a 4-week screening period and 12-week treatment period.

*Study Treatment*: The investigational product is AVP-786 (deuterated [d6]-dextromethorphan hydrobromide [d6-DM]/quinidine sulfate [Q]). Two doses of AVP-786 will be evaluated by titration, d6-DM 28 mg/Q 4.9 mg and d6-DM 18 mg/Q 4.9 mg, hereafter referred to as AVP-786-28/4.9 and AVP-786-18/4.9, respectively.

*Composition of AVP-786*: The qualitative and quantitative compositions of the 2 doses of the IP and the placebo are listed in Table 9 below.

**Table 9:**

| **Ingredient (amounts in mg)** | **AVP-786-28/4.9** | **AVP-786-18/4.9** | **AVP-786 Placebo** |
|---|---|---|---|
| D6-Dextromethorphan hydrobromide | 28.00 | 18.00 | 0 |
| Quinidine sulfate USP, EP | 4.90 | 4.90 | 0 |
| Croscarmellose sodium NF | 6.60 | 6.60 | 6.60 |
| Microcrystalline cellulose NF | 177.20 | 187.20 | 210.10 |
| Colloidal silicone dioxide NF | 2.20 | 2.20 | 2.20 |
| Magnesium stearate NF | 1.10 | 1.10 | 1.10 |
| **Total** | **220.00** | **220.00** | **220.00** |
| **Size 3 Blue Opaque capsules (average weight)** | 48.00 | 48.00 | 48.00 |
| **Total** | **268.00** | **268.00** | **268.00** |

| | | | |
|---|---|---|---|
| EP - European Pharmacopoeia; USP - United States Pharmacopoeia; NF - National Formulary | | | |

*Control*: Placebo capsules of identical appearance to study medication will be used as control.

*RandomizationlStratification*: Eligible patients will be randomized into the study to receive either AVP-786 or matching placebo capsules. The randomization will be stratified by the Neuropsychiatric Inventory (NPI) Agitation/Aggression domain score (≤ 6 vs. > 6), risk assessment for falls (normal/mild vs. moderate/severe), and concomitant use of antipsychotic medications (yes vs. no).

*Dose Regimen*: Eligible patients will be randomly assigned at the Baseline visit to receive AVP-786 or matching placebo capsules. Study medication will be administered orally twice daily (BID, 1 capsule in the morning and 1 capsule in the evening approximately 12 hours apart) throughout the study. Patients randomized to receive AVP-786 will start with AVP-786-18/4.9 once a day in the morning and placebo in the evening for the first 7 days of the study. From Day 8, patients will receive AVP-786-18/4.9 BID for 14 days. From Day 22, patients will receive AVP-786-28/4.9 BID for the remaining 9 weeks of the study. If deemed necessary by the investigator, a one-time downward dose adjustment to AVP-786-18/4.9 will be allowed after Visit 3 up to and including Visit 5 (i.e., Day 23 to Day 64), and the patient will remain on the lower dose of study medication for the remaining study duration. Patients requiring a dose-adjustment will need to have an unscheduled visit to perform safety assessments. Patients will have at least a 50% chance of receiving AVP-786 at some point during the study.

### Assessments and Visits

Patients will attend clinic visits at Screening, Baseline (Day 1), and on Days 8 (Week 1), 22 (Week 3), 43 (Week 6), 64 (Week 9), and 85 (Week 12). Safety follow-up phone calls will be made on Days 29 (Week 4) and 71 (Week 10). Study procedures will be performed at each visit as outlined in the Schedule of Evaluations and Visits (Table 10).

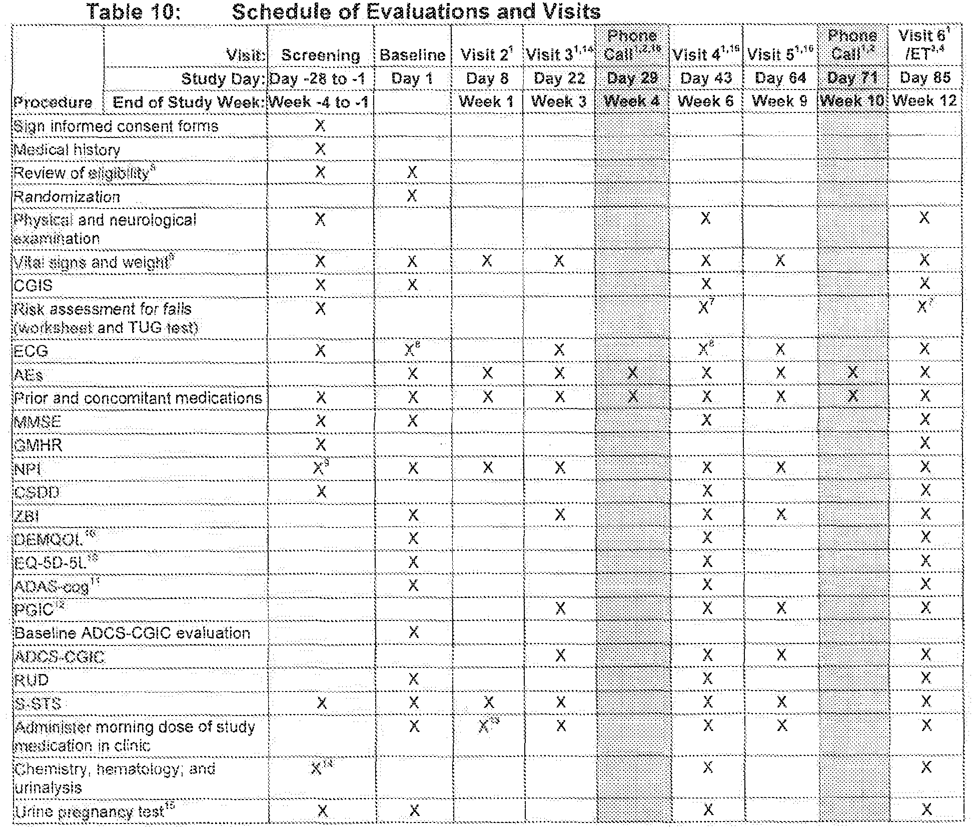

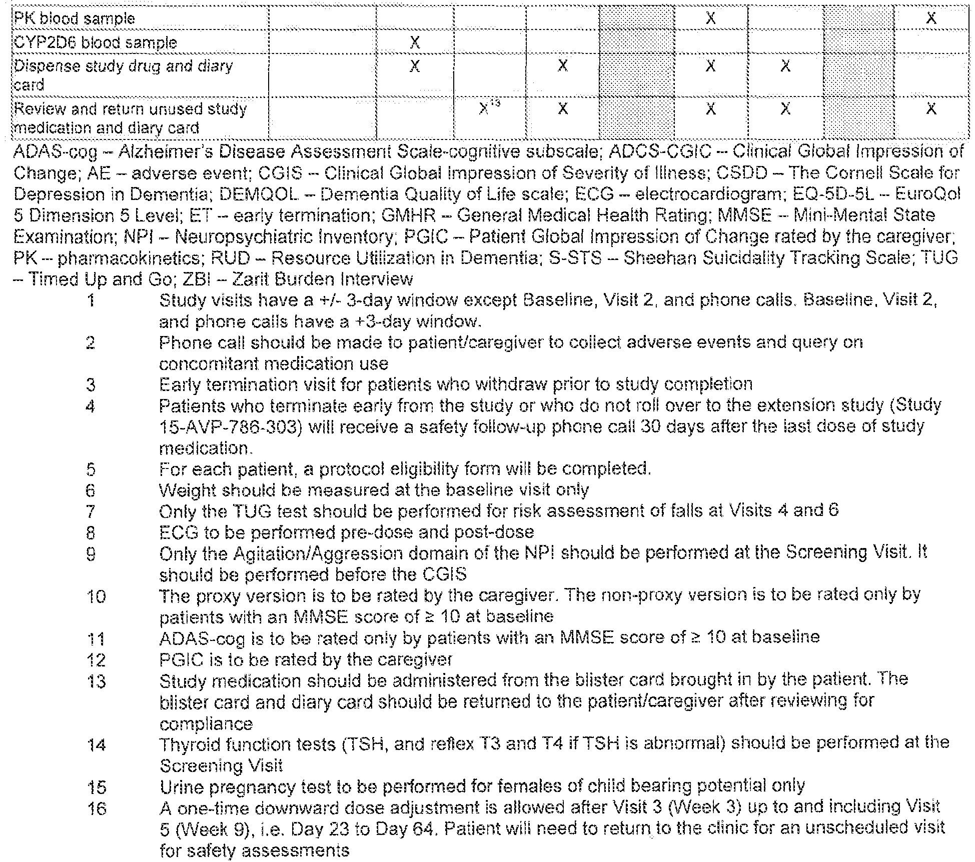

### Response Measures

*Efficacy Primary Measure*: Primary efficacy will be assessed using the Agitation/Aggression domain of the Neuropsychiatric Inventory (NPI).

*Efficacy Secondary Measures*: Secondary efficacy measures include Clinical Global Impression of Change (CGIC) Agitation, NPI-Agitation/Aggression domain Caregiver Distress score, NPI-Aberrant Motor Behavior domain, Zarit Burden Interview (ZBI), NPI-Irritability/Lability domain, Patient Global Impression of Change (PGIC-rated by caregiver), Dementia Quality of Life (DEMQOL), Cornell Scale for Depression in Dementia (CSDD), Resource Utilization in Dementia (RUD), total NPI, CGIS Agitation, Alzheimer's Disease Assessment Scale-Cognitive Subscale (ADAS-cog), General Medical Health Rating (GMHR), and EuroQol 5-Dimension 5-Level (EQ-5D-5L).

*Pharmacokinetics:* Plasma concentrations of d6-DM, its metabolites, and Q will be measured.

*Safety and Tolerability*: Safety and tolerability of AVP-786 will be assessed by reported adverse events (AEs), physical and neurological examinations, vital signs, clinical laboratory assessments, resting 12-lead electrocardiograms (ECGs), Sheehan Suicidality Tracking Scale (S-STS), Mini Mental State Examination (MMSE), and the Timed Up and Go (TUG) test. Pregnancy tests will be conducted for females of childbearing potential.

### General Statistical Methods and Types of Analyses

*Analysis Populations*: Three analysis populations will be used; modified intent-to-treat (mITT), intent-to-treat (ITT), and safety. The mITT population includes all patients randomized in the study who had at least one post-baseline efficacy assessment, and will be used for all analyses of efficacy. Patients in the mITT population will be included in the treatment group to which they were randomized regardless of treatment received. The ITT population includes all randomized patients in the study, and will be used for exploratory efficacy analyses. The safety population includes all patients who received study treatment, and will be used for all analyses of safety. Patients will be included in the treatment group based on the actual treatment received.

*Efficacy Analyses*: the primary efficacy endpoint is the change from Baseline to Week 12 (Day 85) in the NPI Agitation/Aggression domain score. Treatment comparison will be performed by using a linear mixed effects model repeated measures (MMRM). The model will include fixed effects for treatment, visit, treatment-by-visit interaction, baseline-by-visit interaction, and baseline covariates which include baseline value and other randomization stratification factors. An unstructured covariance model will be used. Secondary efficacy endpoints include change from Baseline to Week 12 (Day 85) for the following efficacy measures: CGIC Agitation, NPI-Agitation/Aggression domain Caregiver Distress score, NPI-Aberrant Motor Behavior domain, ZBI, NPI-Irritability/Labllity domain, PGIC, DEMQOL, CSDD, RUD, total NPI, CGIS Agitation, ADAS-cog, GMHR, and EQ-5D-5L.

*Safety Analyses*: Safety analyses will consist of data summaries for biological parameters and AEs. Safety analyses will be tabulated by treatment.

*Sample Size Calculation*: Power calculation was performed assuming a bivariate normal distribution for the primary efficacy endpoint. The treatment effect size observed for the primary efficacy measure in a completed Phase 2 study was -0.51 in Stage 1 and -0.34 in Stage 2. For this single-stage 12-week study, the treatment effect size (AVP-786 vs. placebo) is assumed to be -0.40. The planned total sample size of 325 patients (∼163/arm) will have 90% power with type one error rate at 2-sided α = 0.05, taking into consideration a dropout rate of 20% during the study.

### Example 5: A Phase 3, Multicenter, Long-term, Extension Study of the Safety and Efficacy of AVP-786 (deuterated [d6] dextromethorphan hydrobromide [d6-DM]/quinidine sulfate [Q]) for the Treatment of Agitation in Patients with Dementia of the Alzheimer's Type.

The objectives of the study are to evaluate the long-term safety and maintenance of efficacy of AVP-786 for the treatment of agitation in patients with dementia of the Alzheimer's type.

### Study Population

*Number of Patients*: Approximately 550 patients will be enrolled at approximately 110 centers in the US.

*Condition*/*Disease*: Patients with agitation secondary to dementia of the Alzheimer's type. The diagnosis of probable Alzheimer's disease will be based on the "2011 Diagnostic Guidelines for Alzheimer's Diseas" issued by the National Institute on Aging (NIA)-Alzheimer's Association (AA) workgroups (McKhann GM et al., Alzheimers Dement. 2011;7(3):263-269). Diagnosis of agitation will be based on the provisional consensus definition of agitation in patients with cognitive disorders developed by the International Psychogeriatric Association (IPA) Agitation Definition Work Group (Cummings J et al., Mintzer J, Brodaty H, et al. Agitation in cognitive disorders: International Psychogeriatric Association provisional consensus clinical and research definition. Int Psychogeriatr. 2014:1-11).

*Key Inclusion Criteria*: Patient has successfully completed Studies 15-AVP-786-301, 15-AVP-786-302, or 12-AVR-131. Eligible patients must have a reliable caregiver who is able and willing to comply with all required study procedures, including not administering any prohibited medications during the course of the study.

*Key Exclusion Criteria*: Patient is currently participating in, or has participated in other interventional (drug or device) clinical study since exiting Studies 15-AVP-786-301 and 15-AVP-786-302, or within 30 days prior to Baseline for patients from Study 12-AVR-131. Patients with co-existent, clinically significant, or unstable systemic diseases that could confound the interpretation of the safety results of the study.

### Study Design

*Structure*: This is a Phase 3, multicenter, long-term, extension study of the Phase 3 Studies 15-AVP-786-301 and 15-AVP-786-302, which also allows patients from the Phase 2 Study 12-AVR-131 to be included. Patients from Studies 15-AVP-786-301 and 15-AVP-786-302 will enroll in the current study within 3 days of the last visit (Visit 6, Day 85) in the preceding study. Although all patients enrolled will receive AVP-786, the treatment dose assigned will be masked to the patient, investigator, study staff, and the sponsor.

*Duration*: Patients will be enrolled in the study for approximately 52 weeks.

*Study Treatment*: The investigational product is AVP-786 (deuterated [d6]-dextromethorphan hydrobromide [d6-DM]/quinidine sulfate [Q]). Two doses of AVP-786 will be evaluated by titration: d6-DM 28 mg/Q 4.9 mg and d6-DM 18 mg/Q 4.9 mg, hereafter referred to as AVP-786-28/4.9 and AVP-786-18/4.9, respectively.

*Composition of AVP-786*: The qualitative and quantitative compositions of the 2 doses of the IP are listed below in Table 11.

**Table 11:**

| **Ingredient (amounts in mg)** | **AVP-786-28/4.9** | **AVP-786-18/4.9¹** |
|---|---|---|
| D6-Dextromethorphan hydrobromide | 28.00 | 18.00 |
| Quinidine sulfate USP, EP | 4.90 | 4.90 |
| Croscarmellose sodium NF | 6.60 | 6.60 |
| Microcrystalline cellulose NF | 177.20 | 187.20 |
| Colloidal silicone dioxide NF | 2.20 | 2.20 |
| Magnesium stearate NF | 1.10 | 1.10 |
| **Total** | **220.00** | **220.00** |
| **Size 3 Blue Opaque capsules (average weight)** | 48.00 | 48.00 |
| **Total** | **268.00** | **268.00** |

| | | |
|---|---|---|
| EP - European Pharmacopocia; USP - United States Pharmacopocia; NF - National Formulary 1 Blister cards for the AVP-786-18/4.9 dose will contain AVP-786 placebo capsules for the evening dose for Days 1 to 7. | | |

### Control: None

*Treatment Assignment*: Eligible patients will be assigned to receive either AVP-786-28/4.9 or AVP-786-18/4.9 capsules in a masked manner, depending on the last treatment received in the preceding study (15-AVP-786-301 and 15-AVP-786-302) and the investigator's assessment of dose-adjustment. Study medication will be allocated via an interactive web response system (IWRS).

Patients who received placebo in the preceding studies and Patients from Study 12-AVR-131 will be assigned to AVP-786-18/4.9 in the current study. Patients who received AVP-786-18/4.9 previously will be assigned to the same dose if no dose-adjustment is needed, or to AVP-786-28/4.9 if the investigator deems it necessary to increase the dose. Patients who received AVP-786-28/4.9 previously will either continue to receive AVP-786-28/4.9 or will be assigned to the lower dose AVP-786-18/4.9 at the discretion of the investigator.

Dose Regimen: Study medication will be administered orally twice daily (BID, 1 capsule in the morning and 1 capsule in the evening approximately 12 hours apart) throughout the study. For patients who received placebo in the preceding studies and patients from Study 12-AVR-131, the evening dose for Days 1 to 7 will be AVP-786 placebo capsule and from Day 22 (Week 3) onwards will receive AVP-786- 28/4.9 BID unless a dose adjustment is required. At the discretion of the investigator, the dose of study medication can be down-titrated at any time during the study for safety reasons.

### Assessments and Visits

There are 8 scheduled visits in the study. Patients will attend clinic visits at Baseline (Day 1), and on Days 15 (Week 2), 43 (Week 6), 85 (Week 12), 169 (Week 24), 253 (Week 36), 337, (week 48), and 365 (Week 52). Safety follow-up phone calls will be made on Days 29 (Week 4), 127 (Week 18), 211 (Week 30), and 295 (Week 42). Patients who require dose-adjustments may have unscheduled visits for safety assessments at the discretion of the investigator. Study procedures will be performed at each visit as outlined in the Schedule of Evaluations and Visits (Table 12).

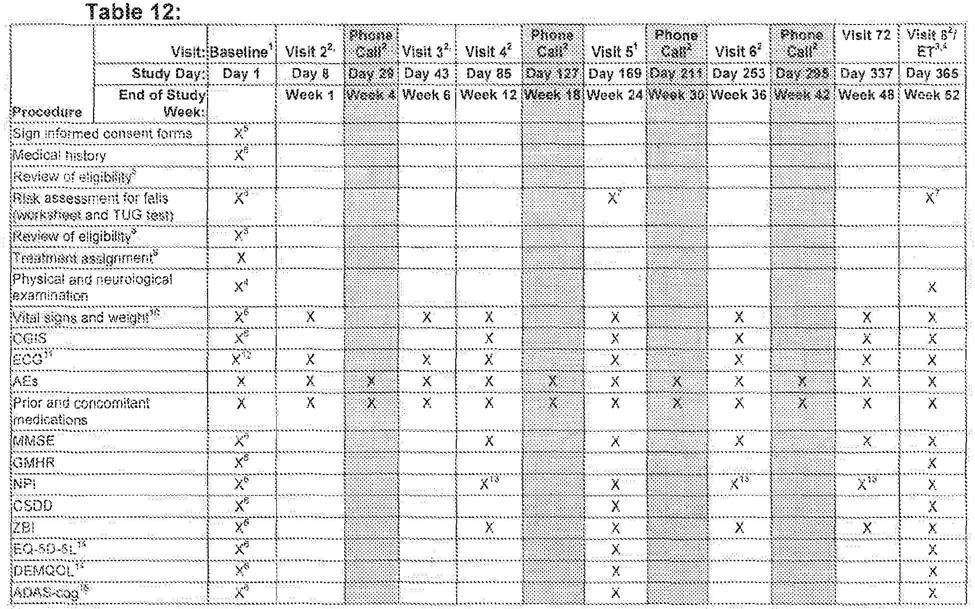

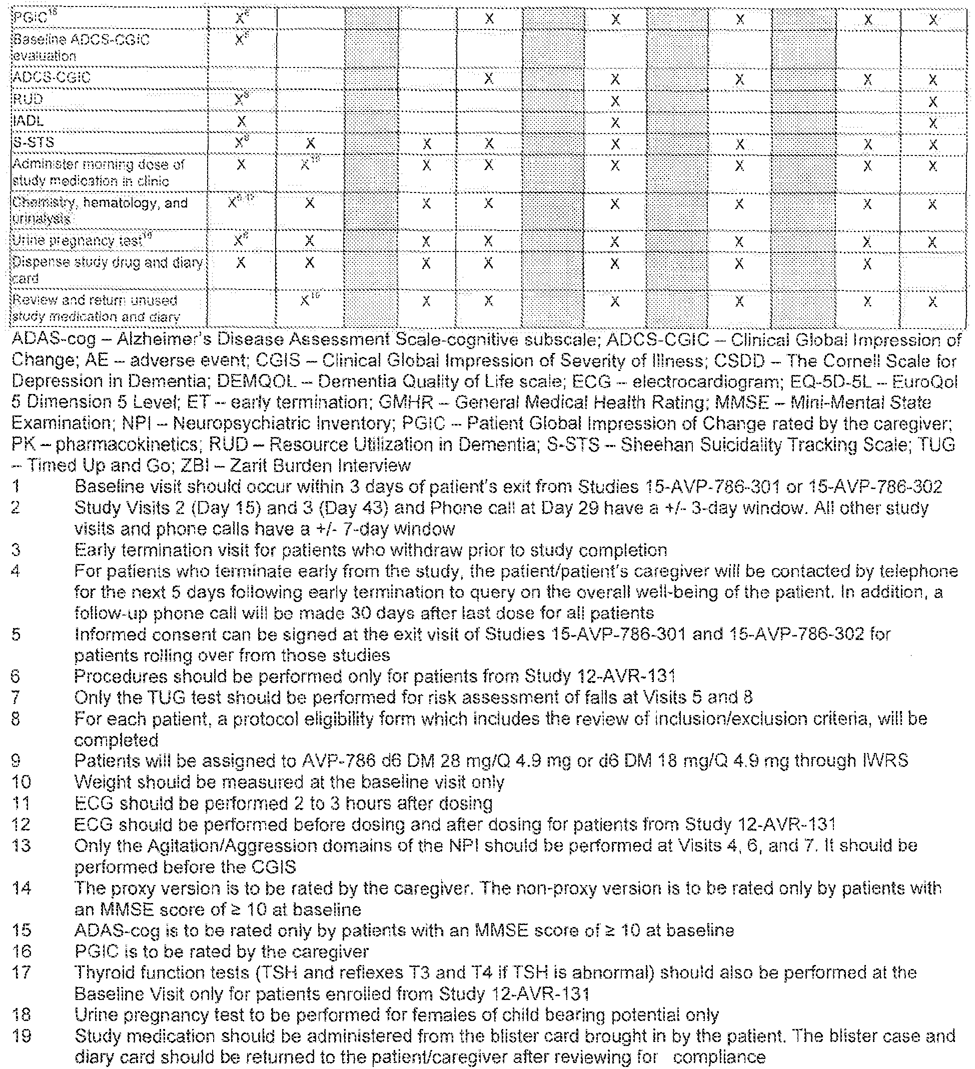

### Response Measures

*Safety and Tolerability*: Safety and tolerability of AVP-786 will be assessed by reported adverse events (AEs), physical and neurological examinations, vital signs, clinical laboratory assessments, resting 12-lead electrocardiograms (ECGs), Sheehan Suicidality Tracking Scale (S-STS), Mini Mental State Examination (MMSE), Alzheimer's Disease Assessment Scale - cognitive subscale (ADAS-cog), and the Timed Up and Go (TUG) test. Pregnancy tests will be conducted for females of childbearing potential.

*Efficacy*: Efficacy will be assessed using the Neuropsychiatric Inventory (NPI), Clinical Global Impression of Change (CGIC)-Agitation, Clinical Global Impression of Severity of Illness scale (CGIS)-Agitation, Zarit Burden Interview (ZBI), Patient Global Impression of Change (PGIC-rated by caregiver), Dementia Quality of Life (DEMQOL), Cornell Scale for Depression in Dementia (CSDD), Resource Utilization in Dementia (RUD), General Medical Health Rating (GMHR), EuroQol 5-Dimension 5-Level (EQ-5D-5L), and Instrumental Activities of Daily Living (IADL).

### General Statistical Methods and Types of Analyses

*Analysis Populations*: The safety population that includes all patients who received study treatment will be used for all efficacy and safety data summaries. Three treatment groups will be presented for both safety and efficacy: AVP-786-28/4.9, AVP-786-18/4.9, and all patients combined. No treatment comparisons will be performed.

*Safety Analyses*: Safety and tolerability measures including reported AEs, vital signs, clinical laboratory assessments, resting 12-lead ECGs, S-STS, MMSE, ADAS-cog, and TUG will be summarized using descriptive statistics and/or frequency tables.

*Efficacy Analyses*: Summary statistics will be provided for observed efficacy data by visit. Observed raw value and change from baseline will be presented where applicable.

*Sample Size Calculation*: The sample size of 550 patients enrolled will provide adequate study medication exposure to satisfy regulatory requirements.

### Example 6: A Phase 1, single-center, open-label, sequential drug interaction study between AVP-786 (deuterated [d6] dextromethorphan hydrobromide [DM]/quinidine sulfate [Q]) and paroxetine and between AVP-786 and duloxetine in healthy subjects.

Primary objectives of this study were to determine the effect of AVP-786 (d6-DM/Q) on the steady-state pharmacokinetics of paroxetine in healthy subjects, to determine the effect of AVP-786 on the steady-state pharmacokinetics of duloxetine in healthy subjects, and to determine the effect of paroxetine and duloxetine on the steady-state pharmacokinetics of AVP-786 in healthy subjects. Secondary objectives of the study were to evaluate the safety and tolerability of concurrent administration of AVP-786 with paroxetine and duloxetine in healthy subjects. Paroxetine and duloxetine are anti-depressants that could conceivably be administered to subjects with Alzheimer's Disease.

Two studies investigating the PK, safety, and tolerability of d6-DM were completed. 12-AVR-132 was a Phase 1 randomized, double-blind, single- and multiple-dose study to assess the single- and multiple-dose PK of AVP 786 in plasma after administration of various oral doses, alone and in combination with Q in healthy subjects. 13-AVR-134 was a Phase 1, single-center, randomized, open-label study to assess the pharmacokinetics, safety and tolerability of AVP-786 in healthy volunteers.

### Methodology

This study was conducted at a single center and consisted of an open-label, sequential design to determine the effects of AVP-786 on the PK of paroxetine and duloxetine, and the effects of paroxetine and duloxetine on the PK of AVP-786. 56 subjects were enrolled into one of 4 treatment groups:
Group 1: n=14
   - Paroxetine 20 mg, once daily Days 1 - 20
   - AVP-786-30/4.75 (d6-DM 30 mg/Q 4.75 mg), BID Day 13 until morning of Day 20
Group 2: n=14
   - AVP-786-30/4.75 (d6-DM 30 mg/Q 4.75 mg), BID Day 1 until morning of Day 20
   - Paroxetine 20 mg, once daily Days 9 - 20
      Group 3: n=14
   - Duloxetine 20 mg, BID Day 1 until morning of Day 13
   - AVP-786-30/4.75 (d6-DM 30 mg/Q 4.75 mg), BID Day 6 until morning of Day 13 Group 4: n=14
   - AVP-786-30/4.75 (d6-DM 30 mg/Q 4.75 mg), BID Day 1 until morning of Day 13
   - Duloxetine 20 mg, BID Day 9 until morning of Day 13

Subjects attended a Screening visit at the clinic within 14 days prior to enrolment (Day 1). If eligible, the subjects were enrolled on Day 1 to one of 4 Groups (Day 1 occurred on different days for different groups to ensure the availability of beds in the units for those dates). Subjects returned to the clinic daily for out-patient dosing until the second day of (morning) PK sampling (this day differed between treatment groups). After the second day (morning) PK sampling, subjects were admitted to the clinic that night before (full) PK sampling the next day. Subjects stayed in the in-patient unit until the last day of PK sampling (length of time differed between treatment groups based on the amount of time for the study medications to reach steady state).

Samples were collected for PK evaluation up to 36 hours post the last dose of study medication. All samples were assayed for: d6-DM, d3-dextrorphan (d3-DX), Q, paroxetine, and duloxetine as appropriate.

Safety was evaluated in all subjects throughout the study and included adverse event (AE) reporting, electrocardiograms (ECGs), laboratory evaluations, physical examinations, and vital signs. The Columbia-Suicide Severity Rating Scale (C-SSRS) was used to prospectively assess suicidal ideation and behavior throughout the study.

Subjects returned for a follow-up safety visit 7 - 10 days after the last dose of study medication.

### Number of Subjects Planned (and Analyzed)

Group 1: Planned n=14; Enrolled n=14 (safety population); Analyzed n=11 (PK population). Group 2: Planned n=14; Enrolled n=14 (safety population); Analyzed n=14 (PK population). Group 3: Planned n=14; Enrolled n=14 (safety population); Analyzed n=14 (PK population). Group 4: Planned n=14; Enrolled n=14 (safety population); Analyzed n=11 (PK population).

### Diagnosis and Criteria for Inclusion

Healthy adult male and female subjects aged 18 - 50 years inclusive with Body Mass Index (BMI) 18 - 300 kg/m² and who exhibited CYP2D6 genotype that confers extensive metabolizer profile.

### Study Treatments

*Test Products*: AVP-786-30/4.75 (d6-DM 30 mg/Q 4.75 mg) capsules. Paroxetine 20 mg tablets. Duloxetine 20 mg capsules. Subjects received study medication as per the study schedule, provided above in Methodology.

Each dose (consisting of one capsule/tablet) was administered orally with 240 mL of room temperature water. Morning doses were preceded by an overnight fast (i.e., at least 8 hours) from food (not including water) and evening doses were preceded by at least a 2-hour fast from food. All doses were followed by a fast from food (not including water) for at least 2 hours post-dose.

### Criteria for Evaluation

*Safety*: Safety was evaluated in all subjects throughout the study and included adverse event (AE) reporting, electrocardiograms (ECGs), laboratory evaluations, physical examinations, and vital signs. The Columbia-Suicide Severity Rating Scale (C-SSRS) was used to prospectively assess suicidal ideation and behavior throughout the study as part of the safety assessments per the FDA guidance, "Suicidal Ideation and Behavior: Prospective Assessment of Occurrence in Clinical Trials."

*Pharmacokinetics:* Blood samples (6 mL for single dosing and 12 mL for combined dosing) for determination of plasma d6-DM, d3-DX, Q, paroxetine, and duloxetine were collected at the following time points:

Pre-dose and 0.5, 1, 2, 3, 4, 6, 8, and 12 hours after the morning dose on the last day of single medication dosing. For Group 1 only, as paroxetine was administered once a day, an additional sample was taken at 24 hours after the last day of single dosing (prior to the first combined dosing).

Pre-dose and 0.5, 1, 2, 3, 4, 6, 8, 12, 24, and 36 hours after the morning dose on the last day of combined dosing

Prior to the morning dose on Day 4 (for Groups 2 and 4 only) and on each of the 2 days prior to the last day of single medication dosing and the last day of combined dosing.

Non-compartmental methods were used to determine pharmacokinetic parameters, including AUC₀₋₁₂, AUC₀₋₂₄, Cₘₐₓ, Tₘₐₓ, kel, t_{1/2}, Cₘᵢₙ, and Tₘᵢₙ.

### Statistical Methods

*Safety*: Safety variables, including incidence of adverse events, vital signs, ECG parameters, and clinical laboratory results, were summarized by treatment. Responses to the C-SSRS questionnaire were listed for each subject. Individual subject profiles were presented for any questions with an abnormal response.

*Pharmacokinetics*: For plasma concentrations of d6-DM, d3-DX, Q, and paroxetine or duloxetine (as applicable), descriptive statistics were tabulated at each time point by treatment. Mean plasma concentration-time profiles were plotted by treatment on linear and semi-logarithmic scales. In addition, non-compartmental PK parameters were derived. Comparisons between combined treatments (Group 1 vs Group 2, AVP-786 and paroxetine; Group 3 vs Group 4, AVP-786 and duloxetine) were presented as geometric mean ratios of Cmax and AUC₀₋₁₂ (AUC₀₋₂₄ for paroxetine) with 90% confidence intervals. The point estimates and confidence limits were determined using the log transformed data and then transformed back to the original scale. The analysis was performed using analysis of variance with the terms Subject and Treatment.

### Results

*Pharmacokinetics:*

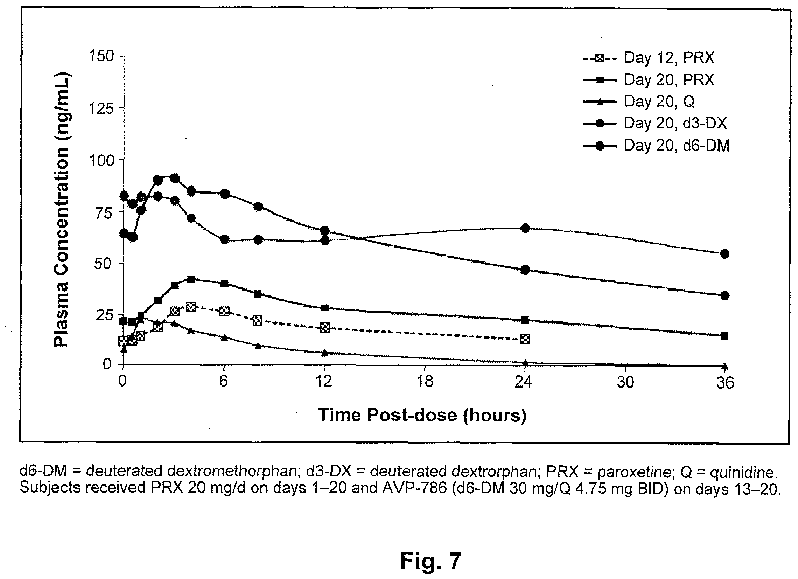

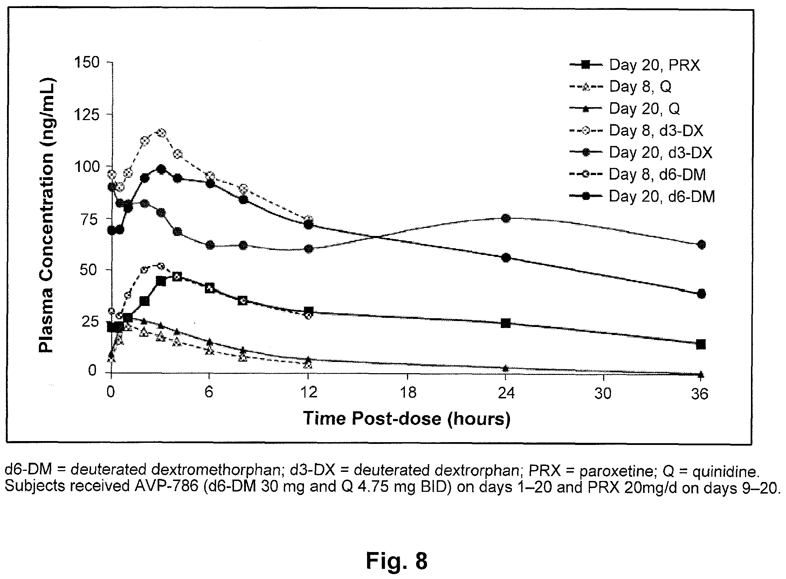

*Paroxetine and AVP-786 Interaction*: Mean multiple dose plasma concentrations over time by treatment group of the various analytes are shown in Figures 7 and 8. Mean (CV%) by Treatment and Analyte (# Median (range) for Tmax) (Table 13):

**Table 13:**

| **Analyte** | **Treatment** | **N (n for t_{1/2})** | **Cₘₐₓ** (ng/mL) | **Tₘₐₓ^{#}** (hr) | **AUC₀₋₁₂ (PAR: AUC₀₋₂₄)** (hr*ng/mL) | **t_{1/2}** (hr) |
|---|---|---|---|---|---|---|
| **d6-DM** | **Group 1, Day 20** | 11 (3) | 93.5 (21%) | 2.00 (2.00-4.00) | 943 (22%) | 18.5 (4.8%) |
| | **Group 2, Day 8** | 14 (0) | 54.6 (30%) | 3.00 (2.00-4.00) | 467 (36%) | NA |
| | **Group 2, Day 20** | 14 (4) | 100 (34%) | 3.00 (1.00-8.00) | 1027 (37%) | 17.3 (20%) |
| **d3-DX** | **Group 1, Day 20** | 11 (0) | 87.2 (22%) | 1.00 (0.00-4.00) | 820 (21%) | NA |
| | **Group 2, Day 8** | 14 (0) | 117 (22%) | 3.00 (0.00-3.00) | 1142 (23%) | NA |
| | **Group 2, Day 20** | 14 (0) | 94.1 (45%) | 0.50 (0.00-2.00) | 820 (23%) | NA |
| **Quinidine (Q)** | **Group 1, Day 20** | 11 (10) | 24.2 (41%) | 1.03 (1.00-3.00) | 163 (48%) | 6.97 (31%) |
| | **Group 2, Day 8** | 14 (13) | 23.1 (40%) | 1.00 (0.50-2.00) | 146 (38%) | 4.80 (15%) |
| | **Group 2, Day 20** | 14 (14) | 28.3 (34%) | 1.00 (0.50-2.00) | 191 (34%) | 8.47 (31%) |
| **Paroxetine (PAR)** | **Group 1, Day 12** | 11 (0) | 29.5 (39%) | 4.00 (3.00-8.00) | 452 (42%) | NA |
| | **Group 1, Day 20** | 11 (2) | 42.8 (32%) | 4.00 (2.00-6.00) | 712 (35%) | 19.2 (8.7%) |
| | **Group 2, Day 20** | 14 (8) | 48.0 (36%) | 4.00 (3.00-6.00) | 768 (40%) | 16.8 (16%) |

Comparison of combined dosing vs. single treatment dosing by log-transformed ANOVA (Table 14):

**Table 14:**

| **Analyte** | **N** | **Parameter** | **Ratio of LS Means** | **90% confidence Interval of LS Means Ratio** |
|---|---|---|---|---|
| **Paroxetine (PAR)** | **11 (Group 1)** | **Cₘₐₓ** (ng/mL) | 149.4% | [135.5 - 164.7] |
| | | **AUC₀₋₂₄** (hr*ng/mL) | 163.7% | [141.7 - 189.2] |
| **d6-DM** | **14 (Group 2)** | **Cₘₐₓ** (ng/mL) | 180.7% | [163,0 - 200.3] |
| | | **AUC₀₋₁₂** (hr*ng/mL) | 217.9% | [193.1 - 246.0] |
| **d3-DX** | **14 (Group 2)** | **Cₘₐₓ** (ng/mL) | 77.1% | [67.1 - 88.4] |
| | | **AUC₀₋₁₂** (hr*ng/mL) | 71.6% | [66.2 - 77.5] |
| **Quinidine (Q)** | **14 (Group 2)** | **Cₘₐₓ** (ng/mL) | 125.3% | [110.2 - 142.5] |
| | | **AUC₀₋₁₂** (hr*ng/mL) | 132.5% | [118.7 - 148.0] |

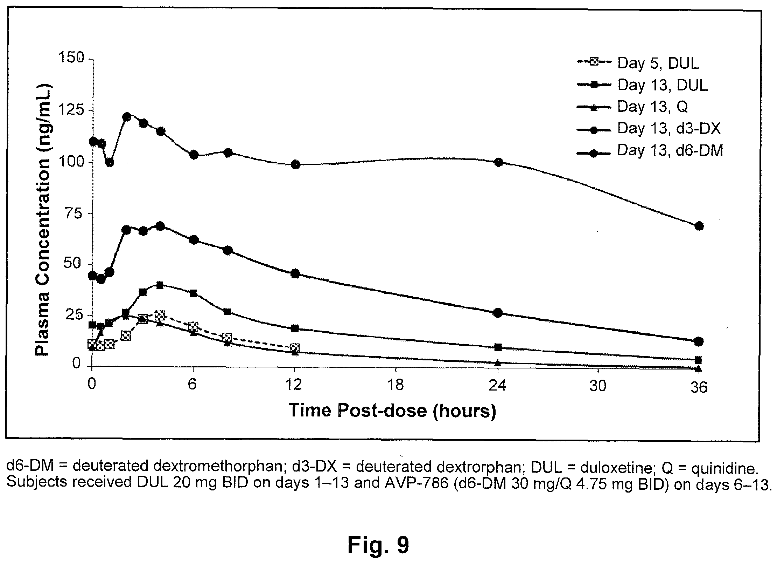

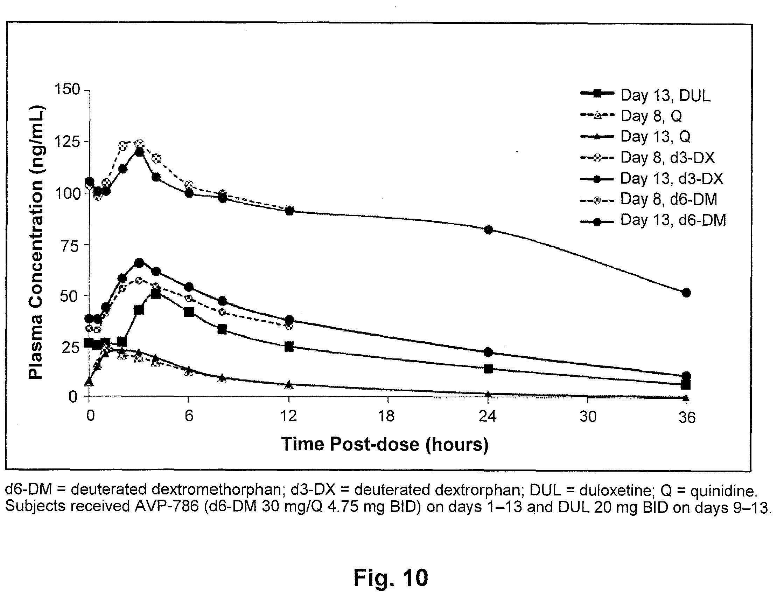

*Duloxetine and AVP-786 Interaction*: Mean multiple dose plasma concentrations over time by treatment group of the various analytes are shown in Figures 9 and 10. Mean (CV%) by Treatment and Analyte (# Median (range) for Tmax) (Table 15):

**Table 15:**

| **Analyte** | **Treatment** | **N (n for t_{1/2})** | **Cₘₐₓ** (ng/mL) | **Tₘₐₓ^{#}** (hr) | **AUC₀₋₁₂** (hr*ng/mL) | **t_{1/2}** (hr) |
|---|---|---|---|---|---|---|
| **d6-DM** | **Group 3, Day 13** | 14 (13) | 72.7 (31%) | 4.00 (2.00-8.00) | 692 (34%) | 13.3 (14%) |
| | **Group 4, Day** 8 | 11 (0) | 59.0 (36%) | 3.00 (2.00-4.00) | 540 (46%) | NA |
| | **Group 4, Day 13** | 11 (11) | 66.8 (31%) | 3.00 (2.00-4.00) | 605 (34%) | 12.6 (24%) |
| **d3-DX** | **Group 3, Day 13** | 14 (1) | 128 (17%) | 3.00 (0.00-8.00) | 1295 (16%) | 21.7 (NA) |
| | **Group 4, Day 8** | 11 (0) | 127 (30%) | 3.00 (0.00-4.00) | 1267 (30%) | NA |
| | **Group 4, Day 13** | 11 (3) | 121 (27%) | 3.00 (0.00-12.00) | 1224 (27%) | 20.0 (5.2%) |
| **Quinidine (Q)** | **Group 3, Day 13** | 14 (14) | 25.5 (31%) | 2.00 (1.00-4.03) | 191 (32%) | 7.39 : (36%) |
| | **Group 4, Day 8** | 11 (11) | 24.9 (46%) | 1.00 (0.50-3.00) | 160 (44%) | 5.19 (13%) |
| | **Group 4, Day 13** | 11 (10) | 25.5 (43%) | 2.00 (0.50-3.08) | 168 (43%) | 6.59 (39%) |
| **Duloxetine (DUL)** | **Group 3, Day 5** | 14 (8) | 26.1 (46%) | 4.00 (3.00-6.00) | 194 (46%) | 5.04 (14%) |
| | **Group 3, Day 13** | 14 (14) | 41.9 (35%) | 3.50 (3,00-6.00) | 345 (38%) | 11.1 (15%) |
| | **Group 4 Day 13** | 11 (11) | 51.4 (37%) | 4.00 (3.00-6.00) | 419 (46%) | 11.4 (22%) |

Comparison of combined dosing vs single treatment dosing by log-transformed ANOVA (Table 16):

**Table 16:**

| **Analyte** | **N** | **Parameter** | **Ratio of LS Means** | **90% confidence Interval of LS Means Ratio** |
|---|---|---|---|---|
| **Duloxetine (DUL)** | **14 (Group 3)** | **Cₘₐₓ** (ng/mL) | 167.6% | [151.0 - 186.1] |
| | | **AUC₀₋₁₂** (hr*ng/mL) | 184.0% | [167.5 - 202.1] |
| **d6-DM** | **11 (Group 4)** | **Cₘₐₓ** (ng/mL) | 113.9% | [106.8 - 121.4] |
| | | **AUC₀₋₁₂** (hr*ng/mL) | 114.8% | [108.9 - 121.0] |
| **d3-DX** | **11 (Group 4)** | **Cₘₐₓ** (ng/mL) | 96.3% | [90.1 - 102.9] |
| | | **AUC₀₋₁₂** (hr*ng/mL) | 97.6% | [91.8 - 103.7[ |
| **Quinidine (Q)** | **11 (Group 4)** | **Cₘₐₓ** (ng/mL) | 103.1% | [91.0 - 116.8] |
| | | **AUC₀₋₁₂** (hr*ng/mL) | 105.4% | [99.5 - 111.6] |

### Safety:

There were no deaths. There was one subject (Subject R112, paroxetine/AVP-786) who experienced 2 concomitant serious adverse events (SAEs): hypokalemia and hypochloremia after receiving paroxetine 20 mg QD (Day 1-16) and AVP-786-30/4.75 BID (Day 13-16). Both were considered moderate in severity. The subject presented with dizziness associated with orthostatic hypotension on Day 15 and muscle weakness of both legs with tingling sensation on Day 16. Blood tests and ECG obtained on Day 17 to help assess the clinical symptoms revealed hypokalemia, metabolic alkalosis, and prolongation of QTc. Symptoms fully resolved with fluid and electrolyte replacement. Based upon Applicant's medical review of the details of these SAEs, and an independent evaluation by an expert cardiologist, the clinical picture was not consistent with known drug effects of either paroxetine or AVP-786.

There were 4 withdrawals due to AEs of suicidal ideation (Subject R107, following 8 doses of paroxetine), hypokalemia and hypochloremia (Subject R112, see above), urinary tract infection (Subject R411, following 12 doses of AVP-786), and vomiting (Subject R412, following 9 doses of AVP-786).

### Paroxetine and AVP-786 Interaction:

In Groups 1 and 2, treatment-emergent AEs were reported for 7 of 14 subjects (50%) following paroxetine (during 12 days of dosing), for 9 of 14 subjects (64%) following AVP-786 (during 8 days of dosing), for 10 of 12 subjects (83%) following paroxetine/AVP-786 combination (during 8 days of dosing), and for 13 of 14 subjects (93%) following AVP-786/paroxetine combination (during 12 days of dosing).

Most AEs (104 of 115 AEs, 90%) were deemed related (possibly or probably) to study treatment. The most common related adverse events were dizziness and postural dizziness, headache, diarrhea, nausea, nightmare, and disturbance in attention. Adverse events of nightmare and disturbance in attention were only reported in subjects who received the combination of paroxetine and AVP-786. Dizziness was reported at a higher incidence when subjects received the combination therapies compared with single treatments of paroxetine or AVP-786. It is noted that headache and nausea were only reported in subjects following paroxetine or the combination of paroxetine and AVP- 786, but not following AVP-786 alone.

Most AEs were classified as mild in severity and no AEs were classified as severe. Adverse events of moderate severity deemed related to study treatment were suicidal ideation, hypokalemia and hypochloremia, and depression.

### Duloxetine and AVP-786 Interaction:

In Groups 3 and 4, treatment-emergent AEs were reported for 6 of 14 subjects (43%) following duloxetine (during 5 days of dosing), for 7 of 14 subjects (50%) following AVP-786 (during 8 days of dosing), for 7 of 14 subjects (50%) following duloxetine/AVP-786 combination (during 8 days of dosing), and for 5 of 11 subjects (45%) following AVP-786/duloxetine combination (during 5 days of dosing).

Most AEs (40 of 50 AEs, 80%) were deemed related (possibly or probably) to study treatment. The most common related adverse events were fatigue and muscle tightness. There were no common AEs with higher incidence in subjects following the combination of AVP-786 and duloxetine compared with single treatments of duloxetine or AVP-786. It is noted that fatigue was only reported in subjects following AVP-786 alone in Group 4, but was not reported following AVP-786 in subjects in Group 2.

Most AEs were classified as mild in severity with one AE classified as severe. The AE of severe intensity was vomiting (1 subject, AVP-786), deemed possibly related to study treatment. Adverse events of moderate severity deemed related to study treatment were vomiting, cold sweat, headache, dizziness, and lower abdominal pain.

Summaries of AEs by severity for each of the groups showed no differences between treatments. There were no clear differences in safety as assessed by clinical laboratory tests, vital signs, and ECG assessments between treatments.

### Conclusions

There were no clinically significant differences in safety assessments for multiple dose combinations of AVP-786 with paroxetine or with duloxetine, compared with multiple dose of the single treatments.

The steady state systemic exposure of paroxetine increased significantly (to approximately 150%) when co-administered with AVP-786, in comparison with paroxetine alone. There was a significant change in the steady state systemic exposure of the analytes of AVP-786 when co-administered with paroxetine compared with AVP-786 alone, with increased systemic exposure of d6-DM approximately two-fold and a substantial decrease in d3-DX (to approximately 75%), indicating that there was a reduction in the rate of metabolism of d6-DM to d3-DX when AVP-786 was administered in combination with paroxetine. There was a moderate increase (to approximately 130%) in the systemic exposure of Q.

The steady state systemic exposure of duloxetine increased significantly (to approximately 180%) when co-administered with AVP-786, in comparison with duloxetine alone. There were no significant changes in the steady state systemic exposure of the analytes of AVP-786 when co-administered with duloxetine compared with AVP-786 alone.

Comprising the following embodiments:
1. A method of treating agitation and/or aggression and/or associated symptoms in a subject with dementia comprising administering to the subject in need thereof deuterated dextromethorphan, or a pharmaceutically acceptable salt thereof, in combination with quinidine, or a pharmaceutically acceptable salt thereof.
2. The method according to embodiment 1, wherein the dementia is of the Alzheimer's type.
3. The method according to embodiment 1 or embodiment 2, wherein deuterated dextromethorphan is administered in an amount ranging from about 10 mg to about 200 mg per day and quinidine is administered in an amount ranging from about 0.05 mg to less than about 50 mg per day.
4. The method according to any one of embodiments 1 to 3, wherein quinidine is administered in an amount ranging from about 4.75 mg to about 20 mg per day.
5. The method according to any one of embodiments 1 to 4, wherein deuterated dextromethorphan is administered in an amount ranging from about 10 mg to about 90 mg per day.
6. The method according to any one of embodiments 1 to 5, wherein at least one of the quinidine and the deuterated dextromethorphan is in a form of a pharmaceutically acceptable salt selected from the group consisting of salts of alkali metals, salts of lithium, salts of sodium, salts of potassium, salts of alkaline earth metals, salts of calcium, salts of magnesium, salts of lysine, salts of N,N'dibenzylethylenediamine, salts of chloroprocaine, salts of choline, salts of diethanolamine, salts of ethylenediamine, salts of meglumine, salts of procaine, salts of tris, salts of free acids, salts of free bases, inorganic salts, salts of sulfate, salts of hydrochloride, and salts of hydrobromide.
7. The method according to any one of embodiments 1 to 6, wherein the deuterated dextromethorphan is in a form of deuterated dextromethorphan hydrobromide.
8. The method according to any one of embodiments 1 to 7, wherein the quinidine is in a form of quinidine sulfate.
9. The method of according to any one of embodiments 1 to 8, wherein deuterated dextromethorphan and quinidine are administered in a unit dosage form comprising about 24 mg deuterated dextromethorphan hydrobromide and about 4.75 mg quinidine sulfate.
10. The method of according to any one of embodiments 1 to 8, wherein deuterated dextromethorphan and quinidine are administered in a unit dosage form comprising about 34 mg deuterated dextromethorphan hydrobromide and about 4.75 mg quinidine sulfate.
11. The method of according to any one of embodiments 1 to 8, wherein deuterated dextromethorphan and quinidine are administered in a unit dosage form comprising about 18 mg deuterated dextromethorphan hydrobromide and about 4.9 mg quinidine sulfate.
12. The method of according to any one of embodiments 1 to 8, wherein deuterated dextromethorphan and quinidine are administered in a unit dosage form comprising about 28 mg deuterated dextromethorphan hydrobromide and about 4.9 mg quinidine sulfate.
13. The method of according to any one of embodiments 1 to 8, wherein deuterated dextromethorphan and quinidine are administered in a unit dosage form comprising about 30 mg deuterated dextromethorphan hydrobromide and about 4.9 mg quinidine sulfate.
14. The method of according to any one of embodiments 1 to 8, wherein deuterated dextromethorphan and quinidine are administered in a unit dosage form comprising about 24 mg deuterated dextromethorphan hydrobromide and about 4.9 mg quinidine sulfate.
15. The method of according to any one of embodiments 1 to 8, wherein deuterated dextromethorphan and quinidine are administered in a unit dosage form comprising about 34 mg deuterated dextromethorphan hydrobromide and about 4.9 mg quinidine sulfate.
16. The method according to any one of embodiments 1 to 15, wherein the deuterated dextromethorphan and the quinidine are administered in a combined dose, and wherein a weight ratio of deuterated dextromethorphan to quinidine in the combined dose is selected from the group consisting of about 1:0.16, about 1:0.18, about 1:0.20, about 1:0.22, about 1:0.23, and about 1:0.31.
17. The method according to any one of embodiments 1 to 16, wherein the deuterated dextromethorphan and quinidine are administered as one combined dose per day.
18. The method according to any one of embodiments 1 to 16, wherein the deuterated dextromethorphan and quinidine are administered as at least two combined doses per day.
19. The method according to any one of embodiments 1 to 18, wherein deuterated dextromethorphan and quinidine are administered in a tablet unit dosage form or a capsule unit dosage form.
20. A combination of deuterated dextromethorphan, or a pharmaceutically acceptable salt thereof, and quinidine, or a pharmaceutically acceptable salt thereof, for use in treating agitation and/or aggression and/or associated symptoms in a subject with dementia.
21. The combination according to embodiment 20, wherein the dementia is of the Alzheimer's type.
22. The combination according to any one of embodiments 20 to 21, wherein deuterated dextromethorphan is administered in an amount ranging from about 10 mg to about 200 mg per day, and wherein quinidine is administered in an amount ranging from about 0.05 mg to less than about 50 mg per day.
23. The combination according to any one of embodiments 20 to 22, wherein quinidine is administered in an amount ranging from about 4.75 mg to about 20 mg per day.
24. The combination according to any one of embodiments 20 to 23, wherein deuterated dextromethorphan is administered in an amount ranging from about 10 mg to about 90 mg per day.
25. The combination according to any one of embodiments 20 to 24, wherein at least one of the quinidine and the deuterated dextromethorphan is in a form of a pharmaceutically acceptable salt selected from the group consisting of salts of alkali metals, salts of lithium, salts of sodium, salts of potassium, salts of alkaline earth metals, salts of calcium, salts of magnesium, salts of lysine, salts of N,N'dibenzylethylenediamine, salts of chloroprocaine, salts of choline, salts of diethanolamine, salts of ethylenediamine, salts of meglumine, salts of procaine, salts of tris, salts of free acids, salts of free bases, inorganic salts, salts of sulfate, salts of hydrochloride, and salts of hydrobromide.
26. The combination according to any one of embodiments 20 to 25, wherein the deuterated dextromethorphan is in a form of deuterated dextromethorphan hydrobromide.
27. The combination according to any one of embodiments 20 to 26, wherein the quinidine is in a form of quinidine sulfate.
28. The combination according to any one of embodiments 20 to 27, wherein deuterated dextromethorphan and quinidine are administered in a unit dosage form comprising about 24 mg deuterated dextromethorphan hydrobromide and about 4.75 mg quinidine sulfate.
29. The combination according to any one of embodiments 20 to 27, wherein deuterated dextromethorphan and quinidine are administered in a unit dosage form comprising about 34 mg deuterated dextromethorphan hydrobromide and about 4.75 mg quinidine sulfate.
30. The combination according to any one of embodiments 20 to 27, wherein deuterated dextromethorphan and quinidine are administered in a unit dosage form comprising about 18 mg deuterated dextromethorphan hydrobromide and about 4.9 mg quinidine sulfate.
31. The combination according to any one of embodiments 20 to 27, wherein deuterated dextromethorphan and quinidine are administered in a unit dosage form comprising about 28 mg deuterated dextromethorphan hydrobromide and about 4.9 mg quinidine sulfate.
32. The combination according to any one of embodiments 20 to 27, wherein deuterated dextromethorphan and quinidine are administered in a unit dosage form comprising about 30 mg deuterated dextromethorphan hydrobromide and about 4.9 mg quinidine sulfate.
33. The combination according to any one of embodiments 20 to 27, wherein deuterated dextromethorphan and quinidine are administered in a unit dosage form comprising about 24 mg deuterated dextromethorphan hydrobromide and about 4.9 mg quinidine sulfate.
34. The combination according to any one of embodiments 20 to 27, wherein deuterated dextromethorphan and quinidine are administered in a unit dosage form comprising about 34 mg deuterated dextromethorphan hydrobromide and about 4.9 mg quinidine sulfate.
35. The combination according to any one of embodiments 20 to 34, wherein the deuterated dextromethorphan and the quinidine are administered in a combined dose, and wherein a weight ratio of deuterated dextromethorphan to quinidine in the combined dose is selected from the group consisting of about 1:0.16, about 1:0.18, about 1:0.20, about 1:0.22, about 1:0.23, and about 1:0.31.
36. The combination according to any one of embodiments 20 to 35, wherein the deuterated dextromethorphan and quinidine are administered as one combined dose per day.
37. The combination according to any one of embodiments 20 to 35, wherein the deuterated dextromethorphan and quinidine are administered as at least two combined doses per day.
38. The combination according to any one of embodiments 20 to 37, wherein deuterated dextromethorphan and quinidine are administered in a tablet unit dosage form or a capsule unit dosage form.
39. A method of treating agitation and/or aggression and/or associated symptoms in a subject with dementia comprising administering to the subject in need thereof dextromethorphan, or a pharmaceutically acceptable salt thereof, in combination with quinidine, or a pharmaceutically acceptable salt thereof.
40. The method according to embodiment 39, wherein the dementia is of the Alzheimer's type.
41. The method according to embodiment 39 or 40, wherein dextromethorphan is administered in an amount ranging from about 10 mg to about 200 mg per day, and wherein quinidine is administered in an amount ranging from about 0.05 mg to less than about 50 mg per day.
42. The method according to any one of embodiments 39 to 41, wherein quinidine is administered in an amount ranging from about 4.75 mg to about 20 mg per day.
43. The method according to any one of embodiments 39 to 42, wherein dextromethorphan is administered in an amount ranging from about 10 mg to about 90 mg per day.
44. The method according to any one of embodiments 39 to 43, wherein at least one of the quinidine and the dextromethorphan is in a form of a pharmaceutically acceptable salt selected from the group consisting of salts of alkali metals, salts of lithium, salts of sodium, salts of potassium, salts of alkaline earth metals, salts of calcium, salts of magnesium, salts of lysine, salts of N,N'dibenzylethylenediamine, salts of chloroprocaine, salts of choline, salts of diethanolamine, salts of ethylenediamine, salts of meglumine, salts of procaine, salts of tris, salts of free acids, salts of free bases, inorganic salts, salts of sulfate, salts of hydrochloride, and salts of hydrobromide.
45. The method according to any one of embodiments 39 to 44, wherein the dextromethorphan is in a form of dextromethorphan hydrobromide.
46. The method according to any one of embodiments 39 to 45, wherein the quinidine is in a form of quinidine sulfate.
47. The method of according to any one of embodiments 39 to 46, wherein dextromethorphan and quinidine are administered in a unit dosage form comprising about 20 mg dextromethorphan hydrobromide and about 10 mg quinidine sulfate.
48. The method of according to any one of embodiments 39 to 46, wherein dextromethorphan and quinidine are administered in a unit dosage form comprising about 30 mg dextromethorphan hydrobromide and about 10 mg quinidine sulfate.
49. The method of according to any one of embodiments 39 to 46, wherein dextromethorphan and quinidine are administered in a unit dosage form comprising about 45 mg dextromethorphan hydrobromide and about 10 mg quinidine sulfate.
50. The method of according to any one of embodiments 39 to 46, wherein dextromethorphan and quinidine are administered in a unit dosage form comprising about 15 mg dextromethorphan hydrobromide and about 9 mg quinidine sulfate.
51. The method of according to any one of embodiments 39 to 46, wherein dextromethorphan and quinidine are administered in a unit dosage form comprising about 23 mg dextromethorphan hydrobromide and about 9 mg quinidine sulfate.
52. The method according to any one of embodiments 39 to 51, wherein the dextromethorphan and the quinidine are administered in a combined dose, and wherein a weight ratio of dextromethorphan to quinidine in the combined dose is selected from the group consisting of about 1:0.68, about 1:0.56, about 1:0.44, about 1:0.38, and about 1:0.25.
53. The method according to any one of embodiments 39 to 52, wherein the dextromethorphan and quinidine are administered as one combined dose per day.
54. The method according to any one of embodiments 39 to 52, wherein the dextromethorphan and quinidine are administered as at least two combined doses per day.
55. The method according to any one of embodiments 39 to 54, wherein dextromethorphan and quinidine are administered in a tablet unit dosage form or a capsule unit dosage form.
56. A combination of dextromethorphan, or a pharmaceutically acceptable salt thereof, and quinidine, or a pharmaceutically acceptable salt thereof, for use in treating agitation and/or aggression and/or associated symptoms in a subject with dementia.
57. The combination according to embodiment 56, wherein the dementia is of the Alzheimer's type.
58. The combination according to embodiment 56 or embodiment 57, wherein dextromethorphan is administered in an amount ranging from about 10 mg to about 200 mg per day, and wherein quinidine is administered in an amount ranging from about 0.05 mg to less than about 50 mg per day.
59. The combination according to any one of embodiments 56 to 58, wherein quinidine is administered in an amount ranging from about 4.75 mg to about 20 mg per day.
60. The combination according to any one of embodiments 56 to 59, wherein dextromethorphan is administered in an amount ranging from about 10 mg to about 90 mg per day.
61. The combination according to any one of embodiments 56 to 60, wherein at least one of the quinidine and the dextromethorphan is in a form of a pharmaceutically acceptable salt selected from the group consisting of salts of alkali metals, salts of lithium, salts of sodium, salts of potassium, salts of alkaline earth metals, salts of calcium, salts of magnesium, salts of lysine, salts of N,N'dibenzylethylenediamine, salts of chloroprocaine, salts of choline, salts of diethanolamine, salts of ethylenediamine, salts of meglumine, salts of procaine, salts of tris, salts of free acids, salts of free bases, inorganic salts, salts of sulfate, salts of hydrochloride, and salts of hydrobromide.
62. The combination according to any one of embodiments 56 to 61, wherein the dextromethorphan is in a form of dextromethorphan hydrobromide.
63. The combination according to any one of embodiments 56 to 62, wherein the quinidine is in a form of quinidine sulfate.
64. The combination according to any one of embodiments 56 to 63, wherein dextromethorphan and quinidine are administered in a unit dosage form comprising about 20 mg dextromethorphan hydrobromide and about 10 mg quinidine sulfate.
65. The combination according to any one of embodiments 56 to 63, wherein dextromethorphan and quinidine are administered in a unit dosage form comprising about 30 mg dextromethorphan hydrobromide and about 10 mg quinidine sulfate.
66. The combination according to any one of embodiments 56 to 63, wherein dextromethorphan and quinidine are administered in a unit dosage form comprising about 45 mg dextromethorphan hydrobromide and about 10 mg quinidine sulfate.
67. The combination according to any one of embodiments 56 to 63, wherein dextromethorphan and quinidine are administered in a unit dosage form comprising about 15 mg dextromethorphan hydrobromide and about 9 mg quinidine sulfate.
68. The combination according to any one of embodiments 56 to 63, wherein dextromethorphan and quinidine are administered in a unit dosage form comprising about 23 mg dextromethorphan hydrobromide and about 9 mg quinidine sulfate.
69. The combination according to any one of embodiments 56 to 68, wherein the dextromethorphan and the quinidine are administered in a combined dose, and wherein a weight ratio of dextromethorphan to quinidine in the combined dose is selected from the group consisting of about 1:0.68, about 1:0.56, about 1:0.44, about 1:0.38, and about 1:0.25.
70. The combination according to any one of embodiments 56 to 69, wherein the dextromethorphan and quinidine are administered as one combined dose per day.
71. The combination according to any one of embodiments 56 to 69, wherein the dextromethorphan and quinidine are administered as at least two combined doses per day.
72. The combination according to any one of embodiments 56 to 71, wherein dextromethorphan and quinidine are administered in a tablet unit dosage form or a capsule unit dosage form.

## Claims

1. A combination of deuterated dextromethorphan, or a pharmaceutically acceptable salt thereof, and quinidine, or a pharmaceutically acceptable salt thereof, for use in treating agitation and/or aggression in a subject with dementia, wherein the dementia is of the Alzheimer's type; and
wherein deuterated dextromethorphan is administered in an amount ranging from about 10 mg to about 200 mg per day, and wherein quinidine is administered in an amount ranging from about 0.05 mg to less than about 50 mg per day.

2. The combination according to claim 1, wherein quinidine is administered in an amount ranging from about 4.75 mg to about 20 mg per day.

3. The combination according to claims 1 or 2, wherein deuterated dextromethorphan is administered in an amount ranging from about 10 mg to about 90 mg per day.

4. The combination according to any one of claims 1 to 3, wherein at least one of the quinidine and the deuterated dextromethorphan is in a form of a pharmaceutically acceptable salt selected from the group consisting of salts of alkali metals, salts of lithium, salts of sodium, salts of potassium, salts of alkaline earth metals, salts of calcium, salts of magnesium, salts of lysine, salts of N.N'dibenzylethylenediamine, salts of chloroprocaine, salts of choline, salts of diethanolamine, salts of ethylenediamine, salts of meglumine, salts of procaine, salts of tris, salts of free acids, salts of free bases, inorganic salts, salts of sulfate, salts of hydrochloride, and salts of hydrobromide.

5. The combination according to any one of claims 1 to 4, wherein the deuterated dextromethorphan is in a form of deuterated dextromethorphan hydrobromide.

6. The combination according to any one of claims 1 to 5, wherein the quinidine is in a form of quinidine sulfate.

7. The combination according to any one of claims 1 to 6, wherein deuterated dextromethorphan and quinidine are administered in a unit dosage form comprising:
(i) about 24 mg deuterated dextromethorphan hydrobromide and about 4.75 mg quinidine sulfate; or
(ii) about 34 mg deuterated dextromethorphan hydrobromide and about 4.75 mg quinidine sulfate; or
(iii) about 18 mg deuterated dextromethorphan hydrobromide and about 4.9 mg quinidine sulfate; or
(iv) about 28 mg deuterated dextromethorphan hydrobromide and about 4.9 mg quinidine sulfate; or
(v) about 30 mg deuterated dextromethorphan hydrobromide and about 4.9 mg quinidine sulfate; or
(vi) about 24 mg deuterated dextromethorphan hydrobromide and about 4.9 mg quinidine sulfate; or
(vii) about 34 mg deuterated dextromethorphan hydrobromide and about 4.9 mg quinidine sulfate.

8. The combination according to any one of claims 1 to 7, wherein the deuterated dextromethorphan and the quinidine are administered in a combined dose, and wherein a weight ratio of deuterated dextromethorphan to quinidine in the combined dose is selected from the group consisting of about 1 :0.16, about 1 :0.18, about 1 :0.20, about 1 :0.22, about 1 :0.23, and about 1 :0.31.

9. The combination according to any one of claims 1 to 8, wherein the deuterated dextromethorphan and quinidine are administered as one combined dose per day.

10. The combination according to any one of claims 1 to 9, wherein the deuterated dextromethorphan and quinidine are administered as at least two combined doses per day.

11. The combination according to any one of claims 1 to 10, wherein deuterated dextromethorphan and quinidine are administered in a tablet unit dosage form or a capsule unit dosage form.

12. A combination of deuterated dextromethorphan, or a pharmaceutically acceptable salt thereof, and nortriptyline, or a pharmaceutically acceptable salt thereof, for use in treating agitation and/or aggression in a subject with dementia, wherein the dementia is of the Alzheimer's type.

13. A compound of formula (1-1) or (1-2):
